# EUROPEAN PATENT APPLICATION

(11) **EP 3 438 264 A1**
(43) Date of publication of application: **06.02.2019**
(21) Application number: 17775532.9
(22) Date of filing: 31.03.2017
(51) Int. Cl.: C12N 15/09, C07K 14/435, C07K 19/00, C12M 3/04, C12N 1/15, C12N 1/19, C12N 1/21, C12N 5/0735, C12N 5/10

(54) **FIBROIN-LIKE PROTEIN VARIANT AND CELL CULTURE METHOD**

(30) Priority: 31.03.2016 JP 2016071530; 21.10.2016 JP 2016207359
(71) Applicant: Ajinomoto Co., Inc., Tokyo 104-8315 (JP)
(72) Inventor: KITAZAWA, Manabu, Kawasaki-shi Kanagawa 210-8681 (JP); SUGIMOTO, Nao, Kawasaki-shi Kanagawa 210-8681 (JP); KODAMA, Riho, Kawasaki-shi Kanagawa 210-8681 (JP); NAGAHIKO, Takeshi, Kawasaki-shi Kanagawa 210-8681 (JP); ITO, Yoshihiro, Kawasaki-shi Kanagawa 210-8681 (JP); NAKASE, Kentaro, Kawasaki-shi Kanagawa 210-8681 (JP); TAKASHIMA, Yoshinori, Kawasaki-shi Kanagawa 210-8681 (JP); MINE, Ayako, Kawasaki-shi Kanagawa 210-8681 (JP); KUWABARA, Yoko, Kawasaki-shi Kanagawa 210-8681 (JP); TAGAMI, Uno, Kawasaki-shi Kanagawa 210-8681 (JP); SUGIKI, Masayuki, Kawasaki-shi Kanagawa 210-8681 (JP)
(74) Representative: Hoffmann Eitle
(86) International application number: PCT/JP2017/013587
(87) International publication number: WO 2017/171001

(57) **Abstract**

A technique for efficiently culturing cells such as stem cells is provided. Cells such as stem cells are cultured using a cell culture vessel coated with a fibroin-like protein inserted with a cell adhesion sequence containing RGD (Arg-Gly-Asp).

## Description

### Technical Field

The present invention relates to a fibroin-like protein variant and a cell culture method using the same.

### Background Art

Stem cells, particularly pluripotent stem cells such as embryonic stem cells (ES cells) and induced pluripotent stem cells (iPS cells), are expected to be applied to regenerative medicine. For culturing stem cells, feeder cells are usually used. However, due to constraints in clinical applications or the like, technology for replacing feeder cells has been developed.

For example, it is known to cultivate stem cells using a cell adhesion protein, such as fibronectin or laminin, as a scaffold for cell culture. It is also known to cultivate stem cells using a fibroin variant obtained by introducing a cell adhesion sequence into spider or silk fibroin as a scaffold for cell culture (Patent Documents 1 and 2, and Non-Patent Document 1). As a typical example of the cell adhesion sequence, there is known an amino acid sequence containing RGD (Arg-Gly-Asp).

However, it has not been known that stem cells can be efficiently cultured by using a fibroin variant having a fibroin repetitive structure introduced with a cell adhesion sequence containing RGD as a scaffold for cell culture.

### Prior art references

### Patent documents

Patent document 1: WO2015/036619
Patent document 2: JP2013-523174

### Non-patent documents

Non-patent document 1: Bini E, Foo CW, Huang J, Karageorgiou V, Kitchel B, Kaplan DL., Biomacromolecules. 2006 Nov;7(11):3139-45., RGD-functionalized bioengineered spider dragline silk biomaterial.

### Summary of the Invention

### Object to be Achieved by the Invention

An object of the present invention is to provide a technique for efficiently culturing cells such as stem cells.

### Means for Achieving the Object

The inventors of the present invention conducted various researches, as a result, found that stem cells can be efficiently cultured by using a fibroin-like protein variant having a fibroin-like protein repetitive structure introduced with a cell adhesion sequence containing RGD as a scaffold for cell culture, and accomplished present invention.

Thus, the present invention can be embodied, for example, as follows.
[1] A protein comprising a repetitive structure and a cell adhesion sequence,
   wherein the repetitive structure consists of a 3 to 15 times repeat of a repeating unit,
   wherein each repeating unit consists of an Ala-rich region and an Ala-non-rich region mutually linked,
   wherein each Ala-rich region consists of an amino acid sequence having a length of 8 to 54 residues,
   wherein the ratio of Ala residue in each Ala-rich region is 30% or more,
   wherein each Ala-rich region contains at least one Ala residue in any continuous four amino acid residues,
   wherein each Ala-non-rich region consists of an amino acid sequence having a length of 4 residues or longer,
   wherein the ratio of Ala residue in each Ala-non-rich region is 20% or less,
   wherein the cell adhesion sequence is contained in one or more repeating units selected from repeating units constituting the repetitive structure, and
   wherein each cell adhesion sequence consists of an amino acid sequence containing Arg-Gly-Asp and having a length of 3 to 18 residues.
[2] The protein mentioned above, wherein the cell adhesion sequence is contained in two or more repeating units selected from repeating units constituting the repetitive structure.
[3] The protein mentioned above, wherein the total number of the cell adhesion sequence contained in the protein is 1 to 50.
[4] The protein mentioned above, wherein each cell adhesion sequence has been inserted in the Ala-non-rich region, or in the linkage site between the Ala-rich region and the Ala-non-rich region
[5] The protein mentioned above, wherein each cell adhesion sequence consists of an amino acid sequence of (a), (b), or (c) shown below:
   (a) the amino acid sequence of SEQ ID NO: 22, 23, 24, 25, 30, or 31;
   (b) the amino acid sequence of SEQ ID NO: 22, 23, 24, 25, 30, or 31, including substitution, deletion, insertion, and/or addition of 1 to 3 amino acid residues at position(s) other than Arg-Gly-Asp;
   (c) an amino acid sequence showing an identity of 80% or higher to the amino acid sequence of SEQ ID NO: 22, 23, 24, 25, 30, or 31, provided that Arg-Gly-Asp is conserved.
[6] The protein mentioned above, wherein each Ala-rich region consists of an amino acid sequence having a length of 8 to 15 residues.
[7] The protein mentioned above, wherein each Ala-rich region consists of an amino acid sequence having a length of 9 residues.
[8] The protein mentioned above, wherein each Ala-rich region contains one or more Gly residues and one or more Ser residues.
[9] The protein mentioned above, wherein the ratio of Ala residue in each Ala-rich region is 50% or more.
[10] The protein mentioned above, wherein the ratio of Ala, Gly, and Ser residues in each Ala-rich region is 90% or more in total.
[11] The protein mentioned above, wherein each Ala-rich region consists of an amino acid sequence of (a), (b), or (c) shown below:
   (a) the amino acid sequence of SEQ ID NO: 7, 8, 9, or 10;
   (b) the amino acid sequence of SEQ ID NO: 7, 8, 9, or 10, including substitution, deletion, insertion, and/or addition of 1 to 3 amino acid residues;
   (c) an amino acid sequence showing an identity of 80% or higher to the amino acid sequence of SEQ ID NO: 7, 8, 9, or 10.
[12] The protein mentioned above, wherein each Ala-non-rich region consists of an amino acid sequence having a length of 15 to 100 residues.
[13] The protein mentioned above, wherein the ratio of Ala residue in each Ala-non-rich region is 5% or less.
[14] The protein mentioned above, wherein the ratio of Gly, Ser, Gln, Pro, and Tyr residues in each Ala-non-rich region is 90% or more in total.
[15] The protein mentioned above, wherein each Ala-non-rich region comprises one or more kinds of amino acid sequences selected from amino acid sequences of (a), (b), and (c) shown below:
   (a) the amino acid sequences of SEQ ID NOS: 11 to 21;
   (b) the amino acid sequences of SEQ ID NOS: 11 to 21, including substitution, deletion, insertion, and/or addition of 1 to 3 amino acid residues;
   (c) amino acid sequences showing an identity of 80% or higher to the amino acid sequences of SEQ ID NOS:11 to 21.
[16] The protein mentioned above, wherein each Ala-non-rich region comprises an amino acid sequence of (a), (b), or (c) shown below:
   (a) the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof;
   (b) the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof, including substitution, deletion, insertion, and/or addition of 1 to 10 amino acid residues;
   (c) an amino acid sequence showing an identity of 90% or higher to the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof.
[17] A composition containing the protein mentioned above.
[18] The composition mentioned above, wherein the composition is a cell scaffold material.
[19] The composition mentioned above, wherein the cell scaffold material is for culturing a stem cell.
[20] The composition mentioned above, wherein the stem cell is an embryonic stem cell or an induced pluripotent stem cell.
[21] The composition mentioned above, wherein the composition is a coating agent for a cell culture vessel.
[22] The composition mentioned above, wherein the cell culture vessel is for culturing a stem cell.
[23] The composition mentioned above, wherein the stem cell is an embryonic stem cell or an induced pluripotent stem cell.
[24] The composition mentioned above, wherein the composition further contains another protein, which is a protein other than the proteins according to claims 1 to 16.
[25] The composition mentioned above, wherein the other protein is any one of proteins (1) to (8) shown below:
   (1) a protein that stabilizes a component in a culture medium;
   (2) a protein comprising an amino acid sequence that stabilizes a component in a culture medium;
   (3) a protein having a cell adhesion ability;
   (4) a protein comprising an amino acid sequence having a cell adhesion ability;
   (5) a protein that promotes proliferation of a cell;
   (6) a protein comprising an amino acid sequence that promotes proliferation of a cell;
   (7) a protein that binds to any one of the proteins (1) to (6);
   (8) a protein comprising an amino acid sequence that binds to any one of the proteins (1) to (6).
[26] An apparatus comprising the protein mentioned above.
[27] The apparatus mentioned above, wherein the apparatus is an apparatus for medical or research use.
[28] The apparatus mentioned above, wherein the apparatus is a cell culture vessel.
[29] The apparatus mentioned above, wherein the apparatus is a cell scaffold material.
[30] The apparatus mentioned above, wherein a cell culture surface of the apparatus has been coated with the protein.
[31] The apparatus mentioned above, wherein the apparatus is for culturing a stem cell.
[32] The apparatus mentioned above, wherein the stem cell is an embryonic stem cell or an induced pluripotent stem cell.
[33] The apparatus mentioned above, wherein the apparatus further comprises another protein, which is a protein other than the proteins according to of claims 1 to 16.
[34] The apparatus mentioned above, wherein the other protein is any one of proteins (1) to (8) shown below:
   (1) a protein that stabilizes a component in a culture medium;
   (2) a protein comprising an amino acid sequence that stabilizes a component in a culture medium;
   (3) a protein having a cell adhesion ability;
   (4) a protein comprising an amino acid sequence having a cell adhesion ability;
   (5) a protein that promotes proliferation of a cell;
   (6) a protein comprising an amino acid sequence that promotes proliferation of a cell;
   (7) a protein that binds to any one of the proteins (1) to (6);
   (8) a protein comprising an amino acid sequence that binds to any one of the proteins (1) to (6).
[35] A method for producing a cultured cell, the method comprising:
   culturing a cell by using the apparatus mentioned above.
[36] The method mentioned above, wherein the cell to be cultured is a stem cell.
[37] The method mentioned above, wherein the stem cell is an embryonic stem cell or an induced pluripotent stem cell.
[38] The method mentioned above, wherein another protein, which is a protein other than the proteins according to claims 1 to 16, is further used in combination.
[39] The method mentioned above, wherein the other protein is any one of proteins (1) to (8) shown below:
   (1) a protein that stabilizes a component in a culture medium;
   (2) a protein comprising an amino acid sequence that stabilizes a component in a culture medium;
   (3) a protein having a cell adhesion ability;
   (4) a protein comprising an amino acid sequence having a cell adhesion ability;
   (5) a protein that promotes proliferation of a cell;
   (6) a protein comprising an amino acid sequence that promotes proliferation of a cell;
   (7) a protein that binds to any one of the proteins (1) to (6);
   (8) a protein comprising an amino acid sequence that binds to any one of the proteins (1) to (6).
[40] A gene encoding the protein mentioned above.
[41] A vector having the gene mentioned above.
[42] A host having the gene mentioned above.

### Brief Description of the Drawings

[Fig. 1]
   A graph showing results of evaluating a scaffold function for an iPS cell using various cell culture vessels coated with a cell adhesion sequence-inserted fibroin-like protein or with an existing basement membrane matrix or a mimic thereof.
[Fig. 2]
   A graph showing results of evaluating a scaffold function for an iPS cell using a cell culture plate immediately after coating with a cell adhesion sequence-inserted fibroin-like protein, or after storage for 6 days at 4°C after the coating.
[Fig. 3]
   A graph showing results of evaluating a scaffold function for an iPS cell using various culture media with a cell culture plate coated with a cell adhesion sequence-inserted fibroin-like protein.
[Fig. 4]
   A graph showing results of evaluating a scaffold function for an iPS cell over a period of 3 weeks using a cell culture plate coated with a cell adhesion sequence-inserted fibroin-like protein. The horizontal axis represents "week".
[Fig. 5]
   A graph showing results of evaluating a scaffold function for an iPS cell using a cell culture plate immediately after coating with a cell adhesion sequence-inserted fibroin-like protein, or after storage for 6 days at 37°C after the coating.
[Fig. 6]
   A graph showing results of evaluating a scaffold function for an iPS cell over a period of 3 weeks using a cell culture plate coated with a cell adhesion sequence-inserted fibroin-like protein. The horizontal axis represents "week".
[Fig. 7]
   A graph showing results of evaluating a scaffold function for an iPS cell using a cell culture plate coated with a cell adhesion sequence-inserted fibroin-like protein and/or a heparin-binding sequence-inserted fibroin-like protein.

### Modes for Carrying out the Invention

Hereinafter, the present invention will be explained in detail.

### <1> Protein of the present invention

### <1-1> Protein of the present invention

The protein of the present invention is a protein comprising a repetitive structure and a cell adhesion sequence.

The protein of the present invention may specifically be a fibroin-like protein inserted with a cell adhesion sequence. Hence, the protein of the present invention is also referred to as "cell adhesion sequence-inserted fibroin-like protein".

In the present invention, the term "fibroin-like protein" is a generic term for referring to fibroin and a protein having a structure similar to that of fibroin.

The term "fibroin" refers to a fibrous protein constituting spider's thread or silkworm's thread. That is, examples of fibroin include fibroin of spider, and fibroin of silkworm. Species of spider, species of silkworm, and type of thread are not particularly limited. Examples of spider include *Araneus diadematus* and *Nephila clavipes.* Other examples of spider include *Araneus bicentenarius, Argiope amoena, Argiope aurantia, Argiope trifasciata, Cyrtophora moluccensis, Dolomedes tenebrosus, Euprosthenops australis, Gasteracantha mammosa, Latrodectus geometricus, Latrodectus hesperus, Macrothele holsti, Nephila pilipes, Nephila madagascariensis, Nephila senegalensis, Octonoba varians, Psechrus sinensis, Tetragnatha kauaiensis,* and *Tetragnatha versicolor.* Examples of fibroin of spider include proteins of drag line, frame thread, and radius thread produced by major ampullate gland (major ampullate gland proteins), proteins of scaffolding thread produced by minor ampullate gland (minor ampullate gland proteins), and proteins of spiral line produced by flagelliform gland (flagelliform gland proteins). Specific examples of fibroin of spider include, for example, the major ampullate gland proteins ADF3 and ADF4 of *Araneus diadematus* and the major ampullate gland proteins MaSp1 and MaSp2 of *Nephila clavipes.* Examples of silkworm include *Bombyx mori* and *Samia cynthia.* The amino acid sequences of these fibroins and the nucleotide sequences of the genes encoding these fibroins (also referred to as "fibroin gene") can be obtained from public databases such as NCBI (http://www.ncbi.nlm.nih.gov/).

The term "protein having a structure similar to that of fibroin" refers to a protein having a sequence similar to a repetitive sequence of fibroin. The "sequence similar to a repetitive sequence of fibroin" may be a sequence actually found in fibroin, or may be a sequence similar to such a sequence. Examples of the sequence similar to a repetitive sequence of fibroin include the "repetitive sequence" defined in the present invention. Examples of the protein having a structure similar to that of fibroin include polypeptides derived from the large spigot drag line proteins described in WO2012/165476 and recombinant spider silk proteins described in WO2006/008163. Examples of the protein having a structure similar to that of fibroin also include the ADF3 protein used in the Examples section. The nucleotide sequence of the ADF3 gene used in the Examples section is shown in SEQ ID NO: 1, and the amino acid sequence of the ADF3 protein encoded by the gene is shown in SEQ ID NO: 2. Incidentally, the nucleotide sequence of positions 7-1989 of SEQ ID NO: 1 encodes the amino acid sequence shown in SEQ ID NO: 2. The protein having a structure similar to that of fibroin may or may not be a fibrous protein.

The term "fibrous protein" refers to a protein that has a fibrous form under predetermined conditions. That is, the fibrous protein may be a protein expressed in a fibrous form, or a protein that is not in a fibrous form when it is expressed, but can be processed into a fibrous form. The fibrous protein may be, for example, a protein that is expressed as an inclusion body, and can be then processed into a fibrous form by an appropriate technique. Examples of methods for processing a protein into a fibrous form include the method disclosed in WO2012/165476.

That is, the fibroin-like protein may be, for example, a protein having any of the amino acid sequences of fibroin-like proteins disclosed in the aforementioned database or documents, or a protein having the amino acid sequence shown in SEQ ID NO: 2. The fibroin-like protein may also be, for example, a protein having a partial sequence of any of those amino acid sequences. Examples of the partial sequence of an amino acid sequence include a portion having a sequence similar to the repetitive sequence of fibroin. Specific examples of the partial sequence of an amino acid sequence include, for example, the amino acid sequence of positions 25-610 of SEQ ID NO: 2 and the amino acid sequence of positions 53-536 of SEQ ID NO: 2. Similarly, a gene encoding a fibroin-like protein (also referred to as "fibroin-like protein gene") may be, for example, a gene having any of the nucleotide sequences of the fibroin genes disclosed in the aforementioned database or documents, or a gene having the nucleotide sequence of positions 7-1989 of SEQ ID NO: 1. The fibroin-like protein gene may also be, for example, a gene having a partial sequence of any of those nucleotide sequences. Examples of the partial sequence of a nucleotide sequence include a portion encoding an amino acid sequence having a sequence similar to the repetitive sequence of fibroin. The expression of "having an (amino acid or nucleotide) sequence" encompasses both the case of "comprising the (amino acid or nucleotide) sequence", and the case of "consisting of the (amino acid or nucleotide) sequence".

The fibroin-like protein may also be a variant of any of the fibroin-like proteins exemplified above (that is, fibroin and the proteins having a structure similar to that of fibroin exemplified above), so long as the original function thereof is maintained. Similarly, the fibroin-like protein gene may also be a variant of any of the fibroin-like protein genes exemplified above (that is, genes encoding fibroin and the proteins having a structure similar to that of fibroin exemplified above), so long as the original function thereof is maintained. Such variants that maintain the original function are also referred to as "conservative variants". Examples of the conservative variants include, for example, homologues and artificially-modified proteins or genes of the fibroin-like proteins exemplified above and genes encoding them.

The expression "the original function is maintained" means that a variant of a gene or protein has a function (e.g. activity and property) corresponding to the function (e.g. activity and property) of the original gene or protein. That is, in the case of the fibroin-like protein, the expression "the original function is maintained" means that a variant of the protein shows a scaffold function for a cell, such as a stem cell, due to insertion of a cell adhesion sequence. In the case of the fibroin-like protein gene, the expression "the original function is maintained" means that a variant of the gene encodes a protein that maintains the original function (namely, a protein showing a scaffold function for a cell, such as a stem cell, due to insertion of a cell adhesion sequence).

Examples of homologues of the fibroin-like protein include, for example, a protein obtained from a public database by BLAST search or FASTA search using any of the aforementioned amino acid sequences of fibroin-like proteins as a query sequence. Also, a homologue of the aforementioned fibroin-like protein genes can be obtained by, for example, PCR using a chromosome of various microorganisms as the template, and oligonucleotides prepared on the basis of any of the aforementioned nucleotide sequences of fibroin-like protein genes as the primers.

Conservative variants of the fibroin-like protein and fibroin-like protein gene will be explained below.

The fibroin-like protein may be a protein having any of the aforementioned amino acid sequences of fibroin-like proteins including substitution, deletion, insertion, and/or addition of one or several amino acid residues at one or several positions, so long as the original function of the protein is maintained. Although the number meant by the term "one or several" can differ depending on the positions of amino acid residues in the three-dimensional structure of the protein, or the types of amino acid residues, it is specifically, for example, 1 to 50, 1 to 40, 1 to 30, preferably 1 to 20, more preferably 1 to 10, still more preferably 1 to 5, particularly preferably 1 to 3.

The aforementioned substitution, deletion, insertion, and/or addition of one or several amino acid residues are/is each a conservative mutation that maintains the original function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Further, such substitution, deletion, insertion, and/or addition of amino acid residues as mentioned above includes a naturally occurring mutation due to an individual difference, or a difference of species of the organism from which the gene is derived (mutant or variant).

The fibroin-like protein may be a protein having an amino acid sequence showing a homology of 80% or higher, preferably 90% or higher, more preferably 95% or higher, still more preferably 97% or higher, particularly preferably 99% or higher, to any of the aforementioned amino acid sequences of fibroin-like proteins, so long as the original function is maintained. In this description, "homology" means "identity".

The fibroin-like protein may be a protein encoded by a DNA that is able to hybridize under stringent conditions with a probe that can be prepared from any of the aforementioned nucleotide sequences of fibroin-like protein genes, such as a sequence complementary to the whole sequence or a partial sequence of any of the aforementioned nucleotide sequences, so long as the original function is maintained. Such a probe can be prepared by PCR using oligonucleotides prepared on the basis of any of the aforementioned nucleotide sequences as the primers, and a DNA fragment comprising any of the aforementioned nucleotide sequences as the template. The term "stringent conditions" refers to conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, still more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C. Further, for example, when a DNA fragment having a length of about 300 bp is used as the probe, the washing conditions of the hybridization can be, for example, 50°C, 2 x SSC, and 0.1% SDS.

Furthermore, any codons in the fibroin-like protein gene may have been replaced with respective equivalent codons. That is, the fibroin-like protein gene may also be a variant of any of the fibroin-like protein genes exemplified above due to the degeneracy of the genetic code. For example, the fibroin-like protein gene may be a gene modified so that it has optimal codons according to codon frequencies in a host to be used.

The percentage of the sequence identity between two sequences can be determined by, for example, using a mathematical algorithm. Non-limiting examples of such a mathematical algorithm include the algorithm of Myers and Miller (1988) CABIOS 4:11-17, the local homology algorithm of Smith et al (1981) Adv. Appl. Math. 2:482, the homology alignment algorithm of Needleman and Wunsch (1970) J. Mol. Biol. 48:443-453, the method for searching homology of Pearson and Lipman (1988) Proc. Natl. Acad. Sci. 85:2444-2448, and an modified version of the algorithm of Karlin and Altschul (1990) Proc. Natl. Acad. Sci. USA 87:2264, such as that described in Karlin and Altschul (1993) Proc. Natl. Acad. Sci. USA 90:5873-5877.

By using a program based on such a mathematical algorithm, sequence comparison (i.e. alignment) for determining the sequence identity can be performed. The program can be appropriately executed by a computer. Examples of such a program include, but not limited to, CLUSTAL of PC/Gene program (available from Intelligenetics, Mountain View, Calif.), ALIGN program (Version 2.0), and GAP, BESTFIT, BLAST, FASTA, and TFASTA of Wisconsin Genetics Software Package, Version 8 (available from Genetics Computer Group (GCG), 575 Science Drive, Madison, Wis., USA). Alignment using these programs can be performed by using, for example, initial parameters. The CLUSTAL program is well described in Higgins et al. (1988) Gene 73:237-244 (1988), Higgins et al. (1989) CABIOS 5:151-153, Corpet et al. (1988) Nucleic Acids Res. 16:10881-90, Huang et al. (1992) CABIOS 8:155-65, and Pearson et al. (1994) Meth. Mol. Biol. 24:307-331.

In order to obtain a nucleotide sequence homologous to a target nucleotide sequence, in particular, for example, BLAST nucleotide search can be performed by using BLASTN program with score of 100 and word length of 12. In order to obtain an amino acid sequence homologous to a target protein, in particular, for example, BLAST protein search can be performed by using BLASTX program with score of 50 and word length of 3. See http://www.ncbi.nlm.nih.gov for BLAST nucleotide search and BLAST protein search. In addition, Gapped BLAST (BLAST 2.0) can be used in order to obtain an alignment including gap(s) for the purpose of comparison. In addition, PSI-BLAST (BLAST 2.0) can be used in order to perform repetitive search for detecting distant relationships between sequences. See Altschul et al. (1997) Nucleic Acids Res. 25:3389 for Gapped BLAST and PSI-BLAST. When using BLAST, Gapped BLAST, or PSI-BLAST, initial parameters of each program (e.g. BLASTN for nucleotide sequences, and BLASTX for amino acid sequences) can be used. Alignment can also be manually performed.

The sequence identity between two sequences is calculated as the ratio of residues matching in the two sequences when aligning the two sequences so as to fit maximally with each other.

The protein of the present invention may be a protein having an amino acid sequence identical to that of any of the fibroin-like proteins exemplified above and conservative variants thereof, except that the protein of the present invention contains a cell adhesion sequence. Also, a gene encoding the protein of the present invention may be a gene having a nucleotide sequence identical to that of any of the fibroin-like protein genes exemplified above and conservative variants thereof, except that gene encoding the protein of the present invention contains a nucleotide sequence encoding a cell adhesion sequence.

Specific examples of the protein of the present invention include the cell adhesion sequence-inserted fibroin-like proteins used in the Examples section. The nucleotide sequences of genes encoding the cell adhesion sequence-inserted fibroin-like proteins used in the Examples section are shown in SEQ ID NOS: 3, 5, 26, 28, 35, 37, 39, 41, 43, 45, 47, 49, 51, and 53, and the amino acid sequences of the cell adhesion sequence-inserted fibroin-like proteins encoded by these genes are shown in SEQ ID NOS: 4, 6, 27, 29, 36, 38, 40, 42, 44, 46, 48, 50, 52, and 54. That is, the protein of the present invention may be, for example, a protein having the amino acid sequence shown in SEQ ID NO: 4, 6, 27, 29, 36, 38, 40, 42, 44, 46, 48, 50, 52, or 54. Specific examples of the protein of the present invention also include conservative variants of the cell adhesion sequence-inserted fibroin-like proteins used in the Examples section, provided that the variants each have a cell adhesion sequence. The aforementioned descriptions concerning conservative variants of the fibroin-like protein can be applied *mutatis mutandis* to conservative variants of the cell adhesion sequence-inserted fibroin-like protein. The expression "the original function is maintained" used for the cell adhesion sequence-inserted fibroin-like protein means that a variant of the protein shows a scaffold function for a cell, such as a stem cell.

In the present invention, the term "gene" is not limited to DNA, but may include any polynucleotide, so long as it encodes a target protein. That is, the term "gene encoding the protein of the present invention" may mean any polynucleotide encoding the protein of the present invention. The gene encoding the protein of the present invention may be DNA, RNA, or a combination thereof. The gene encoding the protein of the present invention may be single-stranded or double-stranded. The gene encoding the protein of the present invention may be a single-stranded DNA or a single-stranded RNA. The gene encoding the protein of the present invention may be a double-stranded DNA, a double-stranded RNA, or a hybrid strand consisting of a DNA strand and an RNA strand. The gene encoding the protein of the present invention may contain both a DNA residue and an RNA residue in a single polynucleotide chain. When the gene encoding the protein of the present invention contains RNA, the aforementioned descriptions concerning DNA, such as those concerning nucleotide sequences exemplified above, may be applied to RNA with appropriately changing wordings to those for RNA as required. The mode of the gene encoding the protein of the present invention can be chosen according to various conditions such as use mode thereof.

Hereinafter, the structure of the protein of the present invention will be specifically explained.

The protein of the present invention comprises a repetitive structure and a cell adhesion sequence. The repetitive structure consists of a repeat of a repeating unit. Each repeating unit (the repeating unit in each repetition) consists of an Ala-rich region and an Ala-non-rich region mutually linked. In each repeating unit, the Ala-rich region is present at the N-terminus side and the Ala-non-rich region is present at the C-terminus side. That is, specifically, the repetitive structure consists of Ala-rich regions and Ala-non-rich regions linked alternately from the N-terminus toward the C-terminus.

The repetition number of the repeating unit is 3 to 15. The repetition number of the repeating unit may be, for example, 4 or more, 5 or more, 6 or more, 7 or more, 8 or more, 9 or more, or 10 or more. The repetition number of the repeating unit may be, for example, 12 or less, 10 or less, 8 or less, or 6 or less. The repetition number of the repeating unit may be, for example, within a range defined as a non-contradictory combination of the ranges exemplified above. The repetition number of the repeating unit may be, specifically, for example, 3 to 12, 3 to 10, 3 to 8, 3 to 6, 5 to 15, 7 to 15, or 10 to 15.

The structure of the repeating unit may or may not be the same in each repetition. That is, the structure of the Ala-rich region may or may not be the same in each repetition. Also, the structure of the Ala-non-rich region may or may not be the same in each repetition. That is, the structure of the repeating unit, the structure of the Ala-rich region, and the structure of the Ala-non-rich region each may be selected independently in each repetition. That is, as each of the repeating unit, the Ala-rich region, and the Ala-non-rich region, a single kind of amino acid sequence may be used, or two or more kinds of amino acid sequences may be used in combination.

### < Ala-rich region >

The term "Ala-rich region" refers to a region having a high ratio of Ala residue, i.e. a region containing Ala residues at a high ratio.

The length of the Ala-rich region is 8 to 54 residues. That is, the Ala-rich region consists of an amino acid sequence having a length of 8 to 54 residues. The length of the Ala-rich region may be, for example, 40 residues or shorter, 30 residues or shorter, 20 residues or shorter, 15 residues or shorter, or 10 residues or shorter. The length of the Ala-rich region may be, for example, within a range defined as a combination of the ranges exemplified above. The length of the Ala-rich region may be, specifically, for example, 8 to 15 residues, or 8 to 10 residues. The length of the Ala-rich region may be, more specifically, for example, 9 residues.

The Ala-rich region may consist of Ala residues, or may consist of a combination of Ala residues and other amino acid residue(s), i.e. a combination of Ala residues and amino acid residue(s) other than Ala residue.

The ratio of Ala residue in the Ala-rich region is 30% or more. The ratio of Ala residue in the Ala-rich region may be, for example, 40% or more, 50% or more, 60% or more, or 70% or more. The ratio of Ala residue in the Ala-rich region may be, for example, 100% or less, 95% or less, 90% or less, 85% or less, or 80% or less. The ratio of Ala residue in the Ala-rich region may be, for example, within a range defined as a combination of the ranges exemplified above. The ratio of Ala residue in the Ala-rich region may be, specifically, for example, 30% to 100%, 50% to 90%, or 60% to 85%. The term "ratio of Ala residue in an Ala-rich region" refers to the ratio of the number of Ala residues in an Ala-rich region with respect to the total number of amino acid residues constituting the Ala-rich region. The ratio of any other amino acid residue in the Ala-rich region can be similarly calculated.

Examples of the other amino acid residue (i.e. amino acid residue other than Ala residue) include Gly, Val, Leu, Ile, Ser, Thr, Cys, Met, Asn, Gln, Pro, Asp, Glu, Lys, Arg, His, Phe, Tyr, and Trp. Specific examples of the other amino acid residue include, for example, Gly residue and Ser residue. The Ala-rich region may contain a single kind of other amino acid residue, or contain two or more kinds of other amino acid residues. The Ala-rich region, for example, may contain one or more Gly residues, may contain one or more Ser residues, or may contain one or more Gly residues and one or more Ser residues. The both termini (i.e. N-terminus and C-terminus) of the Ala-rich region may each be, for example, Ala residue, Gly residue, or Ser residue. Specifically, for example, the N-terminus of the Ala-rich region may be Ala or Gly residue, and the C-terminus of the Ala-rich region may be Ala or Ser residue. The ratio of Ala, Gly, and Ser residues in the Ala-rich region may be, for example, 50% or more, 60% or more, 70% or more, 80% or more, 90% or more, 95% or more, or 100%, in total.

The Ala-rich region contains at least one Ala residue in any continuous 4 amino acid residues in the region. That is, there are no continuous 4 amino acid residues containing no Ala residue in the Ala-rich region. Also, the Ala-rich region may contain at least two Ala residues in any continuous 7, 6, or 5 amino acid residues in the region. That is, there may be no continuous 7, 6, or 5 amino acid residues containing no Ala residue or containing only one Ala residue in the Ala-rich region.

Examples of the amino acid sequence of the Ala-rich region include, for example, the amino acid sequence of a region satisfying such requirement(s) of the Ala-rich region as described above within the amino acid sequence of a fibroin-like protein. The fibroin-like protein is as described above. Examples of the region satisfying such requirement(s) of the Ala-rich region as described above within the amino acid sequence of SEQ ID NO: 2 include the regions of amino acid numbers of 53 to 61, 92 to 100, 121 to 129, 152 to 160, 186 to 194, 219 to 227, 258 to 266, 292 to 300, 326 to 334, 370 to 378, 439 to 447, and 498 to 506. Specific examples of the amino acid sequences of those regions satisfying the requirement(s) of the Ala-rich region include, for example, GASAAAAAA (SEQ ID NO: 7), GSSAAAAAA (SEQ ID NO: 8), GASAASAAS (SEQ ID NO: 9), and GASAAAGAA (SEQ ID NO: 10). That is, the Ala-rich region may have, for example, any of the amino acid sequences of those regions satisfying the requirement(s) of the Ala-rich region (e.g. SEQ ID NO: 7, 8, 9, or 10).

The Ala-rich region may also be a variant of any of the amino acid sequences of those regions satisfying the requirement(s) of the Ala-rich region (e.g. SEQ ID NO: 7, 8, 9, or 10). The aforementioned descriptions concerning conservative variants of the fibroin-like protein can be applied *mutatis mutandis* to variants of those regions satisfying the requirement(s) of the Ala-rich region. The Ala-rich region may be, for example, one having any of the amino acid sequences of those regions satisfying the requirement(s) of the Ala-rich region (e.g. SEQ ID NO: 7, 8, 9, or 10) including substitution, deletion, insertion, and/or addition of one or several amino acid residues at one or several positions. Although the number meant by the term "one or several" can differ depending on the length of those regions satisfying the requirement(s) of the Ala-rich region etc., it may be, for example, 1 to 5, 1 to 4, 1 to 3, 1 to 2, or 1. The Ala-rich region may also be, for example, one having an amino acid sequence showing an identity of 80% or higher, preferably 90% or higher, more preferably 95% or higher, still more preferably 97% or higher, particularly preferably 99% or higher, to the whole of any of the amino acid sequences of those regions satisfying the requirement(s) of the Ala-rich region (e.g. SEQ ID NO: 7, 8, 9, or 10). Variants of those regions satisfying the requirement(s) of the Ala-rich region are not particularly limited, so long as the amino acid sequences after modification satisfy such requirement(s) of the Ala-rich region as described above.

### < Ala-non-rich region >

The term "Ala-non-rich region" refers to a region having a low ratio of Ala residue.

The length of the Ala-non-rich region is 4 residues or longer. That is, the Ala-non-rich region consists of an amino acid sequence having a length of 4 residues or longer. The length of the Ala-non-rich region may be, for example, 10 residues or longer, 15 residues or longer, 20 residues or longer, 30 residues or longer, 40 residues or longer, or 50 residues or longer. The length of the Ala-non-rich region may be, for example, 200 residues or shorter, 150 residues or shorter, 100 residues or shorter, 80 residues or shorter, 60 residues or shorter, or 40 residues or shorter. The length of the Ala-non-rich region may be, for example, within a range defined as a non-contradictory combination of the ranges exemplified above. The length of the Ala-non-rich region may be, specifically, for example, 4 to 200 residues, 10 to 150 residues, or 15 to 100 residues.

The Ala-non-rich region may or may not contain Ala residue(s). The ratio of Ala residue in the Ala-non-rich region is 20% or less. The ratio of Ala residue in the Ala-non-rich region may be, for example, 16% or less, 14% or less, 10% or less, 5% or less, or 0%.

The Ala-non-rich region may contain, specifically, for example, one or more kinds of, e.g. all of, amino acid residues selected from Gly, Ser, Gln, Pro, and Tyr. The ratio of Gly, Ser, Gln, Pro, and Tyr residues in the Ala-non-rich region may be, for example, 50% or more, 60% or more, 70% or more, 80% or more, 90% or more, 95% or more, or 100%, in total. The Ala-non-rich region may contain continuous 4 amino acid residues containing no Ala residue. The Ala-non-rich region may contain, for example, continuous 4 amino acid residues containing no Ala residue at each of the both termini (i.e. N-terminus and C-terminus).

Examples of the amino acid sequence of the Ala-non-rich region include, for example, the amino acid sequence of a region satisfying such requirement(s) of the Ala-non-rich region as described above within the amino acid sequence of a fibroin-like protein. The fibroin-like protein is as described above. Examples of the region satisfying such requirement(s) of the Ala-non-rich region as described above within the amino acid sequence of SEQ ID NO: 2 include the regions of amino acid numbers of 62 to 91, 101 to 120, 130 to 151, 161 to 185, 195 to 218, 228 to 257, 267 to 291, 301 to 325, 335 to 369, 379 to 438, 448 to 497, and 507 to 536. That is, the Ala-non-rich region may have, for example, any of the amino acid sequences of those regions satisfying the requirement(s) of the Ala-non-rich region. The Ala-non-rich region may also have, for example, a partial sequence of any of the amino acid sequences of the Ala-non-rich region exemplified above (e.g. the amino acid sequences of those regions satisfying the requirement(s) of the Ala-non-rich region). The partial sequence is not particularly limited, so long as it satisfies such requirement(s) of the Ala-non-rich region as described above. Examples of the partial sequence include, for example, a partial sequence of any of the amino acid sequences of the Ala-non-rich region exemplified above, which partial sequence satisfies the length of the Ala-non-rich region exemplified above. Examples of the partial sequence also include, for example, a partial sequence of any of the amino acid sequences of the Ala-non-rich region exemplified above, which partial sequence has a length of 30% or longer, 50% or longer, 70% or longer, or 90% or longer of the length of any of those amino acid sequences of the Ala-non-rich region. Furthermore, specific examples of motifs contained in those regions satisfying the requirement(s) of the Ala-non-rich region include, for example, GGYGPGSGQQG (SEQ ID NO: 11), GGNGPGSGQQG (SEQ ID NO: 12), GGYGPGYGQQG (SEQ ID NO: 13), GGYGPGSGQG (SEQ ID NO: 14), PGQQG (SEQ ID NO: 15), AGQQG (SEQ ID NO: 16), SGQQG (SEQ ID NO: 17), PSQQG (SEQ ID NO: 18), PGGQG (SEQ ID NO: 19), PYGP (SEQ ID NO: 20), and AYGP (SEQ ID NO: 21). The motifs shown in SEQ ID NOS: 11 to 14 are also collectively referred to as "motif A", the motifs shown in SEQ ID NOS: 15 to 19 are also collectively referred to as "motif B", and the motifs shown in SEQ ID NOS: 20 to 21 are also collectively referred to as "motif C". That is, the Ala-non-rich region may have, more typically, for example, one or more kinds of motifs selected from these motifs. The Ala-non-rich region, specifically, for example, may have the motifs A and B, and may further have the motif C. When the Ala-non-rich region may have two or more kinds of motifs selected from these motifs, the order of the motifs is not particularly limited. The Ala-non-rich region may have, for example, the motifs A and B or the motifs A, B, and C from the N-terminus toward the C-terminus. The Ala-non-rich region may have only one unit each of any one or more of these motifs, or may have two or more units each of any one or more of these motifs. When the Ala-non-rich region has two or more units of any one motif, the units of the motif may or may not be arranged in tandem in the Ala-non-rich region. The Ala-non-rich region may have, for example, only one unit of the motif B, or may have two or more units of the motif B. The number of the motif B contained in one Ala-non-rich region, for example, may be 1 or more, 2 or more, 3 or more, 5 or more, or 7 or more, may be 15 or less, 10 or less, 7 or less, or 5 or less, or may be within a range defined as a non-contradictory combination thereof.

The Ala-non-rich region may also be a variant of any of the amino acid sequences of those regions satisfying the requirement(s) of the Ala-non-rich region, a variant of any of partial sequences thereof, or a variant of any of motifs contained therein. The aforementioned descriptions concerning conservative variants of the fibroin-like protein can be applied *mutatis mutandis* to variants of the amino acid sequences of those regions satisfying the requirement(s) of the Ala-non-rich region, variants of partial sequences thereof, or variants of motifs contained therein. The Ala-non-rich region may be, for example, one having any of the amino acid sequences of those regions satisfying the requirement(s) of the Ala-non-rich region, any of partial sequences thereof, or any of motifs contained therein including substitution, deletion, insertion, and/or addition of one or several amino acid residues at one or several positions. Although the number meant by the term "one or several" can differ depending on the length of the amino acid sequences of those regions satisfying the requirement(s) of the Ala-non-rich region, partial sequences thereof, or motifs contained therein etc., it may be, for example, 1 to 20, 1 to 15, 1 to 10, 1 to 5, 1 to 4, 1 to 3, 1 to 2, or 1. The Ala-non-rich region may also be, for example, one having an amino acid sequence showing an identity of 80% or higher, preferably 90% or higher, more preferably 95% or higher, still more preferably 97% or higher, particularly preferably 99% or higher, to the whole of any of the amino acid sequences of those regions satisfying the requirement(s) of the Ala-non-rich region, the whole of any of partial sequences thereof, or the whole of any of motifs contained therein. Variants of the amino acid sequences of those regions satisfying the requirement(s) of the Ala-non-rich region, variants of partial sequences thereof, or variants of motifs contained therein are not particularly limited, so long as the amino acid sequences after modification satisfy such requirement(s) of the Ala-non-rich region as described above.

### < Cell adhesion sequence >

The term "cell adhesion sequence" refers to an amino acid sequence capable of imparting a scaffold function for a cell, such as a stem cell, to a protein such as a fibroin-like protein. The term "cell adhesion sequence" specifically refers to an amino acid sequence capable of imparting a scaffold function for a cell, such as a stem cell, to a protein such as a fibroin-like protein due to insertion of the amino acid sequence into the protein. Such a characteristic (i.e. a characteristic capable of imparting a scaffold function for a cell, such as a stem cell, to a protein such as a fibroin-like protein) is also referred to as "characteristic as a cell adhesion sequence".

The cell adhesion sequence consists of an amino acid sequence having a length of 3 to 18 residues containing RGD (Arg-Gly-Asp). That is, the length of the cell adhesion sequence is 3 to 18 residues. The cell adhesion sequence may or may not consist of RGD. Examples of the cell adhesion sequence include a partial amino acid sequence of a cell adhesion protein (i.e. a part of the amino acid sequence of a cell adhesion protein), which partial amino acid sequence contains RGD, i.e. an RGD sequence of a cell adhesion protein. Examples of the cell adhesion sequence also include a combination of partial amino acid sequences of cell adhesion protein(s), which combination contains RGD. Specific examples of the cell adhesion protein include, for example, fibronectin, vitronectin, collagen, osteopontin, and laminin. Specific examples of the RGD sequence of a cell adhesion protein include, for example, GRGDSP (SEQ ID NO: 22), VTGRGDSPAS (SEQ ID NO: 23), PQVTRGDVFTM (SEQ ID NO: 24), VTRGDVF (SEQ ID NO: 25), GRGDNP (SEQ ID NO: 30), and GAAGRGDSPAAGY (SEQ ID NO: 31). That is, the cell adhesion sequence may have, for example, any of those amino acid sequences (e.g. SEQ ID NO: 22, 23, 24, 25, 30, or 31).

The cell adhesion sequence may also be a variant of any of those amino acid sequences (e.g. a partial amino acid sequence of a cell adhesion protein, such as SEQ ID NO: 22, 23, 24, 25, 30, or 31), so long as RGD is conserved. The aforementioned descriptions concerning conservative variants of the fibroin-like protein can be applied *mutatis mutandis* to variants of those amino acid sequences. The cell adhesion sequence may be, for example, one having any of those amino acid sequences (e.g. a partial amino acid sequence of a cell adhesion protein, such as SEQ ID NO: 22, 23, 24, 25, 30, or 31) including substitution, deletion, insertion, and/or addition of one or several amino acid residues at one or several positions other than the position of RGD. Although the number meant by the term "one or several" can differ depending on the length of those regions satisfying the requirement(s) of the cell adhesion sequence etc., it may be, for example, 1 to 5, 1 to 4, 1 to 3, 1 to 2, or 1. The cell adhesion sequence may also be, for example, one having an amino acid sequence showing an identity of 80% or higher, preferably 90% or higher, more preferably 95% or higher, still more preferably 97% or higher, particularly preferably 99% or higher, to the whole of any of the amino acid sequences of those regions satisfying the requirement(s) of the cell adhesion sequence (e.g. a partial amino acid sequence of a cell adhesion protein, such as SEQ ID NO: 22, 23, 24, 25, 30, or 31), provided that RGD is conserved. Variants of those amino acid sequences are not particularly limited, so long as RGD is conserved, and the variants have the characteristic as a cell adhesion sequence (i.e. the variants are capable of imparting a scaffold function for a cell, such as a stem cell, to a protein such as a fibroin-like protein).

Whether or not a certain amino acid sequence has the characteristic as a cell adhesion sequence can be confirmed by, for example, confirming whether or not a fibroin-like protein inserted with the certain amino acid sequence shows a scaffold function for a cell, such as a stem cell. Whether or not such a protein shows a scaffold function for a cell, such as a stem cell, can be confirmed by, for example, culturing a cell, such as a stem cell, using a culture vessel having a cell culture surface coated with the protein. That is, when proliferation of a cell, such as a stem cell, is improved due to coating of the cell culture surface with the protein, it can be concluded that the protein shows a scaffold function for a cell, such as a stem cell.

The protein of the present invention contains the cell adhesion sequence in the repetitive structure. That is, the cell adhesion sequence has been inserted in the repetitive structure. In other words, the repetitive structure possessed by the protein of the present invention is a repetitive structure inserted with the cell adhesion sequence. The cell adhesion sequence has been inserted (is contained) in, specifically, one or more repeating units selected from the repeating units constituting the repetitive structure. The cell adhesion sequence has been inserted (is contained) in, for example, 2 or more, 3 or more, 4 or more, 5 or more, 6 or more, or 7 or more repeating units selected from the repeating units constituting the repetitive structure. The cell adhesion sequence has been inserted (is contained) in, for example, all of the repeating units constituting the repetitive structure. One repeating unit may have only one unit of the cell adhesion sequence, or may have two or more units of the cell adhesion sequence. When one repeating unit has two or more units of the cell adhesion sequence, the units of the cell adhesion sequence may or may not be arranged in tandem in the repeating unit. The number of the cell adhesion sequence contained in one repeating unit, for example, may be 1 or more, 2 or more, or 3 or more, may be 5 or less, 3 or less, or 2 or less, or may be within a range defined as a non-contradictory combination thereof. The number of the cell adhesion sequence contained in one repeating unit may be, specifically, for example, 1 to 3. The total number of the cell adhesion sequence contained in the protein of the present invention, for example, may be 1 or more, 2 or more, 3 or more, 4 or more, 5 or more, 6 or more, or 7 or more, may be 50 or less, 40 or less, 30 or less, 25 or less, 20 or less, 15 or less, or 10 or less, or may be within a range defined as a combination thereof. The total number of the cell adhesion sequence contained in the repetitive structure may be, specifically, for example, 1 to 50, 3 to 30, or 5 to 15. When the protein of the present invention contains two or more cell adhesion sequences, the structures of the cell adhesion sequences each may be independently selected. That is, as the cell adhesion sequence, a single kind of cell adhesion sequence may be used, or two or more kinds of cell adhesion sequences may be used in combination.

The position of the cell adhesion sequence in the repeating unit is not particularly limited. Each cell adhesion sequence may have been inserted, for example, in the Ala-non-rich region, or in the linkage site between the Ala-rich region and the Ala-non-rich region. Examples of the Ala-non-rich region to be inserted with cell adhesion sequence include, for example, the regions of amino acid numbers of 62 to 91, 101 to 120, 130 to 151, 161 to 185, 195 to 218, 228 to 257, 267 to 291, 301 to 325, 335 to 369, 379 to 438, 448 to 497, and 507 to 536 within the amino acid sequence of SEQ ID NO: 2. Specific examples of the Ala-non-rich region to be inserted with cell adhesion sequence include, for example, the positions between amino acid numbers 109 and 110, 170 and 171, 203 and 204, 276 and 277, 310 and 311, 403 and 404, and 472 and 473 within the amino acid sequence of SEQ ID NO: 2. Examples of the linkage site between the Ala-rich region and the Ala-non-rich region to be inserted with the cell adhesion sequence include, for example, the sites between amino acid numbers 61 and 62, 91 and 92, 100 and 101, 120 and 121, 129 and 130, 151 and 152, 160 and 161, 185 and 186, 194 and 195, 218 and 219, 227 and 228, 257 and 258, 266 and 267, 291 and 292, 300 and 301, 325 and 326, 334 and 335, 369 and 370, 378 and 379, 438 and 439, 447 and 448, 497 and 498, and 506 and 507 within the amino acid sequence of SEQ ID NO: 2. The cell adhesion sequence may be further inserted into another region, as well as in the Ala-non-rich region and/or in the linkage site between the Ala-rich region and the Ala-non-rich region. Examples of the other region include the Ala-rich region, and N-terminal and C-terminal regions described below.

### < Other regions >

The protein of the present invention may further contain an N-terminal region before the first repeating unit, i.e. before the repetition structure. The amino acid sequence of the N-terminal region is not particularly limited. The length of the N-terminal region, for example, may be 1 residue or longer, 3 residues or longer, 5 residues or longer, 10 residues or longer, 30 residues or longer, 50 residues or longer, or 100 residues or longer, may be 200 residues or shorter, 150 residues or shorter, 100 residues or shorter, 80 residues or shorter, 60 residues or shorter, or 40 residues or shorter, or may be within a range defined as a non-contradictory combination thereof. The length of the N-terminal region may be, specifically, for example, 1 to 200 residues. The N-terminal region may comprise an amino acid sequence satisfying the requirement(s) of the Ala-non-rich region.

The protein of the present invention may further contain a C-terminal region after the last repeating unit, i.e. after the repetition structure. The amino acid sequence of the C-terminal region is not particularly limited. The length of the C-terminal region, for example, may be 1 residue or longer, 3 residues or longer, 5 residues or longer, 10 residues or longer, 30 residues or longer, 50 residues or longer, or 100 residues or longer, may be 200 residues or shorter, 150 residues or shorter, 100 residues or shorter, 80 residues or shorter, 60 residues or shorter, or 40 residues or shorter, or may be within a range defined as a non-contradictory combination thereof. The length of the C-terminal region may be, specifically, for example, 1 to 200 residues. The C-terminal region may comprise an amino acid sequence satisfying the requirement(s) of the Ala-rich region. Furthermore, the C-terminal region may comprise, for example, an amino acid sequence showing a homology of 90% or higher to an amino acid sequence around the C-terminus of the fibroin of spider at the C-terminus, in addition to the sequence similar to the repetitive sequence of fibroin. Examples of the amino acid sequence around the C-terminus of the fibroin of spider include, for example, the amino acid sequence of the C-terminus 50 residues of the fibroin of spider, the amino acid sequence of the C-terminus 50 residues of the same of which the C-terminus 20 residues are removed, and the amino acid sequence of the C-terminus 50 residues of the same of which the C-terminus 29 residues are removed. Specific examples of the amino acid sequence around the C-terminus of the fibroin of spider include, for example, the amino acid sequence of the positions 587 to 636 (C-terminus 50 residues), the amino acid sequence of the positions 587 to 616, and the amino acid sequence of the positions 587 to 607 of ADF3 of *Araneus diadematus* (partial; NCBIAAC47010.1 GI: 1263287). Specific examples of the protein of the present invention comprising the amino acid sequence of the positions 587 to 636 of ADF3 of *Araneus diadematus* (partial; NCBI AAC47010.1 GI: 1263287) include, for example, a protein having an amino acid sequence of SEQ ID NO: 4, 6, 27, 29, 36, 38, 40, 42, 44, 46, 48, 50, 52, or 54.

Furthermore, the N-terminal and C-terminal regions each may contain a functional sequence, such as a peptide tag, and a recognition sequence for a protease. As the functional sequence, one kind of functional sequence may be used, or two or more kinds of functional sequences may be used in combination. Specific examples of the peptide tag include an His tag, FLAG tag, GST tag, Myc tag, MBP (maltose binding protein), CBP (cellulose binding protein), TRX (thioredoxin), GFP (green fluorescent protein), HRP (horseradish peroxidase), ALP (alkaline phosphatase), and Fc region of antibody. The peptide tag can be used for, for example, detection and purification of the expressed protein. Specific examples of the recognition sequence for a protease include the recognition sequence for the HRV3C protease, the recognition sequence for the factor Xa protease, and the recognition sequence for the proTEV protease. The recognition sequence for a protease can be used for, for example, cleavage of the expressed protein. Specific examples of the protein of the present invention having an His-tag and an HRV3C protease recognition sequence at the N-terminus include, for example, a protein having an amino acid sequence of SEQ ID NO: 4, 6, 36, 42, 48, or 50.

### <1-2> Production of the protein of the present invention

The protein of the present invention can be produced by making a host having a gene encoding the protein of the present invention express encoding the protein of the present invention the gene. The phrase "having a gene encoding the protein of the present invention" is also referred to as "having the protein of the present invention". That is, for example, a host having a gene encoding the protein of the present invention is also referred to as "host having the protein of the present invention". In addition, an expression of a gene encoding the protein of the present invention is also referred to as "expression of the protein of the present invention". Alternatively, the protein of the present invention can also be produced by expressing the same using a cell-free protein synthesis system.

The host having the gene encoding the protein of the present invention can be obtained by introducing the gene encoding the protein of the present invention into an appropriate host.

The host to be introduced with the gene encoding the protein of the present invention is not particularly limited, so long as it is able to express the protein of the present invention. Examples of the host include, for example, bacteria, actinomycetes, yeast, fungi, plant cells, insect cells, and animal cells. Preferred examples of the host include microorganisms such as bacteria and yeast. More preferred examples of the host include bacteria. Examples of the bacteria include gram-negative bacteria and gram-positive bacteria. Examples of the gram-negative bacteria include, for example, bacteria belonging to the family *Enterobacteriaceae,* such as *Escherichia* bacteria, *Enterobacter* bacteria, and *Pantoea* bacteria. Examples of the gram-positive bacteria include *Bacillus* bacteria, and coryneform bacteria such as *Corynebacterium* bacteria. Examples of the *Escherichia* bacteria include, for example, *Escherichia coli.* Examples of the coryneform bacteria include, for example, *Corynebacterium glutamicum* and *Corynebacterium ammoniagenes* (*Corynebacterium stationis*). As the host, *Escherichia coli* can be especially preferably used. Specific examples of *Escherichia coli* include, for example, *Escherichia coli* K-12 strains such as the W3110 strain (ATCC 27325) and MG1655 strain (ATCC 47076); *Escherichia coli* K5 strain (ATCC 23506); *Escherichia coli* B strains such as the BL21(DE3) strain and a recA⁻ strain thereof BLR(DE3); and derivative strains of these.

These strains are available from, for example, the American Type Culture Collection (Address: 12301 Parklawn Drive, Rockville, Maryland 20852, P.O. Box 1549, Manassas, VA20108, United States of America). That is, registration numbers are given to the respective strains, and the strains can be ordered by using these registration numbers (refer to http://www.atcc.org/). The registration numbers of the strains are listed in the catalogue of the American Type Culture Collection. The BL21(DE3) strain is available from, for example, Life Technologies (product number C6000-03). The BLR(DE3) strain is available from, for example, Merck Millipore (product number 69053).

The gene encoding the protein of the present invention can be obtained by, for example, inserting a nucleotide sequence encoding the cell adhesion sequence into a fibroin-like protein gene. The fibroin-like protein gene can be obtained by cloning from an organism having the fibroin-like protein gene, such as a spider or a silkworm. For the cloning, a nucleic acid such as genomic DNA or cDNA containing the gene can be used. The obtained fibroin-like protein gene can be used as a base of the gene encoding the protein of the present invention as it is, or after being modified as required. Modification of a gene, such as insertion of the nucleotide sequence encoding the cell adhesion sequence, can be carried out by a known technique. For example, an objective mutation can be introduced into a target site of DNA by the site-specific mutation method. That is, for example, a coding region of a gene can be modified by the site-specific mutagenesis method so that a specific site of the encoded protein include substitution, deletion, insertion, and/or addition of amino acid residues. Examples of the site-specific mutagenesis method include a method of using PCR (Higuchi, R., 61, in PCR Technology, Erlich, H.A. Eds., Stockton Press (1989); Carter P., Meth., in Enzymol., 154, 382 (1987)), and a method of using a phage (Kramer, W. and Frits, H.J., Meth. in Enzymol., 154, 350 (1987); Kunkel, T.A. et al., Meth. in Enzymol., 154, 367 (1987)). The gene encoding the protein of the present invention can also be obtained by, for example, chemical synthesis (Gene, 60(1), 115-127 (1987)).

Methods for introducing the gene encoding the protein of the present invention into a host are not particularly limited. It is sufficient that the gene encoding the protein of the present invention is harbored in the host in such a manner that it can be expressed under control of a promoter that functions in the host. In the host, the gene encoding the protein of the present invention may exist on a vector autonomously replicable out of the chromosome such as a plasmid, or may be introduced into the chromosome. The host may have only one copy of the gene encoding the protein of the present invention, or may have two or more copies of the gene encoding the protein of the present invention. The host may have only one kind of gene encoding the protein of the present invention, or may have two or more kinds of genes encoding the protein of the present invention.

The promoter for expressing the gene encoding the protein of the present invention is not particularly limited, so long as it is a promoter that functions in the host. The term "promoter that functions in a host" refers to a promoter that shows a promoter activity in the host. The promoter may be a promoter derived from the host, or a heterologous promoter. The promoter may be the native promoter of the gene encoding the protein of the present invention, or may be a promoter of another gene. The promoter may be a promoter stronger than the native promoter of the gene encoding the protein of the present invention. Specific examples of strong promoters that function in *Enterobacteriaceae* bacteria, such as *Escherichia coli,* include, for example, T7 promoter, *trp* promoter, *trc* promoter, *lac* promoter, *tac* promoter, *tet* promoter, *araBAD* promoter, *rpoH* promoter, PR promoter, and PL promoter. Examples of strong promoters that function in coryneform bacteria include the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as *lac* promoter, *tac* promoter, and *trc* promoter. Further, as the stronger promoter, a highly-active type of an existing promoter may also be obtained by using various reporter genes. For example, by making the -35 and -10 regions in a promoter region closer to the consensus sequence, the activity of the promoter can be enhanced (WO00/18935). Examples of highly active-type promoters include various tac-like promoters (Katashkina JI et al., Russian Federation Patent Application No. 2006134574) and *pnlp8* promoter (WO2010/027045). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

A terminator for termination of gene transcription may be located downstream of the gene encoding the protein of the present invention. The terminator is not particularly limited so long as it functions in the host. The terminator may be a terminator derived from the host, or a heterogenous terminator. The terminator may be the native terminator of the gene encoding the protein of the present invention, or a terminator of another gene. Specific examples of the terminator include, for example, T7 terminator, T4 terminator, fd phage terminator, *tet* terminator, and *trpA* terminator.

The gene encoding the protein of the present invention can be introduced into a host, for example, by using a vector containing the gene. A vector containing the gene encoding the protein of the present invention is also referred to as expression vector or recombinant vector for the gene encoding the protein of the present invention. The expression vector for the gene encoding the protein of the present invention can be constructed by, for example, ligating a DNA fragment containing the gene encoding the protein of the present invention with a vector that functions in the host. By transforming the host with the expression vector for the gene encoding the protein of the present invention, a transformant into which the vector has been introduced is obtained, i.e. the gene can be introduced into the host. As the vector, a vector autonomously replicable in the cell of the host can be used. The vector is preferably a multi-copy vector. Further, the vector preferably has a marker such as an antibiotic resistance gene for selection of transformant. Further, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vectors autonomously replicable in *Enterobacteriaceae* bacteria such as *Escherichia coli* include, for example, pUC19, pUC18, pHSG299, pHSG399, pHSG398, pBR322, pSTV29 (all of these are available from Takara Bio), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pACYC, and the broad host spectrum vector RSF1010. Specific examples of vectors autonomously replicable in coryneform bacteria include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 described in Japanese Patent Laid-open (Kokai) No. 3-210184; plasmids pCRY21, pCRY2KE, pCRY2KX, pCRY31, pCRY3KE, and pCRY3KX described in Japanese Patent Laid-open (Kokai) No. 2-72876 and U.S. Patent No. 5,185,262; plasmids pCRY2 and pCRY3 described in Japanese Patent Laid-open (Kokai) No. 1-191686; pAJ655, pAJ611, and pAJ1844 described in Japanese Patent Laid-open (Kokai) No. 58-192900; pCG1 described in Japanese Patent Laid-open (Kokai) No. 57-134500; pCG2 described in Japanese Patent Laid-open (Kokai) No. 58-35197; and pCG4 and pCG11 described in Japanese Patent Laid-open (Kokai) No. 57-183799. When the expression vector is constructed, for example, the gene encoding the protein of the present invention having a native promoter region as it is may be incorporated into a vector, a coding region of the protein of the present invention ligated downstream from such a promoter as mentioned above may be incorporated into a vector, or a coding region of the protein of the present invention may be incorporated into a vector downstream from a promoter originally existing in the vector.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

The gene encoding the protein of the present invention can also be introduced into, for example, a chromosome of a host. A gene can be introduced into a chromosome, for example, by using homologous recombination (Miller, J.H., Experiments in Molecular Genetics, 1972, Cold Spring Harbor Laboratory). Examples of gene transfer method utilizing homologous recombination include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy of the gene may be introduced, or two or more copies of the gene may be introduced. For example, by performing homologous recombination using a sequence which is present in multiple copies on a chromosome as a target, multiple copies of a gene can be introduced into the chromosome. Examples of such a sequence which is present in multiple copies on a chromosome include repetitive DNAs, and inverted repeats located at the both ends of a transposon. Homologous recombination may also be performed by using an appropriate sequence on a chromosome such as a gene unnecessary for carrying out the present invention as a target. Further, a gene can also be randomly introduced into a chromosome by using a transposon or Mini-Mu (Japanese Patent Laid-open (Kokai) No. 2-109985, U.S. Patent No. 5,882,888, EP 805867 B1). When the gene is introduced into a chromosome, for example, the gene encoding the protein of the present invention having a native promoter region as it is may be incorporated into a chromosome, a coding region of the protein of the present invention ligated downstream from such a promoter as mentioned above may be incorporated into a chromosome, or a coding region of the protein of the present invention may be incorporated into a chromosome downstream from a promoter originally existing in the chromosome.

Introduction of a gene into a chromosome can be confirmed by, for example, Southern hybridization using a probe having a sequence complementary to the entire gene or a part thereof, or PCR using primers prepared on the basis of the nucleotide sequence of the gene.

Methods for transformation are not particularly limited, and conventionally known methods can be used. Examples of transformation method include, for example, a method of treating recipient cells with calcium chloride so as to increase permeability thereof for DNA, which has been reported for the *Escherichia coli* K-12 strain (Mandel, M. and Higa, A., J. Mol. Biol., 1970, 53, 159-162), a method of preparing competent cells from cells which are in the growth phase, followed by introducing DNA, which has been reported for *Bacillus subtilis* (Duncan, C.H., Wilson, G.A. and Young, F.E., 1977, Gene, 1:153-167), and so forth. Alternatively, as the transformation method, there can also be used a method of making DNA-recipient cells into protoplasts or spheroplasts, which can easily take up recombinant DNA, and then introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, GR., 1978, Proc. Natl. Acad. Sci. USA, 75:1929-1933). Further, as the transformation method, the electric pulse method reported for coryneform bacteria (Japanese Patent Laid-open (Kokai) No. 2-207791) can also be used.

By culturing the host having the gene encoding the protein of the present invention, the protein of the present invention can be expressed. During the culture, induction of gene expression is carried out as required. Conditions for culture of the host and induction of gene expression may be appropriately chosen depending on various conditions such as the type of marker, the type of promoter, and the type of the host. The culture medium used for the culture is not be particularly limited, so long as the host can proliferate in the culture medium and express the protein of the present invention. As the culture medium, for example, a usual culture medium that contains a carbon source, nitrogen source, sulfur source, inorganic ions, and other organic components as required can be used.

Examples of the carbon source include saccharides such as glucose, fructose, sucrose, molasses, and starch hydrolysate, alcohols such as glycerol and ethanol, and organic acids such as fumaric acid, citric acid, and succinic acid.

Examples of the nitrogen source include inorganic ammonium salts such as ammonium sulfate, ammonium chloride, and ammonium phosphate, organic nitrogen such as soybean hydrolysate, ammonia gas, and aqueous ammonia.

Examples of the sulfur source include inorganic sulfur compounds, such as sulfates, sulfites, sulfides, hyposulfites, and thiosulfates.

Examples of the inorganic ions include calcium ion, magnesium ion, manganese ion, potassium ion, iron ion, and phosphoric acid ion.

Examples of the other organic components include organic trace amount nutrients. Examples of the organic trace amount nutrients include required substances such as vitamin B₁, yeast extract containing such substances, and so forth.

The culture temperature may be, for example, 20 to 45°C, preferably 24 to 45°C, more preferably 30 to 37°C. The culture may preferably be carried out as aerobic culture. Upon the aeration culture, the oxygen concentration may be adjusted to, for example, 5 to 50%, preferably 20 to 40%, with respect to the saturated concentration. The pH of the culture medium may preferably be 5 to 9. For adjusting pH, inorganic or organic acidic or alkaline substances such as calcium carbonate, ammonia gas, and aqueous ammonia can be used. The culture period may be, for example, 10 to 120 hours.

Particularly when using *Escherichia coli* as a host, it is expected that the protein of the present invention can be efficiently produced by reducing accumulation of an organic acid at the time of inducing the expression of the protein of the present invention (WO2015/178465), or by reducing cell growth after inducing the expression of the protein of the present invention (WO2015/178466).

By culturing the host having the gene encoding the protein of the present invention as described above, the protein of the present invention is accumulated in the culture medium and/or cells of the host. The protein of the present invention can be accumulated as, for example, inclusion bodies in the cells.

The protein of the present invention can be collected and quantified by, for example, known methods for collecting and quantifying a heterogeneously expressed protein (see, for example, "Lecture of New Chemical Experiments, Protein VI, Synthesis and Expression", Ed. By Japanese Biochemical Society, Tokyo Kagaku Dojin, 1992, pp.183-184).

Hereinafter, a procedure for collecting and quantifying the protein of the present invention will be exemplified for a case where the protein is accumulated as inclusion bodies in the cells. First, the cells are collected from the culture medium by centrifugation, and then suspended in a buffer. The cell suspension is subjected to such a treatment as ultrasonication or French press to disrupt the cells. Before disrupting the cells, lysozyme may be added to the cell suspension at a final concentration of 0 to 200 mg/l, and the suspension may be incubated on ice for 30 minutes to 20 hours. Then, an insoluble fraction is obtained from the disrupted cell suspension as precipitates by low speed centrifugation (6000 to 15000 rpm, 5 to 10 minutes, 4°C). The insoluble fraction is appropriately washed with a buffer as required. The number of times of washing is not be particularly limited, and may be, for example, once, twice, or 3 times or more. By suspending the insoluble fraction in a buffer, a suspension of the protein of the present invention is obtained. As the buffer for suspending the cells or the protein of the present invention, a buffer in which the protein of the present invention shows a low solubility can be preferably used. Examples of such a buffer include, for example, a Tris-HCl buffer containing NaCl. The pH of the buffer may be, for example, usually 4 to 12, preferably 6 to 9. A solution of the protein of the present invention can also be obtained by dissolving the insoluble fraction in an SDS solution or urea solution. The protein of the present invention collected may contain such components as bacterial cells, culture medium components, and bacterial metabolic by-products, in addition to the protein of the present invention. The protein of the present invention may be purified to a desired degree. The protein of the present invention can be purified by known methods used for purification of proteins. Examples of such methods include, for example, ammonium sulfate fractionation, ion exchange chromatography, hydrophobic chromatography, affinity chromatography, gel filtration chromatography, isoelectric precipitation, and dialysis. These methods may be independently used, or may be used in an appropriate combination. The amount of the protein of the present invention can be determined, for example, as follows: a sample containing the protein of the present invention such as suspension or solution is subjected to SDS-PAGE, and stained, and then, the amount of the protein of the present invention can be determined on the basis of intensity of a band at the position corresponding to the molecular weight of the objective protein of the present invention. The staining can be performed by CBB staining, fluorescence staining, silver staining, or the like. For the quantification, proteins of known concentrations can be used as the standards. Examples of such proteins include, for example, albumin, and the protein of the present invention of which the concentration has been separately determined.

The intended use of the protein of the present invention is not particularly limited. The protein of the present invention may be used for, for example, medical use or research use. The protein of the present invention shows a scaffold function for a cell, such as a stem cell. Hence, the protein of the present invention may be used for, for example, culturing a cell, such as a stem cell. The protein of the present invention may be used as, specifically, for example, a scaffold for cell culture. The protein of the present invention may be used as, for example, a component of an apparatus such as an apparatus for cell culture. The protein of the present invention may be used in such a manner that, specifically, for example, a surface of an apparatus such as a cell culture vessel is coated with the protein. The descriptions below concerning the apparatus of the present invention and the method of the present invention can be applied *mutatis mutandis* to an apparatus and cell culture.

The protein of the present invention can be used, for example, in combination with another protein (i.e. a protein other than the protein of the present invention). That is, the protein of the present invention can be used, for example, in combination with another protein for such use as described above.

The other protein is not particularly limited, so long as the effect provided by the protein of the present invention is not degraded. The other protein may be, for example, a protein that improves cultivation of a cell, such as a stem cell, when using the other protein in combination with the protein of the present invention as compared with when using the protein of the present invention solely. Examples of improvement in cultivation of a cell include, for example, improvement in proliferation of a cell.

Examples of the other protein include, for example, the proteins (1) to (8) shown below:
(1) a protein that stabilizes a component in a culture medium;
(2) a protein comprising an amino acid sequence that stabilizes a component in a culture medium;
(3) a protein having a cell adhesion ability (cell adhesion protein);
(4) a protein comprising an amino acid sequence having a cell adhesion ability;
(5) a protein that promotes proliferation of a cell;
(6) a protein comprising an amino acid sequence that promotes proliferation of a cell;
(7) a protein that binds to any one of the proteins (1) to (6);
(8) a protein comprising an amino acid sequence that binds to any one of the proteins (1) to (6).

Examples of the protein (1) include, for example, heparin. Heparin has been known to stabilize a basic fibroblast growth factor (bFGF) in a culture medium. That is, examples of the component in a culture medium include, for example, bFGF. Examples of the protein (3) or (5) include, for example, proteins having a scaffold function for a cell, such as fibronectin, vitronectin, collagen, osteopontin, and laminin. Examples of the protein (8) include, for example, vitronectin. Vitronectin has been known to have a heparin-binding sequence.

Examples of the protein (2), (4), (6), or (8) include, for example, fibroin-like proteins and proteins having a scaffold function for a cell, provided that the proteins have been inserted with the amino acid sequence recited in (2), (4), (6), or (8), respectively. The aforementioned descriptions concerning insertion of the cell adhesion sequence in the protein of the present invention can be applied mutatis mutandis to insertion of such an amino acid sequence. Examples of the amino acid sequence recited in (2), (4), (6), or (8) include, for example, a partial sequence of the protein (1), (3), (5), or (7), respectively, provided that the partial sequence has the corresponding function. Specific examples of the amino acid sequence recited in (8) include, for example, GKKQRFRHRNRKG (SEQ ID NO: 34). The amino acid sequence of SEQ ID NO: 34 is an amino acid sequence consisting of the heparin-binding sequence of vitronectin added with G at the both termini. Specific examples of proteins having a scaffold function for a cell include, for example, proteins exemplified as the protein (3) or (5), such as fibronectin, vitronectin, collagen, osteopontin, and laminin. That is, the protein having a scaffold function for a cell *per se* may typically be the protein (3) or (5).

Specific examples of the other protein include, for example, a protein having the amino acid sequence shown in SEQ ID NO: 50.

The other protein or a component thereof may also be a variant of any of the amino acid sequences exemplified above or other known amino acid sequences, so long as an intended effect is obtained. The aforementioned descriptions concerning conservative variants of the fibroin-like protein can be applied *mutatis mutandis* to variants of the other protein or a component thereof.

### <2> Composition of the present invention

The composition of the present invention is a composition containing the protein of the present invention. The intended use of the composition of the present invention is not particularly limited. The composition of the present invention may be used for, for example, medical use or research use. That is, the composition of the present invention may be, for example, a composition for medical or research use. The composition of the present invention may be used for, specifically, for example, culturing a cell, such as a stem cell. That is, the composition of the present invention may be, specifically, for example, a composition for culturing a cell. The composition of the present invention may be, more specifically, for example, a cell scaffold material. The term "cell scaffold material" refers to a structure that functions as a scaffold for a cell, specifically, a structure that functions as a scaffold for a cell when culturing the cell. The cell scaffold material can be used for, for example, culturing a cell. That is, a cell can be cultured using the cell scaffold material as a scaffold. A resulting product obtained by culturing a cell using the cell scaffold material, such as cultured cells, cell sheet, tissue, and organ, can be used for a desired use such as medical treatment, for example, after isolation from the cell scaffold material, or together with the cell scaffold material. The cell scaffold material may also be implanted into a living body, and thereby used for culturing a cell in vivo, such as tissue formation mediated by adhesion and proliferation of cells. The composition of the present invention may also be, specifically, for example, a coating agent for an apparatus such as a cell culture vessel. The descriptions below concerning the apparatus of the present invention and the method of the present invention can be applied *mutatis mutandis* to an apparatus and cell culture.

The composition of the present invention may or may not consist of the protein of the present invention. The composition of the present invention may consist of a combination of the protein of the present invention and another ingredient. That is, the protein of the present invention can be used, solely or in combination with another ingredient, as the composition of the present invention. The other ingredient is not particularly limited, so long as it has acceptable properties depending on the use mode of the composition of the present invention. Examples of the other ingredient include, for example, ingredients blended and used in pharmaceuticals or reagents. Specific examples of such ingredients include, for example, additives such as an excipient, binder, disintegrant, lubricant, stabilizer, flavoring agent, odor improving agent, perfume, diluent, and surfactant. Examples of the other ingredient also include, for example, another protein (i.e. a protein other than the protein of the present invention). The other protein is as described above.

The concentration (contained amount) of the protein of the present invention in the composition of the present invention is not particularly limited. The concentration of the protein of the present invention in the composition of the present invention can be appropriately chosen according to various conditions such as use mode of the composition of the present invention. The concentration of the protein of the present invention in the composition of the present invention, for example, may be 0.01%(w/w) or more, 0.1%(w/w) or more, 1%(w/w) or more, 5%(w/w) or more, or 10%(w/w) or more, may be 100%(w/w) or less, 99.9%(w/w) or less, 70%(w/w) or less, 50%(w/w) or less, 30%(w/w) or less, 10%(w/w) or less, 5%(w/w) or less, or 1%(w/w) or less, or may be within a range defined as a non-contradictory combination thereof.

The form of the composition of the present invention is not particularly limited. The form of the composition of the present invention can be appropriately chosen according to various conditions such as use mode of the composition of the present invention. The composition of the present invention may be formulated in a desired form. Specific examples of the form of the composition of the present invention include, for example, liquid (such as solution or suspension), paste, powder, granule, sphere, tablet, foam, sponge, fiber, film, sheet, membrane, mesh, and tube.

The composition of the present invention may be used for a desired use such as coating of an apparatus, for example, as it is, or after being diluted, dispersed, dissolved, or the like in a medium such as water or a aqueous buffer as required. Needless to say, the resulting product obtained by such dilution, dispersion, dissolution, or the like as described above is still included in the scope of the composition of the present invention.

### <3> Apparatus of the present invention

The apparatus of the present invention is an apparatus comprising the protein of the present invention. The intended use of the apparatus of the present invention is not particularly limited. The apparatus of the present invention may be used for, for example, medical use or research use. That is, the apparatus of the present invention may be, for example, an apparatus for medical or research use. The apparatus of the present invention may be used for, specifically, for example, culturing a cell, such as a stem cell. That is, the apparatus of the present invention may be, specifically, for example, an apparatus for culturing a cell. Examples of the apparatus for culturing a cell include, for example, a cell culture vessel and a cell scaffold material. The apparatus of the present invention as a cell culture vessel is also referred to as "cell culture vessel of the present invention". The apparatus of the present invention as a cell scaffold material is also referred to as "cell scaffold material of the present invention". It is sufficient that the apparatus of the present invention is an apparatus comprising the protein of the present invention in such a manner that the function of the protein of the present invention can be exercised. The apparatus of the present invention may comprise the protein of the present invention on the surface thereof. That is, the apparatus of the present invention may be, specifically, for example, an apparatus of which the surface is coated with the protein of the present invention. In addition, when the protein of the present invention or the composition of the present invention is configured as a form usable as an apparatus as it is, the protein of the present invention or the composition of the present invention may be used as the apparatus of the present invention as it is. Specifically, for example, when the composition of the present invention is configured as a cell scaffold material, the composition of the present invention may be used as the cell scaffold material of the present invention as it is. The descriptions below concerning the method of the present invention can be applied *mutatis mutandis* to cell culture.

The apparatus of the present invention may further comprise another protein (i.e. a protein other than the protein of the present invention). The other protein is as described above. The aforementioned descriptions concerning the use mode of the protein of the present invention in the apparatus of the present invention can be applied *mutatis mutandis* to the use mode of the other protein in the apparatus of the present invention. For example, the apparatus of the present invention may be an apparatus of which the surface is coated with the other protein as well as the protein of the present invention.

Hereinafter, although the apparatus of the present invention is explained in reference mainly to a culture vessel, such explanations can be applied *mutatis mutandis* to other apparatuses. That is, the explanations below can be applied to other apparatuses as it is, or after being modified as required according to various conditions such as the embodiment and intended use of the apparatuses.

The culture vessel is not particularly limited, so long as it is made of a material and has a form, which material and form are usable for culturing a cell. Specific examples of the form of the culture vessel include, for example, dish, multi-well plate, flask, cell insert, and membrane. Specific examples of the material of the culture vessel include, for example, plastics such as polyethylene terephthalate (PET), polystyrene (PS), polycarbonate (PC), triacetyl cellulose (TAC), polyimide (PI), nylon (Ny), low-density polyethylene (LDPE), medium-density polyethylene (MDPE), vinyl chloride, vinylidene chloride, polyphenylene sulfide, polyether sulfone, polyethylene naphthalate, polypropylene, and urethane acrylate; biodegradable polymers such as polylactic acid, polyglycolic acid, polycaprolactone, and copolymers thereof; glasses such as glass and modified glass; ceramics; metals; cellulose; and other various macromolecular compounds.

The culture vessel of the present invention comprises the protein of the present invention at least on a cell culture surface thereof. That is, the culture vessel of the present invention may have, for example, a cell culture surface coated with the protein of the present invention. The term "cell culture surface" refers to a surface on which a cell is cultured, specifically, a portion that can be contact with a cell upon culturing the cell. The cell culture surface of the culture vessel of the present invention may or may not be wholly coated with the protein of the present invention, so long as a cell, such as a stem cell, can be cultured using the culture vessel. The ratio of the area of the portion coated with the protein of the present invention with respect to the total area of the cell culture surface of the culture vessel may be, for example, 50% or more, 70% or more, 80% or more, 90% or more, or 95% or more. It is usually preferred that the cell culture surface of the culture vessel is wholly coated with the protein of the present invention. For example, when the culture vessel is a dish-form vessel, it is preferred that the bottom surface is wholly coated with the protein of the present invention.

Methods for coating a cell culture surface of a culture vessel are not particularly limited. The cell culture surface of the culture vessel can be coated with the protein of the present invention by, for example, known methods for coating a surface of a culture vessel with a functional material. For example, by applying a solution containing the protein of the present invention to a cell culture surface of a culture vessel, the cell culture surface of the culture vessel can be coated with the protein of the present invention. Specifically, for example, by applying a solution containing the protein of the present invention to a cell culture surface of a culture vessel and drying the solution (i.e. volatilizing the solvent thereof), the cell culture surface of the culture vessel can be coated with the protein of the present invention. Also, specifically, for example, by applying a solution containing the protein of the present invention to a cell culture surface of a culture vessel in combination with a subsequent lapse of time (hereinafter, also referred to as "coating period"), the cell culture surface of the culture vessel can be coated with the protein of the present invention. The coating period is not particularly limited, so long as the cell culture surface of the culture vessel can be coated with the protein of the present invention to a desired extent. The coating period, for example, may be 30 minutes or longer, 1 hour or longer, 6 hours or longer, 12 hours or longer, or 18 hours or longer, may be 72 hours or shorter, 48 hours or shorter, or 30 hours or shorter, or may be within a range defined as a combination thereof. After the lapse of the coating period, the remaining solution may be removed or dried as required. The culture vessel may be sterilized as required. The culture vessel may be sterilized, for example, after the cell culture surface of the culture vessel is coated with the protein of the present invention. For sterilization, for example, an alcohol such as ethanol and 2-propanol can be used. The alcohol may be used as, for example, an aqueous alcohol such as 70% ethanol. Sterilization can be carried out by, for example, applying an alcohol to the cell culture surface, or immersing the culture vessel in an alcohol. Although sterilization can be carried out by using an alcohol as described above, just in case, a solution containing the protein of the present invention may be applied to the cell culture surface of the culture vessel after sterilization of the solution by filter sterilization or the like, and subsequence operation(s) may be aseptically carried out. For filter sterilization, for example, a filter such as Millex of Merck Millipore can be used. Operation(s) may be aseptically carried out as required. When a culture vessel is coated with both the protein of the present invention and another protein, coating with the protein of the present invention and coating with the other protein may be simultaneously carried out, or may be separately carried out. When coating with the protein of the present invention and coating with the other protein are separately carried out, the order of them is not particularly limited. The culture vessel of the protein of the present invention may be used immediately after production (i.e. immediately after coating with the protein of the present invention), or after being stored as required. The culture vessel of the protein of the present invention is effective for culturing a cell, such as a stem cell, even when the culture vessel is not used immediately after production. The temperature of storage may be, for example, 0 to 50°C, 0 to 40°C, 0 to 25°C, or 0 to 10°C. The period for storage may be, for example, 1 year or shorter, 6 months or shorter, or 1 month or shorter. The culture vessel of the protein of the present invention may be stored in a dry state or a wet state. The culture vessel of the protein of the present invention may be stored, for example, in a state where the cell culture surface is wet with a buffer such as PBS.

The coating amount of the protein of the present invention to the surface of the culture vessel is not particularly limited, so long as a cell, such as a stem cell, can be cultured using the culture vessel of the present invention. The coating amount of the protein of the present invention to the surface of the culture vessel, for example, may be 0.1 µg/cm² or more, 0.2 µg/cm² or more, 0.4 µg/cm² or more, 1 µg/cm² or more, or 5 µg/cm² or more, may be 500 µg/cm² or less, 250 µg/cm² or less, 100 µg/cm² or less, or 50 µg/cm² or less, or may be within a range defined as a combination thereof. The coating amount of the protein of the present invention to the surface of the culture vessel may be, specifically, for example, 0.1 µg/cm² to 500 µg/cm², preferably 0.2 µg/cm² to 250 µg/cm², more preferably 0.4 µg/cm² to 100 µg/cm².

Similarly, the cell scaffold material of the present invention can be appropriately obtained. The cell scaffold material is not particularly limited, so long as it is made of a material and has a form, which material and form are usable for culturing a cell. The cell scaffold material may have, for example, a form depending on the form of an objective resulting product obtained by culturing a cell, such as cell sheet, tissue, and organ. The cell scaffold material may be configured as a form having a cavity, such as a porous form, so that the inside of the cell scaffold material can be filled with cells. Preferred examples of the material of the cell scaffold material include, for example, bioabsorbable macromolecular compounds from the point of view that they are suitable for implant into a living body. Specific examples of bioabsorbable macromolecular compounds include, for example, polylactic acid, polyglycolic acid, copolymer of lactic acid and glycolic acid, polycaprolactone, collagen, gelatin, glycosaminoglycan, chitin, chitosan, hyaluronic acid, and polypeptide. The cell scaffold material of the present invention can be obtained by, for example, coating the surface (specifically, the cell culture surface) of the cell scaffold material with the protein of the present invention. The term "surface of a cell scaffold material" used when the cell scaffold material is configured as a form having a cavity includes the surface of the cavity. Also, for example, as described above, the composition of the present invention configured as a cell scaffold material may be used as the cell scaffold material of the present invention as it is.

### <4> Method of the present invention

A cell, such as a stem cell, can be efficiently cultured using the protein of the present invention. Specifically, a cell, such as a stem cell, can be efficiently cultured using an apparatus comprising the protein of the present invention (i.e. the apparatus of the present invention). More specifically, a cell, such as a stem cell, can be efficiently cultured using a culture apparatus comprising the protein of the present invention, such as the culture vessel of the present invention and the cell scaffold material of the present invention. That is, the present invention provides a method for culturing a cell, the method comprising culturing a cell using the apparatus of the present invention, such as the culture vessel of the present invention and the cell scaffold material of the present invention. This method is also referred to as "the method of the present invention". The protein of the present invention is highly effective especially for proliferation of a stem cell such as an iPS cell. Hence, the protein of the present invention and an apparatus for cell culture, such as a cell culture vessel and a cell scaffold material, comprising the same can be used for, for example, proliferation of a stem cell such as an iPS cell. Furthermore, the protein of the present invention and an apparatus for cell culture, such as a cell culture vessel and a cell scaffold material, comprising the same can be used not only for proliferation of a stem cell such as an iPS cell, but also for, for example, establishment of a stem cell such as an iPS cell, which is a prior stage of proliferation of the stem cell, and differentiation of a stem cell such as an iPS cell, which is a posterior stage of proliferation of the stem cell. That is, in the present invention, the term "culturing a cell" include not only proliferation of a cell, but also, for example, establishment of a cell having differentiation potency, such as a stem cell and a precursor cell, and differentiation from a cell having differentiation potency, such as a stem cell and a precursor cell, to another cell. Specifically, the term "culturing a stem cell" include not only proliferation of a stem cell, but also, for example, establishment of a stem cell (e.g. establishment of a totipotent stem cell or a pluripotent stem cell, such as an iPS cell and an ES cell) and differentiation of a stem cell (e.g. differentiation from a stem cell, such as an iPS cell and an ES cell, to another stem cell, a precursor cell, or a mature cell). According to the method of the present invention, cultured cells are obtained. That is, an embodiment of the method of the present invention is a method for producing a cultured cell, the method comprising culturing a cell using the apparatus of the present invention, such as the culture vessel of the present invention and the cell scaffold material of the present invention. Cultured cells obtained by the method of the present invention may or may not be differentiated. That is, cultured cells obtained by the method of the present invention, for example, may have been kept in undifferentiated state, or may have acquired undifferentiated state. Cultured cells obtained by the method of the present invention may each be, for example, a cell having differentiation potency, such as a stem cell and a precursor cell, or may be a differentiated cell, such as a precursor cell or a mature cell. Cultured cells obtained by the method of the present invention, for example, may be free cells (i.e. individual independent cells), or may be organized cells, such as a cell sheet, tissue, and organ.

The type of cell to be cultured is not particularly limited. The method of the present invention can be suitably used especially for culturing a stem cell. Specific examples of the stem cell include, for example, an embryonic stem cell (ES cell), an induced pluripotent stem cell (iPS cell), a pluripotent germ stem cell (mGS cell), a neural stem cell, a mesenchymal stem cell, and a hematopoietic stem cell. More specific examples of the stem cell include, for example, an embryonic stem cell (ES cell) and an induced pluripotent stem cell (iPS cell). The cell may be derived from any biological tissue. Examples of the biological tissue include skin, cornea, liver, digestive organ, mammary gland, prostate, hair root, trachea, and oral mucosa. The organism from which the cell is derived is not particularly limited, and may be appropriately chosen depending on the use purpose or the like. Examples of the organism from which the cell is derived include, for example, a mammal. Specific examples of the mammal include, for example, human, rat, mouse, guinea pig, marmoset, rabbit, dog, cat, sheep, pig, and chimpanzee. The mammal may be immunodeficient. When using cultured cells for medical treatment of human, for example, a mammal selected from human, pig, and chimpanzee is preferred for the organism from which the cell is derived. In the method of the present invention, a single kind of cell may be cultured, or two or more kinds of cells may be co-cultured.

Culture conditions are not particularly limited, so long as an intended culture result (e.g. proliferation of a cell, establishment of a stem cell, and differentiation of a cell) is obtained. Culture conditions may be identical to, for example, those usually used for culturing a cell, such as an iPS cell and an ES cell, except that the apparatus of the present invention, such as the culture vessel of the present invention and the cell scaffold material of the present invention, is used. Culture conditions can be appropriately chosen depending on various conditions such as the embodiment of the apparatus of the present invention, the type of the cell, and the type of the organism from which the cell is derived.

The composition for the culture medium can be appropriately chosen depending on various conditions such as the properties of the cell to be cultured. Examples of culture medium components include, for example, carbon sources such as glucose, amino acids, vitamins, phosphate salts, buffering agents, growth factors, serum, and serum albumin. Specific examples of usual culture media used for culturing a cell include, for example, α-MEM, DMEM, and KCM. In addition, specific examples of usual culture media used for culturing an embryonic stem cell (ES cell) or an induced pluripotent stem cell (iPS cell) include, for example, Essential 8 (Invitrogen), StemFit(R) medium (Ajinomoto), and other culture media used in the Examples section.

According to the method of the present invention, a cell can be efficiently cultured without using a feeder cell or another cell adhesion protein (i.e. a cell adhesion protein other than the protein of the present invention). However, this fact does not exclude use of a feeder cell or another cell adhesion protein. That is, in the method of the present invention, a feeder cell or another cell adhesion protein (i.e. a cell adhesion protein other than the protein of the present invention) may or may not be used. In the method of the present invention, it is preferred that the use amount of a feeder cell or another cell adhesion protein is reduced as compared with the amount usually used for culturing a cell. In the method of the present invention, it is more preferred that a feeder cell or another cell adhesion protein is not used.

The number of cells inoculated at the start of culture, for example, may be 1×10³ cells/cm² or more, 1×10⁴ cells/cm² or more, or 2×10⁴ cells/cm² or more, may be 1×10⁶ cells/cm² or less, 1×10⁵ cells/cm² or less, or 5×10⁴ cells/cm² or less, or may be within a range defined as a combination thereof. The culture temperature may be, for example, 36 to 38°C, preferably about 37°C. The culture pH may be, for example, pH6.8 to 7.2. The gas phase during culture may be, for example, a mixed gas of air and carbon dioxide. Specifically, for example, culture may be carried out under an atmosphere of 5% of carbon dioxide gas and 95% of air. The culture period may be, for example, 5 to 30 days, preferably 7 to 16 days. The culture medium may be exchanged during the culture.

In the method of the present invention, the protein of the present invention can be used in combination with another protein (i.e. a protein other than the protein of the present invention). The other protein is as described above. For example, by using an apparatus comprising the protein of the present invention (i.e. the apparatus of the present invention), which apparatus further comprises the other protein, the protein of the present invention can be used in combination with the other protein. Also, for example, by using a culture medium containing the other protein, the protein of the present invention can be used in combination with the other protein.

By carrying out cell culture as described above, cultured cells can be obtained.

### Examples

Hereinafter, the present invention will be more specifically explained with reference to examples.

### Example 1: Preparation of cell adhesion sequence-inserted fibroin-like protein (1)

### <1> Construction of expression strain for cell adhesion sequence-inserted fibroin-like protein

A plasmid pET22b-ADF3 for expressing a gene encoding a fibroin-like protein ADF3 was constructed by digesting pET22b(+) vector (Novagen) with restriction enzymes Ndel and EcoRI, and inserting thereto a totally-synthesized fibroin-like protein gene ADF3 using DNA Ligation Kit (TaKaRa). The nucleotide sequence of the ADF3 gene is shown in SEQ ID NO: 1, and the amino acid sequence of the fibroin-like protein ADF3 encoded by this gene is shown in SEQ ID NO: 2. The ADF3 protein shown in SEQ ID NO: 2 has an His-tag and an HRV3C protease recognition sequence at the N-terminus.

A plasmid pET22b-ADF3RGDS#2 for expressing a gene encoding a cell adhesion sequence-inserted fibroin-like protein ADF3RGDS#2 was constructed by digesting pET22b(+) vector (Novagen) with restriction enzymes Ndel and EcoRI, and inserting thereto a totally-synthesized fibroin-like protein gene ADF3RGDS#2 using DNA Ligation Kit (TaKaRa). The nucleotide sequence of the ADF3RGDS#2 gene is shown in SEQ ID NO: 3, and the amino acid sequence of the fibroin-like protein ADF3RGDS#2 encoded by this gene is shown in SEQ ID NO: 4. The nucleotide sequence of positions 8 to 2200 of SEQ ID NO: 3 encodes the amino acid sequence shown in SEQ ID NO: 4. ADF3RGDS#2 consists of the ADF3 protein shown in SEQ ID NO: 2 except that 7 of 12 Ala-non-rich regions therein were each inserted with one unit (i.e. 7 units in total) of a cell adhesion sequence "GRGDSP" (SEQ ID NO: 22).

A plasmid pET22b-ADF3RGDS#3 for expressing a gene encoding a cell adhesion sequence-inserted fibroin-like protein ADF3RGDS#3 was constructed by digesting pET22b(+) vector (Novagen) with restriction enzymes Ndel and EcoRI, and inserting thereto a totally-synthesized fibroin-like protein gene ADF3RGDS#3 using DNA Ligation Kit (TaKaRa). The nucleotide sequence of the ADF3RGDS#3 gene is shown in SEQ ID NO: 5, and the amino acid sequence of the fibroin-like protein ADF3RGDS#3 encoded by this gene is shown in SEQ ID NO: 6. The nucleotide sequence of positions 8 to 2116 of SEQ ID NO: 5 encodes the amino acid sequence shown in SEQ ID NO: 6. ADF3RGDS#3 consists of the ADF3 protein shown in SEQ ID NO: 2 except that 7 of 12 Ala-non-rich regions therein were each inserted with one unit (i.e. 7 units in total) of a cell adhesion sequence "VTGRGDSPAS" (SEQ ID NO: 23).

*Escherichia coli* BLR(DE3) (F⁻ ompT hsdS_{B} (r_{B}⁻ m_{B}⁻) gal dcm (DE3) Δ(srl-recA)306::Tn10 (Tet^{R})) was transformed with pET22b-ADF3, pET22b-ADF3RGDS#2, and pET22b-ADF3RGDS#3, to obtain fibroin-like protein-producing strains BLR(DE3)/pET22b-ADF3, BLR(DE3)/pET22b-ADF3RGDS#2, and BLR(DE3)/pET22b-ADF3RGDS#3, respectively.

### <2> Expression of cell adhesion sequence-inserted fibroin-like protein

The fibroin-like protein-expression strains BLR(DE3)/pET22b-ADF3, BLR(DE3)/pET22b-ADF3RGDS#2, and BLR(DE3)/pET22b-ADF3RGDS#3 obtained above were each cultured in a seed medium shown in (1) under conditions of 1vvm, 37°C, 1500 rpm, and pH6.7 until glucose was completely consumed, to obtain a seed culture broth. Then, 45 mL of the seed culture broth was added to 255 mL of a production medium shown in (2), and main culture was carried out under conditions of 1vvm, 37°C, 700rpm, and pH6.9. At 30 minutes after the start of the culture, a feed medium shown in (3) was start to be added at a feeding rate of 2.6 mL/h, and the culture was continued until the OD value of a culture broth at 620 nm reached 50. Then, the culture temperature was decreased to 30°C, and isopropyl-β-thiogalactopyranoside was added at a concentration of 1 mM to induce protein expression. The culture was finished at 24 hours after addition of IPTG, to obtain a culture broth. A disrupted cell suspension was prepared and applied to SDS-PAGE. As a result, a band having an objective size was observed, and that is, it was confirmed that the fibroin-like protein was expressed in cells.

### (1) Seed medium

Glucose 40 g/L, Magnesium sulfate heptahydrate 1.0 g/L, Corn steep liquor 1.0 g/L (in terms of nitrogen weight), Potassium dihydrogenphosphate 2.0 g/L, Iron sulfate heptahydrate 10 mg/L, Manganese sulfate heptahydrate 10 mg/L, Isoleucine 1.0 g/L, GD-113 (anti-foam agent) 0.1 ml/L, and Ampicillin 100 mg/L

### (2) Production medium

Glucose 2.5 g/L, Magnesium sulfate heptahydrate 2.4 g/L, Corn steep liquor 1.0 g/L (in terms of nitrogen weight), Potassium dihydrogenphosphate 9.0 g/L, Iron sulfate heptahydrate 40 mg/L, Manganese sulfate heptahydrate 40 mg/L, Calcium chloride dihydrate 40 mg/L, Isoleucine 3.0 g/L, GD-113 (anti-foam agent) 0.1 ml/L, and Ampicillin 100 mg/L

### (3) Feed medium

Glucose 700 g/L and Ampicillin 100 mg/L

### <3> Purification of cell adhesion sequence-inserted fibroin-like protein

The obtained culture broth was diluted with a Lysis buffer shown in (1) so as to adjust the OD value to 30, and subjected to ultrasonic treatment. The treated product was centrifuged, and only the precipitate was collected. The precipitate was added with a 3% SDS solution in a volume equivalent to that of the treated product before centrifugation, and suspended for 30 min with a homogenizer. The suspension was centrifuged, and only the precipitate was collected again. The precipitate was added with a solubilization buffer shown in (2) in a volume equivalent to that of the treated product before centrifugation, and suspended for 3 hr with a homogenizer. The suspension was centrifuged, and only the supernatant was collected.

The obtained supernatant was mixed with cOmplete His-Tag Purification Resin (Roche) and stirred for 1 hr so as to allow proteins to bind thereto. The flow-through fraction was collected, contaminated proteins were removed with a washing buffer shown in (3) in a volume 20 times the column volume, and the objective protein was eluted with an elution buffer shown in (4) in a volume 10 times the column volume. The elution fraction was confirmed by SDS-PAGE, and concentrated with a flat sheet membrane of 10K.

The concentrate was diluted with the solubilization buffer shown in (2) so as to adjust the concentration to 0.04-0.05 mg/ml, to obtain 100 ml of the diluted solution. Dialysis was carried out with a dialysis membrane of 15K using 5 L of ultrapure water as the external liquid, to obtain aqueous solutions of the fibroin-like proteins ADF3, ADF3RGDS#2, and ADF3RGDS#3.

### (1) Lysis buffer

30 mM Tris-HCl and 100 mM NaCl, pH7.5

### (2) Solubilization buffer

8 M Urea, 20 mM NaH₂PO₄, and 0.5 M NaCl, pH8.0

### (3) Washing buffer

8 M Urea, 20 mM NaH₂PO₄, 0.5M NaCl, and 40 mM imidazole, pH8.0

### (4) Elution buffer

8 M Urea, 20 mM NaH₂PO₄, 0.5M NaCl, and 500 mM imidazole, pH8.0

### Example 2: Preparation of cell adhesion sequence-inserted fibroin-like protein (2)

### <1> Construction of expression strain for cell adhesion sequence-inserted fibroin-like protein

A plasmid pET22b-ADF3RGDS#53 for expressing a gene encoding a cell adhesion sequence-inserted fibroin-like protein ADF3RGDS#53 was constructed by digesting pET22b(+) vector (Novagen) with restriction enzymes Ndel and EcoRI, and inserting thereto a totally-synthesized fibroin-like protein gene ADF3RGDS#53 using DNA Ligation Kit (TaKaRa). The nucleotide sequence of the ADF3RGDS#53 gene is shown in SEQ ID NO: 26, and the amino acid sequence of the fibroin-like protein ADF3RGDS#53 encoded by this gene is shown in SEQ ID NO: 27. ADF3RGDS#53 consists of the ADF3 protein shown in SEQ ID NO: 2 except that the HRV3C protease recognition sequence therein was removed and 7 of 12 Ala-non-rich regions therein were each inserted with one unit (i.e. 7 units in total) of a cell adhesion sequence "PQVTRGDVFTM" (SEQ ID NO: 24).

A plasmid pET22b-ADF3RGDS#54 for expressing a gene encoding a cell adhesion sequence-inserted fibroin-like protein ADF3RGDS#54 was constructed by digesting pET22b(+) vector (Novagen) with restriction enzymes Ndel and EcoRI, and inserting thereto a totally-synthesized fibroin-like protein gene ADF3RGDS#54 using DNA Ligation Kit (TaKaRa). The nucleotide sequence of the ADF3RGDS#54 gene is shown in SEQ ID NO: 28, and the amino acid sequence of the fibroin-like protein ADF3RGDS#54 encoded by this gene is shown in SEQ ID NO: 29. ADF3RGDS#54 consists of the ADF3 protein shown in SEQ ID NO: 2 except that the HRV3C protease recognition sequence therein was removed and 7 of 12 Ala-non-rich regions therein were each inserted with one unit (i.e. 7 units in total) of a cell adhesion sequence "VTRGDVF" (SEQ ID NO: 25).

*Escherichia coli* BL21(DE3)ΔrecA was transformed with pET22b-ADF3RGDS#53 and pET22b-ADF3RGDS#54, to obtain fibroin-like protein-producing strains BL21(DE3)ΔrecA/pET22b-ADF3RGDS#53 and BL21(DE3)ΔrecA/pET22b-ADF3RGDS#54, respectively. The strain BL21(DE3)ΔrecA can be obtained from the strain BL21(DE3) by deletion of the recA gene using λRed method.

### <2> Expression of cell adhesion sequence-inserted fibroin-like protein

The fibroin-like protein-expression strains BL21ΔrecA/pET22b-ADF3RGDS#53 and BL21ΔrecA/pET22b-ADF3RGDS#54 obtained above were each cultured in a seed medium shown in (1) under conditions of 1vvm, 37°C, 1500 rpm, and pH6.7 until glucose was completely consumed, to obtain a seed culture broth. Then, 45 mL of the seed culture broth was added to 255 mL of a production medium shown in (2), and main culture was carried out under conditions of 1vvm, 37°C, 700rpm, and pH6.9. At 30 minutes after the start of the culture, a feed medium shown in (3) was start to be added at a feeding rate of 2.6 mL/h, and the culture was continued until the OD value of a culture broth at 620 nm reached 50. Then, the culture temperature was decreased to 30°C, and isopropyl-β-thiogalactopyranoside (IPTG) was added at a concentration of 1 mM to induce protein expression. The culture was finished at 24 hours after addition of IPTG, to obtain a culture broth. A disrupted cell suspension was prepared and applied to SDS-PAGE. As a result, a band having an objective size was observed, and that is, it was confirmed that the fibroin-like protein was expressed in cells.

### (1) Seed medium

Glucose 40 g/L, Magnesium sulfate heptahydrate 1.0 g/L, Corn steep liquor 1.0 g/L (in terms of nitrogen weight), Potassium dihydrogenphosphate 2.0 g/L, Iron sulfate heptahydrate 10 mg/L, Manganese sulfate heptahydrate 10 mg/L, GD-113 (anti-foam agent) 0.1 ml/L, and Ampicillin 100 mg/L

### (2) Production medium

Glucose 2.5 g/L, Magnesium sulfate heptahydrate 2.4 g/L, Corn steep liquor 1.0 g/L (in terms of nitrogen weight), Potassium dihydrogenphosphate 5.4 g/L, Iron sulfate heptahydrate 40 mg/L, Manganese sulfate heptahydrate 40 mg/L, Calcium chloride dihydrate 40 mg/L, GD-113 (anti-foam agent) 0.1 ml/L, and Ampicillin 100 mg/L

### (3) Feed medium

Glucose 700 g/L and Ampicillin 100 mg/L

### <3> Purification of cell adhesion sequence-inserted fibroin-like protein

The obtained culture broth was diluted with a Lysis buffer shown in (1) so as to adjust the OD value to 30, and subjected to ultrasonic treatment. The treated product was centrifuged, and only the precipitate was collected. The precipitate was added with a 3% SDS solution in a volume equivalent to that of the treated product before centrifugation, and suspended for 30 min with a homogenizer. The suspension was centrifuged, and only the precipitate was collected again. The precipitate was added with a solubilization buffer shown in (2) so as to adjust the concentration of the objective protein to 5 mg/ml, and suspended with a Vortex mixer. The suspension was centrifuged, and only the supernatant was collected.

Dialysis was carried out for the obtained supernatant with a dialysis membrane of 500-1000 Da using 100 mL of an NaOH aqueous solution (pH10) as the external liquid, to obtain aqueous solutions of the fibroin-like proteins ADF3RGDS#53 and ADF3RGDS#54.

### (1) Lysis buffer

30 mM Tris-HCl and 100 mM NaCl, pH7.5

### (2) Solubilization buffer

50 mM NaOH

### Example 3: Preparation of cell adhesion sequence-inserted fibroin-like protein (3)

### <1> Construction of expression strain for cell adhesion sequence-inserted fibroin-like protein

A plasmid pET22b-ADF3RGDS#60 for expressing a gene encoding a cell adhesion sequence-inserted fibroin-like protein ADF3RGDS#60 was constructed by digesting pET22b(+) vector (Novagen) with restriction enzymes Ndel and EcoRI, and inserting thereto a totally-synthesized fibroin-like protein gene ADF3RGDS#60 using DNA Ligation Kit (TaKaRa). The nucleotide sequence of the ADF3RGDS#60 gene is shown in SEQ ID NO: 35, and the amino acid sequence of the fibroin-like protein ADF3RGDS#60 encoded by this gene is shown in SEQ ID NO: 36. ADF3RGDS#60 consists of the ADF3 protein shown in SEQ ID NO: 2 except that 7 of 12 Ala-non-rich regions therein were each inserted with one unit (i.e. 7 units in total) of a cell adhesion sequence "GRGDNP" (SEQ ID NO: 30).

A plasmid pET22b-ADF3RGDS#65 for expressing a gene encoding a cell adhesion sequence-inserted fibroin-like protein ADF3RGDS#65 was constructed by digesting pET22b(+) vector (Novagen) with restriction enzymes Ndel and EcoRI, and inserting thereto a totally-synthesized fibroin-like protein gene ADF3RGDS#65 using DNA Ligation Kit (TaKaRa). The nucleotide sequence of the ADF3RGDS#65 gene is shown in SEQ ID NO: 37, and the amino acid sequence of the fibroin-like protein ADF3RGDS#65 encoded by this gene is shown in SEQ ID NO: 38. ADF3RGDS#65 consists of the ADF3 protein shown in SEQ ID NO: 2 except that the HRV3C protease recognition sequence therein was removed and partial sequences of 7 of 12 Ala-non-rich regions therein were each replaced with a cell adhesion sequence "GAAGRGDSPAAGY" (SEQ ID NO: 31). In other words, it can be considered that the cell adhesion sequence has been inserted into the Ala-non-rich region in ADF3RGDS#65, which Ala-non-rich region is shorter than that of the ADF3 protein shown in SEQ ID NO: 2.

A plasmid pET22b-ADF3RGDS#68 for expressing a gene encoding a cell adhesion sequence-inserted fibroin-like protein ADF3RGDS#68 was constructed by digesting pET22b(+) vector (Novagen) with restriction enzymes Ndel and EcoRI, and inserting thereto a totally-synthesized fibroin-like protein gene ADF3RGDS#68 using DNA Ligation Kit (TaKaRa). The nucleotide sequence of the ADF3RGDS#68 gene is shown in SEQ ID NO: 39, and the amino acid sequence of the fibroin-like protein ADF3RGDS#68 encoded by this gene is shown in SEQ ID NO: 40. ADF3RGDS#68 consists of the ADF3 protein shown in SEQ ID NO: 2 except that the HRV3C protease recognition sequence therein was removed, 5 of 12 Ala-non-rich regions therein were each inserted with one unit (i.e. 5 units in total) of an amino acid sequence "GPGQQGPGQQGPGQQVTGRGDSPAS" (SEQ ID NO: 32) containing a cell adhesion sequence "VTGRGDSPAS" (SEQ ID NO: 23), and 2 of 12 Ala-non-rich regions therein were each inserted with one unit (i.e. 2 units in total) of a cell adhesion sequence "VTGRGDSPAS" (SEQ ID NO: 23). In other words, regarding the former 5 units in total of the cell adhesion sequence, it can be considered that the cell adhesion sequence has been inserted into the Ala-non-rich region in ADF3RGDS#68, which Ala-non-rich region is longer than that of the ADF3 protein shown in SEQ ID NO: 2.

A plasmid pET22b-ADF3RGDS#72 for expressing a gene encoding a cell adhesion sequence-inserted fibroin-like protein ADF3RGDS#72 was constructed by digesting pET22b(+) vector (Novagen) with restriction enzymes Ndel and EcoRI, and inserting thereto a totally-synthesized fibroin-like protein gene ADF3RGDS#72 using DNA Ligation Kit (TaKaRa). The nucleotide sequence of the ADF3RGDS#72 gene is shown in SEQ ID NO: 41, and the amino acid sequence of the fibroin-like protein ADF3RGDS#72 encoded by this gene is shown in SEQ ID NO: 42. ADF3RGDS#72 consists of the ADF3 protein shown in SEQ ID NO: 2 except that 12 Ala-non-rich regions therein were each inserted with one unit (i.e. 12 units in total) of a cell adhesion sequence "GRGDSP" (SEQ ID NO: 22).

A plasmid pET22b-ADF3RGDS#73 for expressing a gene encoding a cell adhesion sequence-inserted fibroin-like protein ADF3RGDS#73 was constructed by digesting pET22b(+) vector (Novagen) with restriction enzymes Ndel and EcoRI, and inserting thereto a totally-synthesized fibroin-like protein gene ADF3RGDS#73 using DNA Ligation Kit (TaKaRa). The nucleotide sequence of the ADF3RGDS#73 gene is shown in SEQ ID NO: 43, and the amino acid sequence of the fibroin-like protein ADF3RGDS#73 encoded by this gene is shown in SEQ ID NO: 44. ADF3RGDS#73 consists of the ADF3 protein shown in SEQ ID NO: 2 except that the HRV3C protease recognition sequence therein was removed and 2 of 12 Ala-non-rich regions therein were each inserted with one unit (i.e. 2 units in total) of a cell adhesion sequence "GRGDSP" (SEQ ID NO: 22).

A plasmid pET22b-ADF3RGDS#76 for expressing a gene encoding a cell adhesion sequence-inserted fibroin-like protein ADF3RGDS#76 was constructed by digesting pET22b(+) vector (Novagen) with restriction enzymes Ndel and EcoRI, and inserting thereto a totally-synthesized fibroin-like protein gene ADF3RGDS#76 using DNA Ligation Kit (TaKaRa). The nucleotide sequence of the ADF3RGDS#76 gene is shown in SEQ ID NO: 45, and the amino acid sequence of the fibroin-like protein ADF3RGDS#76 encoded by this gene is shown in SEQ ID NO: 46. ADF3RGDS#76 consists of the ADF3 protein shown in SEQ ID NO: 2 except that the HRV3C protease recognition sequence therein was removed and 7 of 12 Ala-non-rich regions therein were each inserted with one unit (i.e. 7 units in total) of an amino acid sequence "VTGRGDSPASVTGRGDSPAS" (SEQ ID NO: 33) consisting of two mutually-linked units of a cell adhesion sequence "VTGRGDSPAS" (SEQ ID NO: 23). In other words, it can be considered that 14 units in total of the cell adhesion sequence have been inserted in ADF3RGDS#76.

A plasmid pET22b-ADF3RGDS#77 for expressing a gene encoding a cell adhesion sequence-inserted fibroin-like protein ADF3RGDS#77 was constructed by digesting pET22b(+) vector (Novagen) with restriction enzymes Ndel and EcoRI, and inserting thereto a totally-synthesized fibroin-like protein gene ADF3RGDS#77 using DNA Ligation Kit (TaKaRa). The nucleotide sequence of the ADF3RGDS#77 gene is shown in SEQ ID NO: 47, and the amino acid sequence of the fibroin-like protein ADF3RGDS#77 encoded by this gene is shown in SEQ ID NO: 48. ADF3RGDS#77 consists of the ADF3 protein shown in SEQ ID NO: 2 except that 12 Ala-non-rich regions therein were each inserted with one unit (i.e. 12 units in total) of a cell adhesion sequence "VTGRGDSPAS" (SEQ ID NO: 23).

A plasmid pET22b-ADF3RGDS#141 for expressing a gene encoding a heparin-binding sequence-inserted fibroin-like protein ADF3RGDS#141 was constructed by digesting pET22b(+) vector (Novagen) with restriction enzymes Ndel and EcoRI, and inserting thereto a totally-synthesized fibroin-like protein gene ADF3RGDS#141 using DNA Ligation Kit (TaKaRa). The nucleotide sequence of the ADF3RGDS#141 gene is shown in SEQ ID NO: 49, and the amino acid sequence of the fibroin-like protein ADF3RGDS#141 encoded by this gene is shown in SEQ ID NO: 50. ADF3RGDS#141 consists of the ADF3 protein shown in SEQ ID NO: 2 except that 7 of 12 Ala-non-rich regions therein were each inserted with one unit (i.e. 7 units in total) of a heparin-binding sequence "GKKQRFRHRNRKG" (SEQ ID NO: 34). This heparin-binding sequence is an amino acid sequence consisting of the heparin-binding sequence of vitronectin (Wrighton PJ, Klim JR, Hernandez BA, Koonce CH, Kamp TJ, Kiessling LL, Proceedings of the National Academy of Sciences of the United States of America 2014 Dec ; 111(51) : 18126-18131., Signals from the surface modulate differentiation of human pluripotent stem cells through glycosaminoglycans and integrins.) added with G at the both termini.

A plasmid pET22b-ADF3RGDS#66 for expressing a gene encoding a cell adhesion sequence-inserted fibroin-like protein ADF3RGDS#66 was constructed by digesting pET22b(+) vector (Novagen) with restriction enzymes Ndel and EcoRI, and inserting thereto a totally-synthesized fibroin-like protein gene ADF3RGDS#66 using DNA Ligation Kit (TaKaRa). The nucleotide sequence of the ADF3RGDS#66 gene is shown in SEQ ID NO: 51, and the amino acid sequence of the fibroin-like protein ADF3RGDS#66 encoded by this gene is shown in SEQ ID NO: 52. ADF3RGDS#66 consists of the ADF3 protein shown in SEQ ID NO: 2 except that the HRV3C protease recognition sequence therein was removed, partial sequences of 5 of 12 Ala-non-rich regions therein were each replaced with a cell adhesion sequence "VTGRGDSPAS" (SEQ ID NO: 23), and 2 of 10 Ala-non-rich regions therein were each inserted with one unit (i.e. 2 units in total) of a cell adhesion sequence "VTGRGDSPAS" (SEQ ID NO: 23). In other words, regarding the former 5 units in total of the cell adhesion sequence, it can be considered that the cell adhesion sequence has been inserted into the Ala-non-rich region in ADF3RGDS#66, which Ala-non-rich region is shorter than that of the ADF3 protein shown in SEQ ID NO: 2.

A plasmid pET22b-ADF3RGDS#78 for expressing a gene encoding a cell adhesion sequence-inserted fibroin-like protein ADF3RGDS#78 was constructed by digesting pET22b(+) vector (Novagen) with restriction enzymes Ndel and EcoRI, and inserting thereto a totally-synthesized fibroin-like protein gene ADF3RGDS#78 using DNA Ligation Kit (TaKaRa). The nucleotide sequence of the ADF3RGDS#78 gene is shown in SEQ ID NO: 53, and the amino acid sequence of the fibroin-like protein ADF3RGDS#78 encoded by this gene is shown in SEQ ID NO: 54. ADF3RGDS#78 consists of the ADF3 protein shown in SEQ ID NO: 2 except that the HRV3C protease recognition sequence therein was removed and 2 of 10 Ala-non-rich regions therein were each inserted with one unit (i.e. 2 units in total) of a cell adhesion sequence "VTGRGDSPAS" (SEQ ID NO: 23).

*Escherichia coli* BL21(DE3)ΔrecA was transformed with pET22b-ADF3RGDS#60, pET22b-ADF3RGDS#65, pET22b-ADF3RGDS#68, pET22b-ADF3RGDS#72, pET22b-ADF3RGDS#73, pET22b-ADF3RGDS#76, pET22b-ADF3RGDS#77, pET22b-ADF3RGDS#141, pET22b-ADF3RGDS#66, and pET22b-ADF3RGDS#78, to obtain fibroin-like protein-producing strains BL21(DE3)ΔrecA/pET22b-ADF3RGDS#60, BL21(DE3)ΔrecA/pET22b-ADF3RGDS#65, BL21(DE3)ΔrecA/pET22b-ADF3RGDS#68, BL21(DE3)ΔrecA/pET22b-ADF3RGDS#72, BL21(DE3)ΔrecA/pET22b-ADF3RGDS#73, BL21(DE3)ΔrecA/pET22b-ADF3RGDS#76, BL21(DE3)ΔrecA/pET22b-ADF3RGDS#77, BL21(DE3)ΔrecA/pET22b-ADF3RGDS#141, BL21(DE3)ΔrecA/pET22b-ADF3RGDS#66, and BL21(DE3)ΔrecA/pET22b-ADF3RGDS#78, respectively.

### <2> Expression of cell adhesion sequence-inserted fibroin-like protein

The fibroin-like protein-expression strains BL21(DE3)ΔrecA/pET22b-ADF3RGDS#60, BL21(DE3)ΔrecA/pET22b-ADF3RGDS#65, BL21(DE3)ΔrecA/pET22b-ADF3RGDS#72, BL21(DE3)ΔrecA/pET22b-ADF3RGDS#73, and BL21(DE3)ΔrecA/pET22b-ADF3RGDS#141 obtained above were each cultured in a seed medium shown in (1) under conditions of 1vvm, 37°C, 1500 rpm, and pH6.7 until glucose was completely consumed, to obtain a seed culture broth. Then, 45 mL of the seed culture broth was added to 255 mL of a production medium shown in (2), and main culture was carried out under conditions of 1vvm, 37°C, 700rpm, and pH6.9. At 30 minutes after the start of the culture, a feed medium shown in (3) was start to be added at a feeding rate of 2.6 mL/h, and the culture was continued until the OD value of a culture broth at 620 nm reached 50. Then, the culture temperature was decreased to 30°C, and isopropyl-β-thiogalactopyranoside (IPTG) was added at a concentration of 1 mM to induce protein expression. The culture was finished at 24 hours after addition of IPTG, to obtain a culture broth. A disrupted cell suspension was prepared and applied to SDS-PAGE. As a result, a band having an objective size was observed, and that is, it was confirmed that the fibroin-like protein was expressed in cells.

### (1) Seed medium

Glucose 40 g/L, Magnesium sulfate heptahydrate 1.0 g/L, Corn steep liquor 1.0 g/L (in terms of nitrogen weight), Potassium dihydrogenphosphate 2.0 g/L, Iron sulfate heptahydrate 10 mg/L, Manganese sulfate heptahydrate 10 mg/L, GD-113 (anti-foam agent) 0.1 ml/L, and Ampicillin 100 mg/L

### (2) Production medium

Glucose 2.5 g/L, Magnesium sulfate heptahydrate 2.4 g/L, Corn steep liquor 1.0 g/L (in terms of nitrogen weight), Potassium dihydrogenphosphate 5.4 g/L, Iron sulfate heptahydrate 40 mg/L, Manganese sulfate heptahydrate 40 mg/L, Calcium chloride dihydrate 40 mg/L, GD-113 (anti-foam agent) 0.1 ml/L, and Ampicillin 100 mg/L

### (3) Feed medium

Glucose 700 g/L and Ampicillin 100 mg/L

The fibroin-like protein-expression strains BL21(DE3)ΔrecA/pET22b-ADF3RGDS#68, BL21(DE3)ΔrecA/pET22b-ADF3RGDS#76, BL21(DE3)ΔrecA/pET22b-ADF3RGDS#77, BL21(DE3)ΔrecA/pET22b-ADF3RGDS#66, and BL21(DE3)ΔrecA/pET22b-ADF3RGDS#78 obtained above are each cultured in a seed medium shown in (1) under conditions of 1vvm, 37°C, 1500 rpm, and pH6.7 until glucose is completely consumed, to obtain a seed culture broth. Then, 45 mL of the seed culture broth is added to 255 mL of a production medium shown in (2), and main culture is carried out under conditions of 1vvm, 37°C, 700rpm, and pH6.9. At 30 minutes after the start of the culture, a feed medium shown in (3) is start to be added at a feeding rate of 2.6 mL/h, and the culture is continued until the OD value of a culture broth at 620 nm reached 50. Then, the culture temperature is decreased to 30°C, and isopropyl-β-thiogalactopyranoside (IPTG) is added at a concentration of 1 mM to induce protein expression. The culture is finished at 24 hours after addition of IPTG, to obtain a culture broth. Then, a disrupted cell suspension is prepared and applied to SDS-PAGE, to confirm the expression of the objective fibroin-like protein.

### (1) Seed medium

Glucose 40 g/L, Magnesium sulfate heptahydrate 1.0 g/L, Corn steep liquor 1.0 g/L (in terms of nitrogen weight), Potassium dihydrogenphosphate 2.0 g/L, Iron sulfate heptahydrate 10 mg/L, Manganese sulfate heptahydrate 10 mg/L, GD-113 (anti-foam agent) 0.1 ml/L, and Ampicillin 100 mg/L

### (2) Production medium

Glucose 2.5 g/L, Magnesium sulfate heptahydrate 2.4 g/L, Corn steep liquor 1.0 g/L (in terms of nitrogen weight), Potassium dihydrogenphosphate 5.4 g/L, Iron sulfate heptahydrate 40 mg/L, Manganese sulfate heptahydrate 40 mg/L, Calcium chloride dihydrate 40 mg/L, GD-113 (anti-foam agent) 0.1 ml/L, and Ampicillin 100 mg/L

### (3) Feed medium

Glucose 700 g/L and Ampicillin 100 mg/L

### <3> Purification of cell adhesion sequence-inserted fibroin-like protein

The obtained culture broth of each of the fibroin-like protein-expression strains BL21(DE3)ΔrecA/pET22b-ADF3RGDS#60, BL21(DE3)ΔrecA/pET22b-ADF3RGDS#65, BL21(DE3)ΔrecA/pET22b-ADF3RGDS#72, and BL21(DE3)ΔrecA/pET22b-ADF3RGDS#141 was diluted with a Lysis buffer shown in (1) so as to adjust the OD value to 30, and subjected to ultrasonic treatment. The treated product was centrifuged, and only the precipitate was collected. The precipitate was added with a 3% SDS solution in a volume equivalent to that of the treated product before centrifugation, and suspended for 5 min or longer with a homogenizer. The suspension was centrifuged, and only the precipitate was collected again. The precipitate was added with a solubilization buffer shown in (2) so as to adjust the concentration of the objective protein to 1-5 mg/ml, and suspended with a Vortex mixer. The suspension was centrifuged, and only the supernatant was collected.

Dialysis was carried out for 1 ml of the obtained supernatant with a dialysis membrane of 500-1000 Da using 100 mL of an NaOH aqueous solution (pH10) as the external liquid, to obtain aqueous solutions of the fibroin-like proteins ADF3RGDS#60, ADF3RGDS#65, ADF3RGDS#72, and ADF3RGDS#141.

### (1) Lysis buffer

30 mM Tris-HCl and 100 mM NaCl, pH7.5

### (2) Solubilization buffer

50 mM NaOH

The obtained culture broth of the fibroin-like protein-expression strain BL21(DE3)ΔrecA/pET22b-ADF3RGDS#73 was diluted with a Lysis buffer shown in (1) so as to adjust the OD value to 30, and subjected to ultrasonic treatment. The treated product was centrifuged, and only the precipitate was collected. The precipitate was added with a 3% SDS solution in a volume equivalent to that of the treated product before centrifugation, and suspended for 5 min or longer with a homogenizer. The suspension was centrifuged, and only the precipitate was collected again. The precipitate was added with a solubilization buffer shown in (2) in a volume equivalent to that of the treated product before centrifugation, and suspended for 1 hr with a homogenizer. The suspension was centrifuged, and only the supernatant was collected.

The obtained supernatant was mixed with cOmplete His-Tag Purification Resin (Roche) and stirred for 1 hr 30 min so as to allow proteins to bind thereto. The flow-through fraction was collected, contaminated proteins were removed with a washing buffer shown in (3) in a volume 20 times the column volume, and the objective fibroin-like protein was eluted with an elution buffer shown in (4) in a volume 10 times the column volume. The elution fraction was confirmed by SDS-PAGE, and concentrated with a membrane of 30 KDa.

The concentrate was subjected to buffer exchange with a solution shown in (5) using a membrane of 30 KDa, to be adjusted to a volume providing a concentration of 0.1 mg/ml. Dialysis was carried out for 1 ml of the solution after the buffer exchange with a dialysis membrane of 500-1000 Da using 100 mL of an NaOH aqueous solution (pH10) as the external liquid, to obtain an aqueous solution of the fibroin-like protein ADF3RGDS#73.

### (1) Lysis buffer

30 mM Tris-HCl and 100 mM NaCl, pH7.5

### (2) Solubilization buffer

8 M Urea, 20 mM NaH₂PO₄, and 0.5 M NaCl, pH8.0

### (3) Washing buffer

8 M Urea, 20 mM NaH₂PO₄, 0.5M NaCl, and 40 mM imidazole, pH8.0

### (4) Elution buffer

8 M Urea, 20 mM NaH₂PO₄, 0.5M NaCl, and 500 mM imidazole, pH8.0

### (5) Buffer exchange solution

50 mM NaOH

The obtained culture broth of each of the fibroin-like protein-expression strains BL21(DE3)ΔrecA/pET22b-ADF3RGDS#68, BL21(DE3)ΔrecA/pET22b-ADF3RGDS#76, BL21(DE3)ΔrecA/pET22b-ADF3RGDS#77, BL21(DE3)ΔrecA/pET22b-ADF3RGDS#66, and BL21(DE3)ΔrecA/pET22b-ADF3RGDS#78 is diluted with a Lysis buffer shown in (1) so as to adjust the OD value to 30, and subjected to ultrasonic treatment. The treated product is centrifuged, and only the precipitate is collected. The precipitate is added with a 3% SDS solution in a volume equivalent to that of the treated product before centrifugation, and suspended for 5 min or longer with a homogenizer. The suspension is centrifuged, and only the precipitate is collected again. The precipitate is added with a solubilization buffer shown in (2) so as to adjust the concentration of the objective protein to 1-5 mg/ml, and suspended with a Vortex mixer. The suspension is centrifuged, and only the supernatant is collected.

Dialysis is carried out for 1 ml of the obtained supernatant with a dialysis membrane of 500-1000 Da using 100 mL of an NaOH aqueous solution (pH10) as the external liquid, to obtain aqueous solutions of the fibroin-like proteins ADF3RGDS#68, ADF3RGDS#76, ADF3RGDS#77, ADF3RGDS#66, and ADF3RGDS#78.

### (1) Lysis buffer

30 mM Tris-HCl and 100 mM NaCl, pH7.5

### (2) Solubilization buffer

50 mM NaOH

### Example 4: Evaluation of cell scaffold function of fibroin-like protein

### <1> Coating to cell culture plate

### <1-1> Coating to cell culture plate (1)

The aqueous solutions of fibroin-like proteins after dialysis obtained in Examples 1<3> and 3<3> were applied to a 6-well cell culture plate (Falcon cell culture plate; Corning) in volumes of 1 to 4 ml/well. The cell culture plate was left to stand at a room temperature for a day and night or for two days and nights, so that water was dried. The cell culture plate was immersed into a 70% ethanol aqueous solution, and then washed with phosphate buffered saline (Nacalai Tesque).

### <1-2> Coating to cell culture plate (2)

The aqueous solutions of fibroin-like proteins after dialysis obtained in Examples 2<3> and 3<3> were applied to a 6-well cell culture plate (Falcon cell culture plate; Corning) in volumes of 1 to 4 ml/well. The cell culture plate was left to stand at 4°C for a day and night, and then the aqueous solutions were removed. The cell culture plate was immersed into a 70% ethanol aqueous solution, and then washed with phosphate buffered saline (Nacalai Tesque).

### <2> Confirmation of coating

The coating surface was analyzed by FT-IR attenuated total reflection (FT-IR-ATR) using IR Prestige-21 (Shimadzu Corporation) and an ATR measuring device DuraSamplIR II (Smiths). A peak derived from an amide group of a protein main chain (a-amide bond) was observed in the range of 1620 to 1700 cm⁻¹ in the obtained spectrum, and hence, it was confirmed that the fibroin-like protein was coated on the cell culture plate. The coating amount of the fibroin-like protein is considered to be approximately 10 µg/cm² when coating is carried out by the method of <1-1>.

### <3> Evaluation in iPS cell proliferation system

The effect on adhesion and proliferation of an induced pluripotent stem cell (iPS cell) on the surface coated with the fibroin-like protein was evaluated. As the iPS cell, the strain 201B7 purchased from iPS Portal was used. Cell culture was carried out under conditions of 5%CO₂/37°C using a culture medium obtained by adding human serum albumin (final concentration of 2.6 mg/ml; Sigma-Aldrich) to Essential 8 medium (Invitrogen), which is a culture medium for iPS cells, or using StemFit(R) medium (Ajinomoto). The cell culture plate was used for culture immediately after coating with the fibroin-like protein, or was stored for 6 days at 4°C after coating with the fibroin-like protein and then used for culture, and thereby the effect of the coating was investigated. Viable cells were inoculated as single cells in amounts of 20,000 to 100,000 cells/well. The culture medium used at the time of the inoculation was added with Y-27632 (final concentration of 10 µM, Nacalai Tesque), and from the next day, culture was carried out with the culture medium not added with Y-27632. The culture medium was exchanged every 1 to 3 days, and culture was carried out for 6 to 7 days. Then, the number of cells released by a treatment with a cell dissociation enzyme (TrypLE Select; Life Technologies) was measured with a cell counter (ViCell; Beckman Coulter). As a comparative control, Pronectin (Sanyo Chemical Industries), which is an artificial cell adhesion peptide, was coated on the cell culture plate according to the product protocol, and evaluation was carried out with the same culture method. As further comparative controls, evaluation was also carried out for various cell culture vessels coated with a basement membrane matrix or a mimic thereof (BioCoat Matrigel of Corning, BioCoat Fibronectin of Corning, and PureCoat Fibronectin peptide of Corning) with the same culture method. Among them, when using BioCoat Matrigel (Corning), the first exchange of the culture medium (i.e. exchange from the culture medium containing Y-27632 used at the time of the inoculation to the culture medium not containing Y-27632) was carried out at 48 hours after the inoculation.

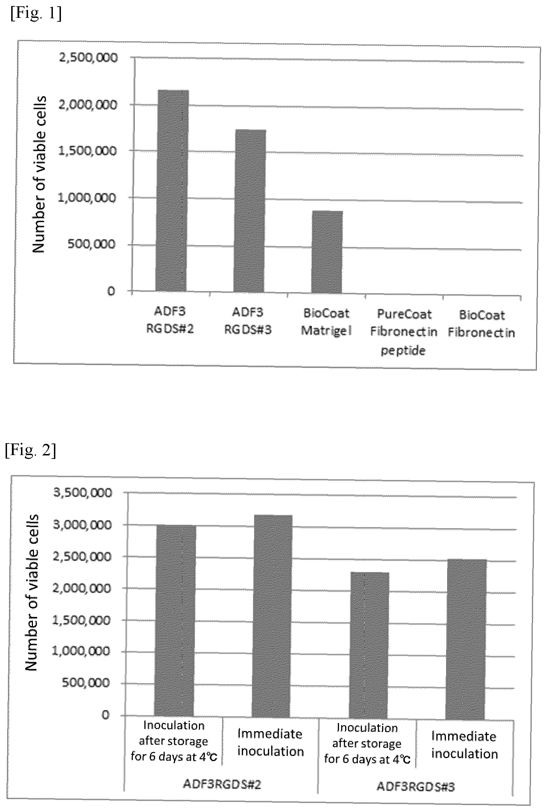

Results are shown in Figs. 1-7 and Table 1.

As shown in Fig. 1, when any of ADF3RGDS#2 and ADF3RGDS#3, which are fibroin-like proteins inserted with a cell adhesion sequence, was used for coating with the method of <1-1> and 100,000 viable cells were inoculated, the number of viable cells after 6 days was larger than that observed with BioCoat Matrigel, which is commercially available as a culture vessel for pluripotent stem cells, and that is, it was revealed that ADF3RGDS#2 and ADF3RGDS#3 have a favorable scaffold function for an iPS cell. Although ADF3RGDS#2 and ADF3RGDS#3 each have been inserted with a cell adhesion sequence of fibronectin, the number of viable cells at 6 days after the inoculation was zero when using BioCoat Fibronectin, which is coated with fibronectin *per se*, or PureCoat Fibronectin peptide, which is coated with a mimic peptide of fibronectin.

When evaluation was carried out for ADF3, which is a fibroin-like protein not inserted with a cell adhesion sequence, with the same method, adhesion of inoculated cells was scarcely observed, and the number of viable cells at 6 days after the inoculation was zero (data not shown). This indicates that a scaffold function for an iPS cell was obtained due to insertion of a cell adhesion sequence.

When evaluation was carried out for Pronectin (Sanyo Chemical Industries), which is a fibroin of silkworm inserted with the same cell adhesion sequence as that inserted in ADF3RGDS#3, with the same method, the number of viable cells at 6 days after the inoculation was zero as with the case of ADF3 (data not shown). This indicates that it is difficult to foresee the present invention.

As shown in Fig. 2, even when any of ADF3RGDS#2 and ADF3RGDS#3, which are fibroin-like proteins inserted with a cell adhesion sequence, was coated on the cell culture plate with the method of <1-1> and the cell culture plate was used for cell culture after storage for 6 days at 4°C, the number of viable cells at 6 days after the inoculation of 100,000 viable cells was equivalent to that observed when the cell culture plate was used for cell culture immediately after the coating. Regarding basement membrane matrices (proteins) used for feeder-free culture of pluripotent stem cells, such as iMatrix (Nippi), it is recommended, according to the product protocol, that a cell culture plate is used for cell culture immediately after coating therewith. By contrast, it is not necessary that a cell culture plate is used for cell culture immediately after coating with the product of the present invention, and hence, the product of the present invention enables easily carrying out culture of pluripotent stem cells.

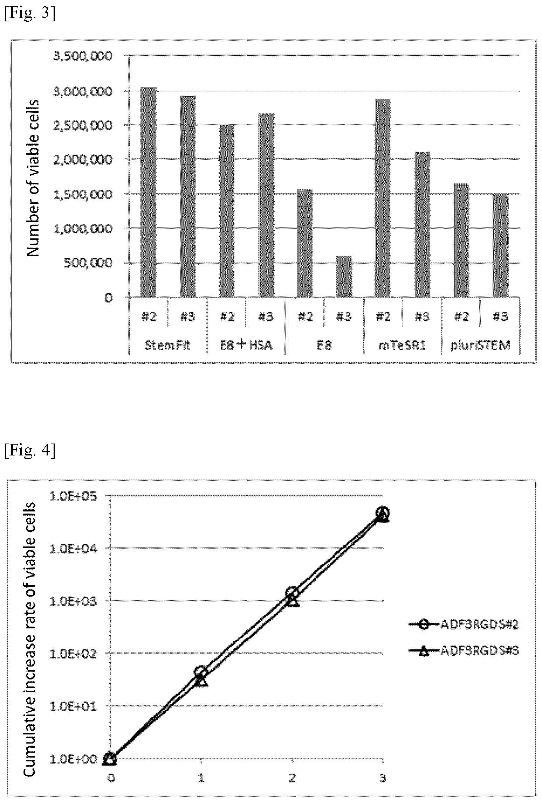

As shown in Fig. 3, when any of ADF3RGDS#2 (#2 in the figure) and ADF3RGDS#3 (#3 in the figure), which are fibroin-like proteins inserted with a cell adhesion sequence, was used for coating with the method of <1-1> and 100,000 viable cells were inoculated, they showed a favorable scaffold function for an iPS cell even when cell culture was carrying out using any of Essential 8 medium alone (E8 in the figure), mTeSR1 medium (STEMCELL Technologies), and pluriSTEM medium (Merck Millipore), as well as using StemFit(R) medium (Ajinomoto) or the culture medium (E8+HSA in the figure) obtained by adding human serum albumin to Essential 8 medium (Invitrogen). This indicates that the product of the present invention is not limited to be used in a specific culture medium, and hence, it is highly versatile.

As shown in Fig. 4, when any of ADF3RGDS#2 and ADF3RGDS#3, which are fibroin-like proteins inserted with a cell adhesion sequence, was used for coating with the method of <1-1> and 100,000 viable cells were inoculated, culture was successfully carried out over a period of 3 weeks wherein subculture was carried out twice. The increase rate of viable cells was not changed during the culture, and that is, it was indicated that culture can be stably carried out. After 3 weeks of the culture, cells were stained with an alkaline phosphatase staining kit (86-R; Sigma-Aldrich), and as a result, it was confirmed that cells were kept in undifferentiated state. This indicates that the product of the present invention enables an iPS cell to proliferate while maintaining undifferentiated state thereof in a long-term culture.

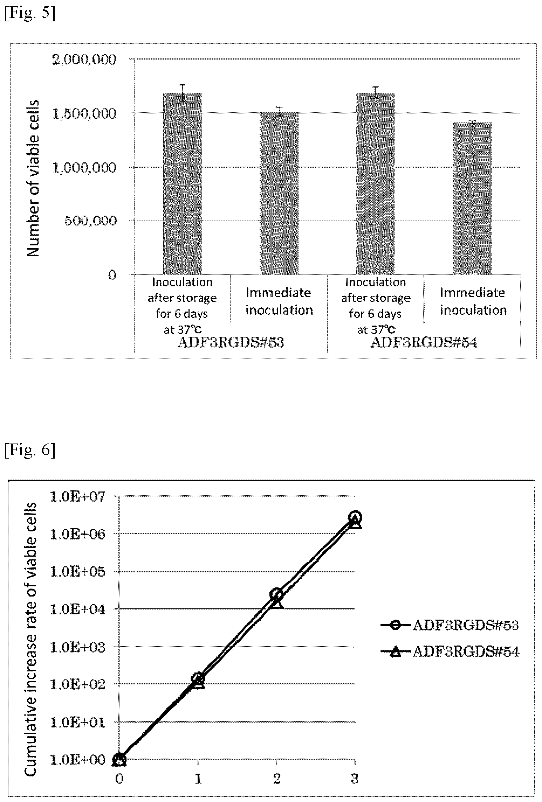

As shown in Fig. 5, even when any of ADF3RGDS#53 and ADF3RGDS#54, which are fibroin-like proteins inserted with a cell adhesion sequence, was coated on the cell culture plate with the method of <1-2> and the cell culture plate was used for cell culture after storage for 6 days at 37°C, the number of viable cells at 6 days after the inoculation of 20,000 viable cells was equivalent to that observed when the cell culture plate was used for cell culture immediately after the coating. That is, it was indicated that coating can be carried out with a method in a short time with a low contamination risk as compared with the method of <1-1>, the product of the present invention can be stored at a normal temperature, and a pluripotent stem cell can be cultured by using the product of the present invention without using a large amount of cells. This indicates that the product of the present invention is widely superior as a scaffold material for an iPS cell in terms not only of functions but also of operability and stability etc.

As shown in Fig. 6, when any of ADF3RGDS#53 and ADF3RGDS#54, which are fibroin-like proteins inserted with a cell adhesion sequence, was used for coating with the method of <1-1> and 20,000 or 25,000 viable cells were inoculated, culture was successfully carried out over a period of 3 weeks wherein subculture was carried out twice. The increase rate of viable cells was not changed during the culture, and that is, it was indicated that culture can be stably carried out without using a large amount of cells. After 3 weeks of the culture, cells were stained with an alkaline phosphatase staining kit (86-R; Sigma-Aldrich), and as a result, it was confirmed that cells were kept in undifferentiated state. This indicates that the product of the present invention enables an iPS cell to proliferate while maintaining undifferentiated state thereof in a long-term culture.

As shown in Table 1, when any of ADF3RGDS#60, ADF3RGDS#65, ADF3RGDS#66, ADF3RGDS#68, ADF3RGDS#72, ADF3RGDS#73, ADF3RGDS#76, ADF3RGDS#77, and ADF3RGDS#78, which are fibroin-like proteins inserted with a cell adhesion sequence, was used for coating with the method of <1-1> or <1-2> and 13,000, 20,000, 50,000, or 10,0000 viable cells were inoculated, the increase rate of viable cells after 6 days or 7 days was equivalent to or larger than that observed with ADF3RGDS#2 or ADF3RGDS#3. Among them, ADF3RGDS#76 showed a high increase rate of viable cells, and it showed an increase rate of viable cells of 40 to 77 folds even when cells were subcultured at 7 days after the inoculation and then further cultured for 1 week.

**Table 1**

| Fibroin-like protein inserted with cell adhesion sequence | Coating | The number of inoculated viable cells | The number of measured viable cells | Increase rate of viable cells |
|---|---|---|---|---|
| ADF3RGDS#2 | Method of <1-2> | 50,000 | 492,120 | 9.8 |
| ADF3RGDS#3 | Method of <1-2> | 50,000 | 1,110,483 | 22.2 |
| | | 20,000 | 153,150 | 7.7 |
| | | 13,000 | 68,318 | 5.3 |
| ADF3RGDS#60 | Method of <1-2> | 50,000 | 791,543 | 15.8 |
| ADF3RGDS#65 | Method of <1-1> | 100,000 | 2,092,500 | 20.9 |
| ADF3RGDS#66 | Method of <1-2> | 50,000 | 578,917 | 11.6 |
| ADF3RGDS#68 | Method of <1-2> | 50,000 | 1,474,619 | 29.5 |
| ADF3RGDS#72 | Method of <1-2> | 50,000 | 2,185,599 | 43.7 |
| ADF3RGDS#73 | Method of <1-1> | 50,000 | 2,045,218 | 40.9 |
| | Method of <1-2> | 50,000 | 2,177,210 | 43.5 |
| ADF3RGDS#76 | Method of <1-2> | 50,000 | 3,655,838 | 73.1 |
| | | 20,000 | 1,982,046 | 99.1 |
| | | 13,000 | 1,045,257 | 80.4 |
| ADF3RGDS#77 | Method of <1-2> | 50,000 | 2,924,998 | 58.5 |
| | | 20,000 | 747,374 | 37.4 |
| ADF3RGDS#78 | Method of <1-2> | 50,000 | 87,365 | 1.7 |

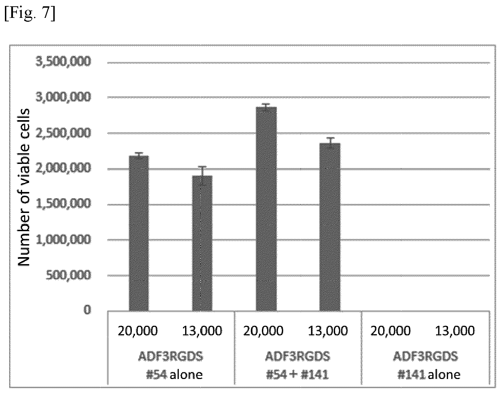

As shown in Fig. 7, when ADF3RGDS#54, which is a fibroin-like protein inserted with a cell adhesion sequence, was mixed with ADF3RGDS#141, which is a fibroin-like protein inserted with a heparin-binding sequence, and coated on the cell culture plate with the method of <1-2>, and 13,000 or 20,000 viable cells were inoculated, the number of viable cells after 6 days was increased by 31% or 24% as compared with that observed when ADF3RGDS#54 was solely used for coating and cell culture. When ADF3RGDS#141 was solely used for coating and cell culture, adhesion or proliferation of cells was not observed. This indicates that the scaffold function for an iPS cell of the product of the present invention is increased by combined use of the product of the present invention with a protein other than the product of the present invention.

### <Explanation of Sequence Listing>

SEQ ID NO: 1, Nucleotide sequence of fibroin-like protein gene ADF3
SEQ ID NO: 2, Amino acid sequence of fibroin-like protein ADF3
SEQ ID NO: 3, Nucleotide sequence of cell adhesion sequence-inserted fibroin-like protein gene ADF3RGDS#2
SEQ ID NO: 4, Amino acid sequence of cell adhesion sequence-inserted fibroin-like protein ADF3RGDS#2
SEQ ID NO: 5, Nucleotide sequence of cell adhesion sequence-inserted fibroin-like protein gene ADF3RGDS#3
SEQ ID NO: 6, Amino acid sequence of cell adhesion sequence-inserted fibroin-like protein ADF3RGDS#3
SEQ ID NOS: 7 to 10, Amino acid sequences of regions satisfying the requirement(s) of the Ala-rich region
SEQ ID NOS: 11 to 21, Amino acid sequences of motifs contained in regions satisfying the requirement(s) of the Ala-non-rich region
SEQ ID NOS: 22 to 25, Cell adhesion sequences containing RGD
SEQ ID NO: 26, Nucleotide sequence of cell adhesion sequence-inserted fibroin-like protein gene ADF3RGDS#53
SEQ ID NO: 27, Amino acid sequence of cell adhesion sequence-inserted fibroin-like protein ADF3RGDS#53
SEQ ID NO: 28, Nucleotide sequence of cell adhesion sequence-inserted fibroin-like protein gene ADF3RGDS#54
SEQ ID NO: 29, Amino acid sequence of cell adhesion sequence-inserted fibroin-like protein ADF3RGDS#54
SEQ ID NOS: 30 and 31, Cell adhesion sequences containing RGD
SEQ ID NO: 32, Amino acid sequence containing cell adhesion sequences containing RGD
SEQ ID NO: 33, Amino acid sequence consisting of two mutually-linked units of cell adhesion sequence containing RGD
SEQ ID NO: 34, Heparin-binding sequence
SEQ ID NO: 35, Nucleotide sequence of cell adhesion sequence-inserted fibroin-like protein gene ADF3RGDS#60
SEQ ID NO: 36, Amino acid sequence of cell adhesion sequence-inserted fibroin-like protein ADF3RGDS#60
SEQ ID NO: 37, Nucleotide sequence of cell adhesion sequence-inserted fibroin-like protein gene ADF3RGDS#65
SEQ ID NO: 38, Amino acid sequence of cell adhesion sequence-inserted fibroin-like protein ADF3RGDS#65
SEQ ID NO: 39, Nucleotide sequence of cell adhesion sequence-inserted fibroin-like protein gene ADF3RGDS#68
SEQ ID NO: 40, Amino acid sequence of cell adhesion sequence-inserted fibroin-like protein ADF3RGDS#68
SEQ ID NO: 41, Nucleotide sequence of cell adhesion sequence-inserted fibroin-like protein gene ADF3RGDS#72
SEQ ID NO: 42, Amino acid sequence of cell adhesion sequence-inserted fibroin-like protein ADF3RGDS#72
SEQ ID NO: 43, Nucleotide sequence of cell adhesion sequence-inserted fibroin-like protein gene ADF3RGDS#73
SEQ ID NO: 44, Amino acid sequence of cell adhesion sequence-inserted fibroin-like protein ADF3RGDS#73
SEQ ID NO: 45, Nucleotide sequence of cell adhesion sequence-inserted fibroin-like protein gene ADF3RGDS#76
SEQ ID NO: 46, Amino acid sequence of cell adhesion sequence-inserted fibroin-like protein ADF3RGDS#76
SEQ ID NO: 47, Nucleotide sequence of cell adhesion sequence-inserted fibroin-like protein gene ADF3RGDS#77
SEQ ID NO: 48, Amino acid sequence of cell adhesion sequence-inserted fibroin-like protein ADF3RGDS#77
SEQ ID NO: 49, Nucleotide sequence of heparin-binding sequence-inserted fibroin-like protein gene ADF3RGDS#141
SEQ ID NO: 50, Amino acid sequence of heparin-binding sequence-inserted fibroin-like protein ADF3RGDS#141
SEQ ID NO: 51, Nucleotide sequence of cell adhesion sequence-inserted fibroin-like protein gene ADF3RGDS#66
SEQ ID NO: 52, Amino acid sequence of cell adhesion sequence-inserted fibroin-like protein ADF3RGDS#66
SEQ ID NO: 53, Nucleotide sequence of cell adhesion sequence-inserted fibroin-like protein gene ADF3RGDS#78
SEQ ID NO: 54, Amino acid sequence of cell adhesion sequence-inserted fibroin-like protein ADF3RGDS#78

### Industrial Applicability

According to the present invention, cells such as stem cells can be efficiently cultured.

## Claims

1. A protein comprising a repetitive structure and a cell adhesion sequence,
wherein the repetitive structure consists of a 3 to 15 times repeat of a repeating unit,
wherein each repeating unit consists of an Ala-rich region and an Ala-non-rich region mutually linked,
wherein each Ala-rich region consists of an amino acid sequence having a length of 8 to 54 residues,
wherein the ratio of Ala residue in each Ala-rich region is 30% or more, wherein each Ala-rich region contains at least one Ala residue in any continuous four amino acid residues,
wherein each Ala-non-rich region consists of an amino acid sequence having a length of 4 residues or longer,
wherein the ratio of Ala residue in each Ala-non-rich region is 20% or less, wherein the cell adhesion sequence is contained in one or more repeating units selected from repeating units constituting the repetitive structure, and
wherein each cell adhesion sequence consists of an amino acid sequence containing Arg-Gly-Asp and having a length of 3 to 18 residues.

2. The protein according to claim 1, wherein the cell adhesion sequence is contained in two or more repeating units selected from repeating units constituting the repetitive structure.

3. The protein according to claim 1 or 2, wherein the total number of the cell adhesion sequence contained in the protein is 1 to 50.

4. The protein according to any one of claims 1 to 3, wherein each cell adhesion sequence has been inserted in the Ala-non-rich region, or in the linkage site between the Ala-rich region and the Ala-non-rich region

5. The protein according to any one of claims 1 to 4, wherein each cell adhesion sequence consists of an amino acid sequence of (a), (b), or (c) shown below:
(a) the amino acid sequence of SEQ ID NO: 22, 23, 24, 25, 30, or 31;
(b) the amino acid sequence of SEQ ID NO: 22, 23, 24, 25, 30, or 31, including substitution, deletion, insertion, and/or addition of 1 to 3 amino acid residues at position(s) other than Arg-Gly-Asp;
(c) an amino acid sequence showing an identity of 80% or higher to the amino acid sequence of SEQ ID NO: 22, 23, 24, 25, 30, or 31, provided that Arg-Gly-Asp is conserved.

6. The protein according to any one of claims 1 to 5, wherein each Ala-rich region consists of an amino acid sequence having a length of 8 to 15 residues.

7. The protein according to any one of claims 1 to 6, wherein each Ala-rich region consists of an amino acid sequence having a length of 9 residues.

8. The protein according to any one of claims 1 to 7, wherein each Ala-rich region contains one or more Gly residues and one or more Ser residues.

9. The protein according to any one of claims 1 to 8, wherein the ratio of Ala residue in each Ala-rich region is 50% or more.

10. The protein according to any one of claims 1 to 9, wherein the ratio of Ala, Gly, and Ser residues in each Ala-rich region is 90% or more in total.

11. The protein according to any one of claims 1 to 10, wherein each Ala-rich region consists of an amino acid sequence of (a), (b), or (c) shown below:
(a) the amino acid sequence of SEQ ID NO: 7, 8, 9, or 10;
(b) the amino acid sequence of SEQ ID NO: 7, 8, 9, or 10, including substitution, deletion, insertion, and/or addition of 1 to 3 amino acid residues;
(c) an amino acid sequence showing an identity of 80% or higher to the amino acid sequence of SEQ ID NO: 7, 8, 9, or 10.

12. The protein according to any one of claims 1 to 11, wherein each Ala-non-rich region consists of an amino acid sequence having a length of 15 to 100 residues.

13. The protein according to any one of claims 1 to 12, wherein the ratio of Ala residue in each Ala-non-rich region is 5% or less.

14. The protein according to any one of claims 1 to 13, wherein the ratio of Gly, Ser, Gln, Pro, and Tyr residues in each Ala-non-rich region is 90% or more in total.

15. The protein according to any one of claims 1 to 14, wherein each Ala-non-rich region comprises one or more kinds of amino acid sequences selected from amino acid sequences of (a), (b), and (c) shown below:
(a) the amino acid sequences of SEQ ID NOS: 11 to 21;
(b) the amino acid sequences of SEQ ID NOS: 11 to 21, including substitution, deletion, insertion, and/or addition of 1 to 3 amino acid residues;
(c) amino acid sequences showing an identity of 80% or higher to the amino acid sequences of SEQ ID NOS: 11 to 21.

16. The protein according to any one of claims 1 to 15, wherein each Ala-non-rich region comprises an amino acid sequence of (a), (b), or (c) shown below:
(a) the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof;
(b) the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof, including substitution, deletion, insertion, and/or addition of 1 to 10 amino acid residues;
(c) an amino acid sequence showing an identity of 90% or higher to the amino acid sequence of amino acid numbers of 62 to 91, 101 to 120, 130 to 151, 161 to 185, 195 to 218, 228 to 257, 267 to 291, 301 to 325, 335 to 369, 379 to 438, 448 to 497, or 507 to 536 of SEQ ID NO: 2, or a partial sequence thereof.

17. A composition containing the protein according to any one of claims 1 to 16.

18. The composition according to claim 17, wherein the composition is a cell scaffold material.

19. The composition according to claim 18, wherein the cell scaffold material is for culturing a stem cell.

20. The composition according to claim 19, wherein the stem cell is an embryonic stem cell or an induced pluripotent stem cell.

21. The composition according to claim 17, wherein the composition is a coating agent for a cell culture vessel.

22. The composition according to claim 21, wherein the cell culture vessel is for culturing a stem cell.

23. The composition according to claim 22, wherein the stem cell is an embryonic stem cell or an induced pluripotent stem cell.

24. The composition according to any one of claims 17 to 23, wherein the composition further contains another protein, which is a protein other than the proteins according to claims 1 to 16.

25. The composition according to claim 24, wherein the other protein is any one of proteins (1) to (8) shown below:
(1) a protein that stabilizes a component in a culture medium;
(2) a protein comprising an amino acid sequence that stabilizes a component in a culture medium;
(3) a protein having a cell adhesion ability;
(4) a protein comprising an amino acid sequence having a cell adhesion ability;
(5) a protein that promotes proliferation of a cell;
(6) a protein comprising an amino acid sequence that promotes proliferation of a cell;
(7) a protein that binds to any one of the proteins (1) to (6);
(8) a protein comprising an amino acid sequence that binds to any one of the proteins (1) to (6).

26. An apparatus comprising the protein according to any one of claims 1 to 16.

27. The apparatus according to claim 26, wherein the apparatus is an apparatus for medical or research use.

28. The apparatus according to claim 26 or 27, wherein the apparatus is a cell culture vessel.

29. The apparatus according to claim 26 or 27, wherein the apparatus is a cell scaffold material.

30. The apparatus according to claim 28, wherein a cell culture surface of the apparatus has been coated with the protein.

31. The apparatus according to any one of claims 26 to 30, wherein the apparatus is for culturing a stem cell.

32. The apparatus according to claim 31, wherein the stem cell is an embryonic stem cell or an induced pluripotent stem cell.

33. The apparatus according to any one of claims 26 to 32, wherein the apparatus further comprises another protein, which is a protein other than the proteins according to of claims 1 to 16.

34. The apparatus according to claim 33, wherein the other protein is any one of proteins (1) to (8) shown below:
(1) a protein that stabilizes a component in a culture medium;
(2) a protein comprising an amino acid sequence that stabilizes a component in a culture medium;
(3) a protein having a cell adhesion ability;
(4) a protein comprising an amino acid sequence having a cell adhesion ability;
(5) a protein that promotes proliferation of a cell;
(6) a protein comprising an amino acid sequence that promotes proliferation of a cell;
(7) a protein that binds to any one of the proteins (1) to (6);
(8) a protein comprising an amino acid sequence that binds to any one of the proteins (1) to (6).

35. A method for producing a cultured cell, the method comprising:
culturing a cell by using the apparatus according to any one of claims 26 to 34.

36. The method according to claim 35, wherein the cell to be cultured is a stem cell.

37. The method according to claim 36, wherein the stem cell is an embryonic stem cell or an induced pluripotent stem cell.

38. The method according to any one of claims 35 to 37, wherein another protein, which is a protein other than the proteins according to claims 1 to 16, is further used in combination.

39. The method according to claim 38, wherein the other protein is any one of proteins (1) to (8) shown below:
(1) a protein that stabilizes a component in a culture medium;
(2) a protein comprising an amino acid sequence that stabilizes a component in a culture medium;
(3) a protein having a cell adhesion ability;
(4) a protein comprising an amino acid sequence having a cell adhesion ability;
(5) a protein that promotes proliferation of a cell;
(6) a protein comprising an amino acid sequence that promotes proliferation of a cell;
(7) a protein that binds to any one of the proteins (1) to (6);
(8) a protein comprising an amino acid sequence that binds to any one of the proteins (1) to (6).

40. A gene encoding the protein according to any one of claims 1 to 16.

41. A vector having the gene according to claim 40.

42. A host having the gene according to claim 40.


SEQUENCE LISTING

<110>  Ajinomoto Co., Inc.

<120>  Fibroin-like protein variant and method for culturing cell

<130>  D860-17052

<150>  JP2016-071530
<151>  2016-03-31

<150>  JP2016-207359
<151>  2016-10-21

<160>  54

<170>  PatentIn version 3.5

<210>  1
<211>  1998
<212>  DNA
<213>  Araneus diadematus

<400>  1
gcgcatatgc atcatcatca ccatcaccac catcatcata gcagcggtag cagcctggaa      60
gttctgtttc agggtccggc acgtgcaggt agcggtcagc agggtccggg tcagcaaggt     120
cctggccagc agggaccagg tcaacagggt ccgtatggtc cgggtgcaag cgcagctgca     180
gcagcagccg gtggttatgg tcctggttca ggtcagcagg gaccgtccca gcagggtcct     240
ggacaacaag gaccgggtgg tcagggtcct tatggccctg gtgccagtgc cgcagcagct     300
gccgcaggcg gttatggtcc aggtagtggc caacagggtc ctggtggtca gggaccatac     360
ggtccgggta gcagtgcagc agccgcagcg gctgggggta atggtccggg aagtggacag     420
cagggtgcag ggcagcaagg cccaggacag caaggtccgg gtgcctcagc agcggcagcc     480
gcagctggcg gatatggacc gggttcaggc caacaaggtc cgggacaaca gggtccaggc     540
ggacaaggtc cgtatggccc aggcgcatca gccgctgctg ccgcagcggg tggctatgga     600
ccaggcagtg gtcaaggtcc aggtcaacaa ggccctggtg gccaaggtcc gtacggtcct     660
ggtgcgtcag ccgcagcagc agctgcgggt ggatacggtc caggctcagg ccagcaaggc     720
cctgggcaac aagggcctgg tcaacaggga cctgggggac aaggccctta cggaccgggt     780
gcatctgcgg cagcggctgc agcgggtggt tacggaccgg gatatggcca gcaaggacct     840
ggtcagcaag gtccaggcgg tcagggaccg tatggaccgg gtgcgtcagc agcaagcgca     900
gccagtggtg gttatggccc aggatctggg cagcagggtc caggccaaca gggacctggc     960
ggacagggtc cttatgggcc aggggcatct gcagcggctg ctgctgccgg tggatatggg    1020
ccaggtagcg gacaacaagg gccaggccag cagggtcctg ggcagcaggg tcctggtcag    1080
caaggcccag gtggacaagg cccttatggt ccaggtgcga gtgcagccgc agcggcagcg    1140
ggtggatatg gccctggaag cggtcagcaa ggaccgggac aacaaggccc aggtcagcaa    1200
ggaccagggc aacagggacc gggacagcag ggacctggcc aacaaggccc tggacagcag    1260
ggaccgggac agcaagggcc aggacaacag ggtccaggtg gccagggtgc ctatggtcct    1320
ggcgcttcag cagcagcggg tgcagcaggg ggatatggtc ctggctctgg gcaacaaggc    1380
ccaggccaac aaggtccagg ccagcaaggg ccaggacagc agggacctgg tcaacaggga    1440
ccgggtcaac aaggtccggg acagcaaggg cctgggcagc aaggacctta tggacctggc    1500
gcaagcgctg cagctgccgc agcaggcggt tacggtccgg gatccggtca acaaggacct    1560
ggccaacagg gtcctggcca gcaaggtccg ggtggccaag gaccgtatgg gccaggtgca    1620
gccagcgcag ccgttagcgt tggtggctat ggtccgcaga gcagcagcgt tccggttgcc    1680
agcgcagttg caagccgtct gagcagtccg gcagcaagca gtcgtgttag cagcgcagtt    1740
agcagcctgg ttagcagcgg tccgaccaaa catgcagcac tgagcaatac cattagcagc    1800
gttgttagcc aggttagcgc aagcaatccg ggtctgagcg gttgtgatgt tctggttcag    1860
gcactgctgg aagttgttag cgcactggtg agcattctgg gtagcagcag cattggtcag    1920
attaactatg gtgccagcgc acagtatacc cagatggttg gtcagagcgt tgcacaggca    1980
ctggcataag aattcgcg                                                  1998

<210>  2
<211>  660
<212>  PRT

<213>  Araneus diadematus

<400>  2
Met His His His His His His His His His His Ser Ser Gly Ser Ser
1               5                   10                  15
Leu Glu Val Leu Phe Gln Gly Pro Ala Arg Ala Gly Ser Gly Gln Gln
            20                  25                  30
Gly Pro Gly Gln Gln Gly Pro Gly Gln Gln Gly Pro Gly Gln Gln Gly
        35                  40                  45
Pro Tyr Gly Pro Gly Ala Ser Ala Ala Ala Ala Ala Ala Gly Gly Tyr
    50                  55                  60
Gly Pro Gly Ser Gly Gln Gln Gly Pro Ser Gln Gln Gly Pro Gly Gln
65                  70                  75                  80
Gln Gly Pro Gly Gly Gln Gly Pro Tyr Gly Pro Gly Ala Ser Ala Ala
                85                  90                  95
Ala Ala Ala Ala Gly Gly Tyr Gly Pro Gly Ser Gly Gln Gln Gly Pro
            100                 105                 110
Gly Gly Gln Gly Pro Tyr Gly Pro Gly Ser Ser Ala Ala Ala Ala Ala
        115                 120                 125
Ala Gly Gly Asn Gly Pro Gly Ser Gly Gln Gln Gly Ala Gly Gln Gln
    130                 135                 140
Gly Pro Gly Gln Gln Gly Pro Gly Ala Ser Ala Ala Ala Ala Ala Ala
145                 150                 155                 160
Gly Gly Tyr Gly Pro Gly Ser Gly Gln Gln Gly Pro Gly Gln Gln Gly
                165                 170                 175
Pro Gly Gly Gln Gly Pro Tyr Gly Pro Gly Ala Ser Ala Ala Ala Ala
            180                 185                 190
Ala Ala Gly Gly Tyr Gly Pro Gly Ser Gly Gln Gly Pro Gly Gln Gln
        195                 200                 205
Gly Pro Gly Gly Gln Gly Pro Tyr Gly Pro Gly Ala Ser Ala Ala Ala
    210                 215                 220
Ala Ala Ala Gly Gly Tyr Gly Pro Gly Ser Gly Gln Gln Gly Pro Gly
225                 230                 235                 240
Gln Gln Gly Pro Gly Gln Gln Gly Pro Gly Gly Gln Gly Pro Tyr Gly
                245                 250                 255
Pro Gly Ala Ser Ala Ala Ala Ala Ala Ala Gly Gly Tyr Gly Pro Gly
            260                 265                 270
Tyr Gly Gln Gln Gly Pro Gly Gln Gln Gly Pro Gly Gly Gln Gly Pro
        275                 280                 285
Tyr Gly Pro Gly Ala Ser Ala Ala Ser Ala Ala Ser Gly Gly Tyr Gly
    290                 295                 300
Pro Gly Ser Gly Gln Gln Gly Pro Gly Gln Gln Gly Pro Gly Gly Gln
305                 310                 315                 320
Gly Pro Tyr Gly Pro Gly Ala Ser Ala Ala Ala Ala Ala Ala Gly Gly
                325                 330                 335
Tyr Gly Pro Gly Ser Gly Gln Gln Gly Pro Gly Gln Gln Gly Pro Gly
            340                 345                 350
Gln Gln Gly Pro Gly Gln Gln Gly Pro Gly Gly Gln Gly Pro Tyr Gly
        355                 360                 365
Pro Gly Ala Ser Ala Ala Ala Ala Ala Ala Gly Gly Tyr Gly Pro Gly
    370                 375                 380
Ser Gly Gln Gln Gly Pro Gly Gln Gln Gly Pro Gly Gln Gln Gly Pro
385                 390                 395                 400
Gly Gln Gln Gly Pro Gly Gln Gln Gly Pro Gly Gln Gln Gly Pro Gly
                405                 410                 415
Gln Gln Gly Pro Gly Gln Gln Gly Pro Gly Gln Gln Gly Pro Gly Gly
            420                 425                 430
Gln Gly Ala Tyr Gly Pro Gly Ala Ser Ala Ala Ala Gly Ala Ala Gly
        435                 440                 445
Gly Tyr Gly Pro Gly Ser Gly Gln Gln Gly Pro Gly Gln Gln Gly Pro
    450                 455                 460
Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495
Pro Gly Ala Ser Ala Ala Ala Ala Ala Ala Gly Gly Tyr Gly Pro Gly
            500                 505                 510
Ser Gly Gln Gln Gly Pro Gly Gln Gln Gly Pro Gly Gln Gln Gly Pro
        515                 520                 525
Gly Gly Gln Gly Pro Tyr Gly Pro Gly Ala Ala Ser Ala Ala Val Ser
    530                 535                 540
Val Gly Gly Tyr Gly Pro Gln Ser Ser Ser Val Pro Val Ala Ser Ala
545                 550                 555                 560
Val Ala Ser Arg Leu Ser Ser Pro Ala Ala Ser Ser Arg Val Ser Ser
                565                 570                 575
Ala Val Ser Ser Leu Val Ser Ser Gly Pro Thr Lys His Ala Ala Leu
            580                 585                 590
Ser Asn Thr Ile Ser Ser Val Val Ser Gln Val Ser Ala Ser Asn Pro
        595                 600                 605
Gly Leu Ser Gly Cys Asp Val Leu Val Gln Ala Leu Leu Glu Val Val
    610                 615                 620
Ser Ala Leu Val Ser Ile Leu Gly Ser Ser Ser Ile Gly Gln Ile Asn
625                 630                 635                 640
Tyr Gly Ala Ser Ala Gln Tyr Thr Gln Met Val Gly Gln Ser Val Ala
                645                 650                 655
Gln Ala Leu Ala
                660

<210>  3
<211>  2457
<212>  DNA
<213>  Araneus diadematus

<400>  3
gcgccatatg catcatcatc accatcacca ccatcatcat agcagcggta gcagcctgga      60
agttctgttt cagggtccgg cacgtgcagg tagcggtcag cagggtccgg gtcagcaagg     120
tcctggccag cagggaccag gtcaacaggg tccgtatggt ccgggtgcaa gcgcagctgc     180
agcagcagcc ggtggttatg gtcctggttc aggtcagcag ggaccgtccc agcagggtcc     240
tggacaacaa ggaccgggtg gtcagggtcc ttatggccct ggtgccagtg ccgcagcagc     300
tgccgcaggc ggttatggtc caggtagtgg ccagggtcgt ggtgatagtc cgcagggtcc     360
aggcggtcag ggtccatacg gtccaggtag cagtgcagca gccgcagcag cgggtggcaa     420
cggtcctggt agtggacagc agggtgcagg ccaacaaggc ccaggccagc aaggtccggg     480
tgcctcagca gcagcggcag cggcaggggg ttatggaccg ggttctggcc aacagggtcg     540
cggtgattca ccgggtccgg gacaacaggg tccaggtggc caaggtccgt atggcccagg     600
cgcatcagct gctgcagcgg ctgcgggtgg ctatggccca ggttctggtc agggacgtgg     660
cgatagccct ggtcctgggc agcaaggccc tggtggccaa ggcccttacg gtccgggtgc     720
ttctgctgcg gcagcagctg ctggcggata tggtccggga agcggtcagc aaggcccagg     780
acaacaaggc cctggtcaac agggacctgg cggacaggga ccgtacggac ctggtgcatc     840
agcggcagct gcggcagccg gtggatacgg accgggttat ggacagcaag gtcgtggcga     900
cagtccggga ccgggacagc aaggaccagg cggacaaggc ccatacggtc ctggcgcaag     960
tgcagcaagc gcagcaagtg gtggttatgg accaggcagc ggacagcagg gacgtggtga    1020
ctctccgggt ccagggcagc agggaccggg tggccaggga ccgtatggtc caggtgcttc    1080
agcagccgca gccgcagctg gcggttacgg acctggcagt ggtcaacaag ggccaggaca    1140
gcagggacct ggacagcagg gtccagggca acagggacct ggcggtcaag gtccttatgg    1200
accgggtgca tctgccgcag cggcagctgc gggtggatat ggtcctggca gtgggcaaca    1260
gggtccgggt caacagggac cgggacaaca aggtccagga cagcaaggcc gtggtgatag    1320
cccaggccca ggccaacagg gaccgggtca gcaaggccca ggtcaacaag gtccgggtca    1380
acaaggccca ggtcagcagg gtccaggggg acagggtgct tatggaccag gtgcgagcgc    1440
agcagcaggg gctgcagggg gatacggacc aggctcaggg caacaaggac cagggcaaca    1500
agggcctggg cagcaaggac caggccagca gggtcgtggc gattctccag gccctgggca    1560
acaaggtcct ggccaacaag gtccgggaca gcagggtccg ggacagcaag gaccgtatgg    1620
tccgggtgcc agtgcggcag ccgcagcggc tgggggatac ggtcctggat ctggccagca    1680
agggcctggt cagcaaggcc ctgggcaaca gggtccaggg ggtcaaggac cttacggacc    1740
aggcgcagcc agcgcagcag ttagcgttgg tggctatggt ccgcagagca gcagcgttcc    1800
ggttgccagc gcagttgcaa gccgtctgag cagtccggca gcaagcagcc gtgttagcag    1860

cgcagttagc agcctggtga gcagcggtcc gaccaaacat gcagcactga gcaataccat 1920
tagcagcgtt gttagccagg ttagcgcaag caatccgggt ctgagcggtt gtgatgttct 1980
ggttcaggca ctgctggaag ttgttagcgc actggttagc attctgggta gcagcagcat 2040
tggtcagatt aactatggtg ccagcgcaca gtatacccag atggttggtc agagcgttgc 2100
acaggcactg gcataatccc acagccgcca gttccgctgg cggcatttta actttcttta 2160
atgaagccgg aaaaatccta aattcattta atatttatct ttttaccgtt tcgcttaccc 2220
cggtcgaacg tcaacttacg tcatttttcc gcccaacagt aatataatca aacaaattaa 2280
tcccgcaaca taacaccagt aaaatcaata attttctcta agtcacttat tcctcaggta 2340
attgttaata tatccagaat gttcctcaaa atatattttc cctctatctt ctcgttgcgc 2400
ttaatttgac taattctcat tagcgactaa ttttaatgag tgtcgacgaa ttcgcgc 2457

<210> 4
<211> 702
<212> PRT
<213> Araneus diadematus

<400> 4
Met His His His His His His His His His His Ser Ser Gly Ser Ser
1               5                   10                  15
Leu Glu Val Leu Phe Gln Gly Pro Ala Arg Ala Gly Ser Gly Gln Gln
            20                  25                  30
Gly Pro Gly Gln Gln Gly Pro Gly Gln Gln Gly Pro Gly Gln Gln Gly
        35                  40                  45
Pro Tyr Gly Pro Gly Ala Ser Ala Ala Ala Ala Ala Ala Gly Gly Tyr
    50                  55                  60
Gly Pro Gly Ser Gly Gln Gln Gly Pro Ser Gln Gln Gly Pro Gly Gln
65                  70                  75                  80
Gln Gly Pro Gly Gly Gln Gly Pro Tyr Gly Pro Gly Ala Ser Ala Ala
                85                  90                  95
Ala Ala Ala Ala Gly Gly Tyr Gly Pro Gly Ser Gly Gln Gly Arg Gly
            100                 105                 110
Asp Ser Pro Gln Gly Pro Gly Gly Gln Gly Pro Tyr Gly Pro Gly Ser
        115                 120                 125
Ser Ala Ala Ala Ala Ala Ala Gly Gly Asn Gly Pro Gly Ser Gly Gln
    130                 135                 140
Gln Gly Ala Gly Gln Gln Gly Pro Gly Gln Gln Gly Pro Gly Ala Ser
145                 150                 155                 160
Ala Ala Ala Ala Ala Ala Gly Gly Tyr Gly Pro Gly Ser Gly Gln Gln
                165                 170                 175
Gly Arg Gly Asp Ser Pro Gly Pro Gly Gln Gln Gly Pro Gly Gly Gln
            180                 185                 190
Gly Pro Tyr Gly Pro Gly Ala Ser Ala Ala Ala Ala Ala Ala Gly Gly
        195                 200                 205
Tyr Gly Pro Gly Ser Gly Gln Gly Arg Gly Asp Ser Pro Gly Pro Gly
    210                 215                 220
Gln Gln Gly Pro Gly Gly Gln Gly Pro Tyr Gly Pro Gly Ala Ser Ala
225                 230                 235                 240
Ala Ala Ala Ala Ala Gly Gly Tyr Gly Pro Gly Ser Gly Gln Gln Gly
                245                 250                 255
Pro Gly Gln Gln Gly Pro Gly Gln Gln Gly Pro Gly Gly Gln Gly Pro
            260                 265                 270
Tyr Gly Pro Gly Ala Ser Ala Ala Ala Ala Ala Ala Gly Gly Tyr Gly
        275                 280                 285
Pro Gly Tyr Gly Gln Gln Gly Arg Gly Asp Ser Pro Gly Pro Gly Gln
    290                 295                 300
Gln Gly Pro Gly Gly Gln Gly Pro Tyr Gly Pro Gly Ala Ser Ala Ala
305                 310                 315                 320
Ser Ala Ala Ser Gly Gly Tyr Gly Pro Gly Ser Gly Gln Gln Gly Arg
                325                 330                 335
Gly Asp Ser Pro Gly Pro Gly Gln Gln Gly Pro Gly Gly Gln Gly Pro
            340                 345                 350
Tyr Gly Pro Gly Ala Ser Ala Ala Ala Ala Ala Ala Gly Gly Tyr Gly

355                 360                 365
Pro Gly Ser Gly Gln Gln Gly Pro Gly Gln Gln Gly Pro Gly Gln Gln
    370                 375                 380
Gly Pro Gly Gln Gln Gly Pro Gly Gly Gln Gly Pro Tyr Gly Pro Gly
385                 390                 395                 400
Ala Ser Ala Ala Ala Ala Ala Ala Gly Gly Tyr Gly Pro Gly Ser Gly
                405                 410                 415
Gln Gln Gly Pro Gly Gln Gln Gly Pro Gly Gln Gln Gly Pro Gly Gln
            420                 425                 430
Gln Gly Arg Gly Asp Ser Pro Gly Pro Gly Gln Gln Gly Pro Gly Gln
        435                 440                 445
Gln Gly Pro Gly Gln Gln Gly Pro Gly Gln Gln Gly Pro Gly Gln Gln
    450                 455                 460
Gly Pro Gly Gly Gln Gly Ala Tyr Gly Pro Gly Ala Ser Ala Ala Ala
465                 470                 475                 480
Gly Ala Ala Gly Gly Tyr Gly Pro Gly Ser Gly Gln Gln Gly Pro Gly
                485                 490                 495
Gln Gln Gly Pro Gly Gln Gln Gly Pro Gly Gln Gln Gly Arg Gly Asp
            500                 505                 510
Ser Pro Gly Pro Gly Gln Gln Gly Pro Gly Gln Gln Gly Pro Gly Gln
        515                 520                 525
Gln Gly Pro Gly Gln Gln Gly Pro Tyr Gly Pro Gly Ala Ser Ala Ala
    530                 535                 540
Ala Ala Ala Ala Gly Gly Tyr Gly Pro Gly Ser Gly Gln Gln Gly Pro
545                 550                 555                 560
Gly Gln Gln Gly Pro Gly Gln Gln Gly Pro Gly Gly Gln Gly Pro Tyr
                565                 570                 575
Gly Pro Gly Ala Ala Ser Ala Ala Val Ser Val Gly Gly Tyr Gly Pro
            580                 585                 590
Gln Ser Ser Ser Val Pro Val Ala Ser Ala Val Ala Ser Arg Leu Ser
        595                 600                 605
Ser Pro Ala Ala Ser Ser Arg Val Ser Ser Ala Val Ser Ser Leu Val
    610                 615                 620
Ser Ser Gly Pro Thr Lys His Ala Ala Leu Ser Asn Thr Ile Ser Ser
625                 630                 635                 640
Val Val Ser Gln Val Ser Ala Ser Asn Pro Gly Leu Ser Gly Cys Asp
                645                 650                 655
Val Leu Val Gln Ala Leu Leu Glu Val Val Ser Ala Leu Val Ser Ile
            660                 665                 670
Leu Gly Ser Ser Ser Ile Gly Gln Ile Asn Tyr Gly Ala Ser Ala Gln
        675                 680                 685
Tyr Thr Gln Met Val Gly Gln Ser Val Ala Gln Ala Leu Ala
    690                 695                 700

<210>  5
<211>  2541
<212>  DNA
<213>  Araneus diadematus

<400>  5
gcgccatatg catcatcatc accatcacca ccatcatcat agcagcggta gcagcctgga      60
agttctgttt cagggtccgg cacgtgcagg tagcggtcag cagggtccgg gtcagcaagg     120
tcctggccag cagggaccag gtcaacaggg tccgtatggt ccgggtgcaa gcgcagctgc     180
agcagcagcc ggtggttatg gtcctggttc aggtcagcag ggaccgtccc agcagggtcc     240
tggacaacaa ggaccgggcg gtcagggtcc ttatggccct ggtgccagtg ccgcagcagc     300
tgccgcaggc ggttatggtc caggtagtgg tcaggttacc ggtcgtggtg atagtccggc     360
aagccagggt cctggtggtc agggaccata cggtccgggt agcagtgcag cagccgcagc     420
ggctgggggt aatggtcctg gtagcggaca gcaaggcgca ggacagcagg gacctggcca     480
gcaaggccct ggtgcatctg cagcggctgc agcagctggc ggatatggac cgggtagtgg     540
ccagcaggtt acaggccgtg gcgattcacc ggcaagcggt ccgggtcaac agggacctgg     600
tggccaagga ccgtatggcc caggtgcatc agctgcggca gcggcagcgg gtggctatgg     660
accaggctct ggtcaggtga caggtcgcgg agattctccg gcatcaggtc cgggacagca     720

gggtccaggc ggtcaaggtc cgtatggacc tggcgcttca gcagcagcgg cagctgcggg 780
tggatatggc cctggcagcg gtcaacaagg accagggcag caaggaccgg gacaacaagg 840
cccaggcgga cagggaccat acgggcctgg tgcgtcagcc gcagccgctg ctgccggtgg 900
ttacggaccg ggttatggtc aacaggtgac cggacgcggt gatagccctg ccagcggtcc 960
aggccaacaa gggcctgggg gtcaaggccc ttacggtcct ggtgcatcag cggcaagcgc 1020
agcaagtggt ggctatggtc cgggatctgg acagcaggtt acgggtcggg gagatagccc 1080
tgcgtcaggt ccaggacagc aaggcccagg tggccaaggc ccttatggac cgggtgcgtc 1140
tgcagccgca gctgcagcgg gtggatacgg tccgggaagc ggacaacagg gtcctgggca 1200
acagggtcca ggccagcagg gaccgggaca gcagggacca ggtggccagg gtccttacgg 1260
tccaggcgca tctgctgccg ctgcagccgc agggggttac ggtccgggat caggccagca 1320
agggccaggc caacaaggtc caggacaaca gggtccgggt caacaagtta ccggtcgcgg 1380
tgactcacca gccagtggcc ctggacagca agggcctggt caacagggac ctggacagca 1440
gggtcctggt cagcagggtc cgggacaaca gggacctggc ggacagggtg cgtatgggcc 1500
aggcgctagt gcagcagcgg gtgcagctgg cggttacggt cctggctctg gccaacaagg 1560
acctggtcag caaggcccag ggcagcaggg tccagggcag caagttacgg gtcgtggcga 1620
cagcccagca agtggccctg gccaacaggg tcctggccaa caaggtcctg gacagcaagg 1680
accgggtcag cagggtcctt acggaccggg tgcttcggca gcagctgcag cagccggtgg 1740
ctacggacct ggcagtggac agcaaggtcc agggcaacaa ggccctggcc agcaaggtcc 1800
aggtgggcag ggaccgtacg gacctggtgc agcctcagca gcagttagcg ttggtggtta 1860
tggaccgcag agcagcagcg ttccggttgc cagcgcagtt gcaagccgtc tgagcagtcc 1920
ggcagcaagc agccgtgtta gcagcgcagt tagcagcctg gtgagcagcg gtccgaccaa 1980
acatgcagca ctgagcaata ccattagcag cgttgttagc caggttagcg caagcaatcc 2040
gggtctgagc ggttgtgatg ttctggttca ggcactgctg gaagttgtta gcgcactggt 2100
tagcattctg ggtagcagca gcattggtca gattaactat ggtgccagcg cacagtatac 2160
ccagatggtt ggtcagagcg ttgcacaggc actggcataa tcccacagcc gccagttccg 2220
ctggcggcat tttaactttc tttaatgaag ccggaaaaat cctaaattca tttaatattt 2280
atctttttac cgtttcgctt accccggtcg aacgtcaact tacgtcattt ttccgcccaa 2340
cagtaatata atcaaacaaa ttaatcccgc aacataacac cagtaaaatc aataattttc 2400
tctaagtcac ttattcctca ggtaattgtt aatatatcca gaatgttcct caaaatatat 2460
tttccctcta tcttctcgtt gcgcttaatt tgactaattc tcattagcga ctaattttaa 2520
tgagtgtcga cgaattcgcg c 2541

<210> 6
<211> 730
<212> PRT
<213> Araneus diadematus

<400> 6
Met His His His His His His His His His His Ser Ser Gly Ser Ser
1               5                   10                  15
Leu Glu Val Leu Phe Gln Gly Pro Ala Arg Ala Gly Ser Gly Gln Gln
            20                  25                  30
Gly Pro Gly Gln Gln Gly Pro Gly Gln Gln Gly Pro Gly Gln Gln Gly
        35                  40                  45
Pro Tyr Gly Pro Gly Ala Ser Ala Ala Ala Ala Ala Ala Gly Gly Tyr
    50                  55                  60
Gly Pro Gly Ser Gly Gln Gln Gly Pro Ser Gln Gln Gly Pro Gly Gln
65                  70                  75                  80
Gln Gly Pro Gly Gly Gln Gly Pro Tyr Gly Pro Gly Ala Ser Ala Ala
                85                  90                  95
Ala Ala Ala Ala Gly Gly Tyr Gly Pro Gly Ser Gly Gln Val Thr Gly
            100                 105                 110
Arg Gly Asp Ser Pro Ala Ser Gln Gly Pro Gly Gly Gln Gly Pro Tyr
        115                 120                 125
Gly Pro Gly Ser Ser Ala Ala Ala Ala Ala Ala Gly Gly Asn Gly Pro
    130                 135                 140
Gly Ser Gly Gln Gln Gly Ala Gly Gln Gln Gly Pro Gly Gln Gln Gly
145                 150                 155                 160
Pro Gly Ala Ser Ala Ala Ala Ala Ala Ala Gly Gly Tyr Gly Pro Gly
                165                 170                 175
Ser Gly Gln Gln Val Thr Gly Arg Gly Asp Ser Pro Ala Ser Gly Pro
            180                 185                 190

Gly Gln Gln Gly Pro Gly Gly Gln Gly Pro Tyr Gly Pro Gly Ala Ser
        195                 200                 205
Ala Ala Ala Ala Ala Ala Gly Gly Tyr Gly Pro Gly Ser Gly Gln Val
    210                 215                 220
Thr Gly Arg Gly Asp Ser Pro Ala Ser Gly Pro Gly Gln Gln Gly Pro
225                 230                 235                 240
Gly Gly Gln Gly Pro Tyr Gly Pro Gly Ala Ser Ala Ala Ala Ala Ala
                245                 250                 255
Ala Gly Gly Tyr Gly Pro Gly Ser Gly Gln Gln Gly Pro Gly Gln Gln
            260                 265                 270
Gly Pro Gly Gln Gln Gly Pro Gly Gly Gln Gly Pro Tyr Gly Pro Gly
        275                 280                 285
Ala Ser Ala Ala Ala Ala Ala Ala Gly Gly Tyr Gly Pro Gly Tyr Gly
    290                 295                 300
Gln Gln Val Thr Gly Arg Gly Asp Ser Pro Ala Ser Gly Pro Gly Gln
305                 310                 315                 320
Gln Gly Pro Gly Gly Gln Gly Pro Tyr Gly Pro Gly Ala Ser Ala Ala
                325                 330                 335
Ser Ala Ala Ser Gly Gly Tyr Gly Pro Gly Ser Gly Gln Gln Val Thr
            340                 345                 350
Gly Arg Gly Asp Ser Pro Ala Ser Gly Pro Gly Gln Gln Gly Pro Gly
        355                 360                 365
Gly Gln Gly Pro Tyr Gly Pro Gly Ala Ser Ala Ala Ala Ala Ala Ala
    370                 375                 380
Gly Gly Tyr Gly Pro Gly Ser Gly Gln Gln Gly Pro Gly Gln Gln Gly
385                 390                 395                 400
Pro Gly Gln Gln Gly Pro Gly Gln Gln Gly Pro Gly Gly Gln Gly Pro
                405                 410                 415
Tyr Gly Pro Gly Ala Ser Ala Ala Ala Ala Ala Ala Gly Gly Tyr Gly
            420                 425                 430
Pro Gly Ser Gly Gln Gln Gly Pro Gly Gln Gln Gly Pro Gly Gln Gln
        435                 440                 445
Gly Pro Gly Gln Gln Val Thr Gly Arg Gly Asp Ser Pro Ala Ser Gly
    450                 455                 460
Pro Gly Gln Gln Gly Pro Gly Gln Gln Gly Pro Gly Gln Gln Gly Pro
465                 470                 475                 480
Gly Gln Gln Gly Pro Gly Gln Gln Gly Pro Gly Gly Gln Gly Ala Tyr
                485                 490                 495
Gly Pro Gly Ala Ser Ala Ala Ala Gly Ala Ala Gly Gly Tyr Gly Pro
            500                 505                 510
Gly Ser Gly Gln Gln Gly Pro Gly Gln Gln Gly Pro Gly Gln Gln Gly
        515                 520                 525
Pro Gly Gln Gln Val Thr Gly Arg Gly Asp Ser Pro Ala Ser Gly Pro
    530                 535                 540
Gly Gln Gln Gly Pro Gly Gln Gln Gly Pro Gly Gln Gln Gly Pro Gly
545                 550                 555                 560
Gln Gln Gly Pro Tyr Gly Pro Gly Ala Ser Ala Ala Ala Ala Ala Ala
                565                 570                 575
Gly Gly Tyr Gly Pro Gly Ser Gly Gln Gln Gly Pro Gly Gln Gln Gly
            580                 585                 590
Pro Gly Gln Gln Gly Pro Gly Gly Gln Gly Pro Tyr Gly Pro Gly Ala
        595                 600                 605
Ala Ser Ala Ala Val Ser Val Gly Gly Tyr Gly Pro Gln Ser Ser Ser
    610                 615                 620
Val Pro Val Ala Ser Ala Val Ala Ser Arg Leu Ser Ser Pro Ala Ala
625                 630                 635                 640
Ser Ser Arg Val Ser Ser Ala Val Ser Ser Leu Val Ser Ser Gly Pro
                645                 650                 655
Thr Lys His Ala Ala Leu Ser Asn Thr Ile Ser Ser Val Val Ser Gln
            660                 665                 670
Val Ser Ala Ser Asn Pro Gly Leu Ser Gly Cys Asp Val Leu Val Gln
        675                 680                 685
Ala Leu Leu Glu Val Val Ser Ala Leu Val Ser Ile Leu Gly Ser Ser

690                 695                 700
Ser Ile Gly Gln Ile Asn Tyr Gly Ala Ser Ala Gln Tyr Thr Gln Met
705                 710                 715                 720
Val Gly Gln Ser Val Ala Gln Ala Leu Ala
                725                 730

<210>  7
<211>  9
<212>  PRT
<213>  Araneus diadematus

<400>  7
Gly Ala Ser Ala Ala Ala Ala Ala Ala
1               5

<210>  8
<211>  9
<212>  PRT
<213>  Araneus diadematus

<400>  8
Gly Ser Ser Ala Ala Ala Ala Ala Ala
1               5

<210>  9
<211>  9
<212>  PRT
<213>  Araneus diadematus

<400>  9
Gly Ala Ser Ala Ala Ser Ala Ala Ser
1               5

<210>  10
<211>  9
<212>  PRT
<213>  Araneus diadematus

<400>  10
Gly Ala Ser Ala Ala Ala Gly Ala Ala
1               5

<210>  11
<211>  11
<212>  PRT
<213>  Araneus diadematus

<400>  11
Gly Gly Tyr Gly Pro Gly Ser Gly Gln Gln Gly
1               5                   10

<210>  12
<211>  11
<212>  PRT
<213>  Araneus diadematus

<400>  12

Gly Gly Asn Gly Pro Gly Ser Gly Gln Gln Gly
1               5                   10


<210>  13
<211>  11
<212>  PRT
<213>  Araneus diadematus

<400>  13
Gly Gly Tyr Gly Pro Gly Tyr Gly Gln Gln Gly
1               5                   10


<210>  14
<211>  10
<212>  PRT
<213>  Araneus diadematus

<400>  14
Gly Gly Tyr Gly Pro Gly Ser Gly Gln Gly
1               5                   10


<210>  15
<211>  5
<212>  PRT
<213>  Araneus diadematus

<400>  15
Pro Gly Gln Gln Gly
1               5


<210>  16
<211>  5
<212>  PRT
<213>  Araneus diadematus

<400>  16
Ala Gly Gln Gln Gly
1               5


<210>  17
<211>  5
<212>  PRT
<213>  Araneus diadematus

<400>  17
Ser Gly Gln Gln Gly
1               5


<210>  18
<211>  5
<212>  PRT
<213>  Araneus diadematus

<400>  18
Pro Ser Gln Gln Gly
1               5

<210>  19
<211>  5
<212>  PRT
<213>  Araneus diadematus

<400>  19
Pro Gly Gly Gln Gly
1               5


<210>  20
<211>  4
<212>  PRT
<213>  Araneus diadematus

<400>  20
Pro Tyr Gly Pro
1


<210>  21
<211>  4
<212>  PRT
<213>  Araneus diadematus

<400>  21
Ala Tyr Gly Pro
1


<210>  22
<211>  6
<212>  PRT
<213>  Homo sapiens

<400>  22
Gly Arg Gly Asp Ser Pro
1               5


<210>  23
<211>  10
<212>  PRT
<213>  Homo sapiens

<400>  23
Val Thr Gly Arg Gly Asp Ser Pro Ala Ser
1               5                   10


<210>  24
<211>  11
<212>  PRT
<213>  Homo sapiens

<400>  24
Pro Gln Val Thr Arg Gly Asp Val Phe Thr Met
1               5                   10


<210>  25
<211>  7

<212>  PRT
<213>  Homo sapiens

<400>  25
Val Thr Arg Gly Asp Val Phe
1               5


<210>  26
<211>  2190
<212>  DNA
<213>  Araneus diadematus

<400>  26
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggtagtggt     300
cagccgcagg ttacccgtgg tgatgttttt accatgcagg gtcctggtgg ccaaggtccg     360
tacggtcctg gtagtagcgc tgctgccgca gcagcgggtg gtaacggtcc gggtagcgga     420
caacagggag ccggtcaaca agggcccggg caacaaggtc ctggcgcaag cgcagccgct     480
gctgctgcgg gtggctatgg cccaggatca ggtcagcagc ctcaggtgac acgcggagat     540
gtgtttacaa tgggtcccgg acagcagggc ccaggcggac aaggccctta tggtcccgga     600
gctagcgcag ctgcagcggc tgccggagga tacggtccag gctctggcca gccgcaagta     660
acacgtggcg acgtgttcac catgggaccg gggcagcaag ggccaggtgg tcaaggacca     720
tacggaccag gtgccagtgc ggctgctgca gccgccggtg gctacggacc tggaagcggc     780
caacaaggcc caggacagca ggggcctgga caacagggtc ccggtggaca gggcccatac     840
ggcccgggtg cgagcgcagc ggccgctgcc gctggcggtt acggacccgg ttatggccag     900
caaccacagg tgacgcgtgg ggacgtattt acgatggggc caggccaaca gggccctgga     960
gggcagggac cttacggccc aggcgcatca gcagccagcg cagcaagtgg aggttatgga    1020
ccaggcagcg gtcaacagcc acaagtcact cgcggtgacg tctttactat gggacctggt    1080
caacaaggtc cgggaggaca aggaccgtat ggccctggag cctctgccgc agccgcagca    1140
gctggcggat acggtcctgg cagcggtcag caaggaccag gccaacaagg gccgggtcag    1200
cagggaccag gacagcaagg tcccggaggt caagggcctt acggtccggg tgcttcagcc    1260
gctgcagccg cagcgggagg gtacggaccg ggttcaggtc aacagggccc aggtcaacag    1320
ggtcccgggc agcaggggcc tggtcagcag ccgcaagtta ctagaggtga tgtgttcacg    1380
atgggccctg gccagcaagg acccgggcag caaggacctg gacaacaagg cccagggcaa    1440
cagggaccgg gtcaacaagg acctggcgga cagggtgcct acggtcccgg ggcctccgcg    1500
gctgcgggag ctgctggagg gtatgggcct ggcagtggac agcagggtcc aggccagcag    1560
ggtcctggcc aacaagggcc tggacaacaa ccgcaggtta cacgcggtga cgtattcaca    1620
atgggcccag ggcagcaggg tccagggcaa cagggcccag gacagcaagg cccaggtcag    1680
caaggtccgt atggtccagg ggcatcagcg gctgctgcgg ctgctggggg ttatggtccg    1740
ggatctgggc aacaaggacc cggccaacag gggccaggcc agcaaggacc tggaggacaa    1800
ggtccttatg gaccgggtgc agcaagcgca gcagttagcg ttggtggcta tggtccgcag    1860
agcagcagcg ttccggttgc cagtgcagtt gcaagccgtc tgagcagtcc ggcagcaagc    1920
agtcgtgtga gcagcgcagt tagcagcctg gttagcagtg gtccgaccaa acatgcagca    1980
ctgagcaata ccattagcag cgttgttagc caggttagcg caagcaatcc gggtctgagc    2040
ggttgtgatg ttctggtgca ggcactgctg gaagttgtta gcgcactggt gagcattctg    2100
ggtagcagca gcattggtca gattaactat ggtgccagcg cacagtatac ccagatggtt    2160
ggtcagagcg ttgcacaggc actggcataa                                     2190


<210>  27
<211>  729
<212>  PRT
<213>  Araneus diadematus

<400>  27
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30

Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Pro Gln Val Thr Arg Gly Asp Val Phe Thr Met
            100                 105                 110
Gln Gly Pro Gly Gly Gln Gly Pro Tyr Gly Pro Gly Ser Ser Ala Ala
        115                 120                 125
Ala Ala Ala Ala Gly Gly Asn Gly Pro Gly Ser Gly Gln Gln Gly Ala
    130                 135                 140
Gly Gln Gln Gly Pro Gly Gln Gln Gly Pro Gly Ala Ser Ala Ala Ala
145                 150                 155                 160
Ala Ala Ala Gly Gly Tyr Gly Pro Gly Ser Gly Gln Gln Pro Gln Val
                165                 170                 175
Thr Arg Gly Asp Val Phe Thr Met Gly Pro Gly Gln Gln Gly Pro Gly
            180                 185                 190
Gly Gln Gly Pro Tyr Gly Pro Gly Ala Ser Ala Ala Ala Ala Ala Ala
        195                 200                 205
Gly Gly Tyr Gly Pro Gly Ser Gly Gln Pro Gln Val Thr Arg Gly Asp
    210                 215                 220
Val Phe Thr Met Gly Pro Gly Gln Gln Gly Pro Gly Gly Gln Gly Pro
225                 230                 235                 240
Tyr Gly Pro Gly Ala Ser Ala Ala Ala Ala Ala Ala Gly Gly Tyr Gly
                245                 250                 255
Pro Gly Ser Gly Gln Gln Gly Pro Gly Gln Gln Gly Pro Gly Gln Gln
            260                 265                 270
Gly Pro Gly Gly Gln Gly Pro Tyr Gly Pro Gly Ala Ser Ala Ala Ala
        275                 280                 285
Ala Ala Ala Gly Gly Tyr Gly Pro Gly Tyr Gly Gln Gln Pro Gln Val
    290                 295                 300
Thr Arg Gly Asp Val Phe Thr Met Gly Pro Gly Gln Gln Gly Pro Gly
305                 310                 315                 320
Gly Gln Gly Pro Tyr Gly Pro Gly Ala Ser Ala Ala Ser Ala Ala Ser
                325                 330                 335
Gly Gly Tyr Gly Pro Gly Ser Gly Gln Gln Pro Gln Val Thr Arg Gly
            340                 345                 350
Asp Val Phe Thr Met Gly Pro Gly Gln Gln Gly Pro Gly Gly Gln Gly
        355                 360                 365
Pro Tyr Gly Pro Gly Ala Ser Ala Ala Ala Ala Ala Ala Gly Gly Tyr
    370                 375                 380
Gly Pro Gly Ser Gly Gln Gln Gly Pro Gly Gln Gln Gly Pro Gly Gln
385                 390                 395                 400
Gln Gly Pro Gly Gln Gln Gly Pro Gly Gly Gln Gly Pro Tyr Gly Pro
                405                 410                 415
Gly Ala Ser Ala Ala Ala Ala Ala Ala Gly Gly Tyr Gly Pro Gly Ser
            420                 425                 430
Gly Gln Gln Gly Pro Gly Gln Gln Gly Pro Gly Gln Gln Gly Pro Gly
        435                 440                 445
Gln Gln Pro Gln Val Thr Arg Gly Asp Val Phe Thr Met Gly Pro Gly
    450                 455                 460
Gln Gln Gly Pro Gly Gln Gln Gly Pro Gly Gln Gln Gly Pro Gly Gln
465                 470                 475                 480
Gln Gly Pro Gly Gln Gln Gly Pro Gly Gly Gln Gly Ala Tyr Gly Pro
                485                 490                 495
Gly Ala Ser Ala Ala Ala Gly Ala Ala Gly Gly Tyr Gly Pro Gly Ser
            500                 505                 510
Gly Gln Gln Gly Pro Gly Gln Gln Gly Pro Gly Gln Gln Gly Pro Gly
        515                 520                 525
Gln Gln Pro Gln Val Thr Arg Gly Asp Val Phe Thr Met Gly Pro Gly

530                 535                 540
Gln Gln Gly Pro Gly Gln Gln Gly Pro Gly Gln Gln Gly Pro Gly Gln
545                 550                 555                 560
Gln Gly Pro Tyr Gly Pro Gly Ala Ser Ala Ala Ala Ala Ala Ala Gly
                565                 570                 575
Gly Tyr Gly Pro Gly Ser Gly Gln Gln Gly Pro Gly Gln Gln Gly Pro
            580                 585                 590
Gly Gln Gln Gly Pro Gly Gly Gln Gly Pro Tyr Gly Pro Gly Ala Ala
        595                 600                 605
Ser Ala Ala Val Ser Val Gly Gly Tyr Gly Pro Gln Ser Ser Ser Val
    610                 615                 620
Pro Val Ala Ser Ala Val Ala Ser Arg Leu Ser Ser Pro Ala Ala Ser
625                 630                 635                 640
Ser Arg Val Ser Ser Ala Val Ser Ser Leu Val Ser Ser Gly Pro Thr
                645                 650                 655
Lys His Ala Ala Leu Ser Asn Thr Ile Ser Ser Val Val Ser Gln Val
            660                 665                 670
Ser Ala Ser Asn Pro Gly Leu Ser Gly Cys Asp Val Leu Val Gln Ala
        675                 680                 685
Leu Leu Glu Val Val Ser Ala Leu Val Ser Ile Leu Gly Ser Ser Ser
    690                 695                 700
Ile Gly Gln Ile Asn Tyr Gly Ala Ser Ala Gln Tyr Thr Gln Met Val
705                 710                 715                 720
Gly Gln Ser Val Ala Gln Ala Leu Ala
                725

<210>  28
<211>  2106
<212>  DNA
<213>  Araneus diadematus

<400>  28
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggtagtggt     300
caggttaccc gtggtgatgt ttttcaaggt cctggtggcc aaggtccgta cggtcctggt     360
agtagcgctg ctgccgcagc agcgggtggt aacggtccgg gtagcggaca acagggagcc     420
ggtcaacaag ggcccgggca acaaggtcca ggtgcctcag cagccgctgc agcggcaggc     480
ggttatggcc caggatcagg tcagcaggtt acacgcggag atgtgtttgg tcccggacag     540
cagggcccag gtggtcaagg accttacgga ccaggtgcca gtgcggctgc tgcagctgcc     600
ggtggatacg gacctggctc tggtcaagtg actcgcggtg acgtttttgg gcctggccag     660
caagggcctg gcggtcaggg tccatacgga ccgggtgctt ccgcagcggc ggctgcagcg     720
ggaggttatg ggccaggtag tgggcagcaa ggacctggac agcaaggtcc cggacaacaa     780
ggccctggcg gacagggccc gtatggccca ggcgcttcag ccgcagctgc agcagccgga     840
ggctacggtc caggttatgg tcagcaagtg acccgtggcg acgtatttgg tccggggcaa     900
caggggcctg gaggccaagg cccttatgga cctggcgcaa gcgcagccag cgcagcaagt     960
ggtggctatg gaccaggcag tggccaacag gtgacacgtg gtgacgtgtt cggaccaggc    1020
caacagggac caggcggtca aggcccctat ggtcccggtg cttctgcggc tgccgctgcc    1080
gccggcgggt atgggcctgg aagcggccaa caaggtcctg ggcagcaggg gccagggcag    1140
cagggtcctg gccaacaagg gccaggggga caaggacctt atggtccagg ggcatctgca    1200
gctgctgctg ctgcgggagg gtacggtccg ggaagtggtc agcaggggcc cggacagcaa    1260
ggaccaggcc agcagggccc tggacagcag gtaacacgtg gggatgtttt cggccctggt    1320
cagcaaggcc cgggacagca gggtccgggt caacagggtc cgggacagca ggggcctgga    1380
caacaaggtc ctggaggcca gggtgcctat ggtccgggag cttcagcagc agcgggagct    1440
gcgggtggtt atggacccgg aagcgggcaa cagggccccg gacaacaggg tcccggccag    1500
caagggccgg gtcagcaggt tacgcgtggg gacgtttttg gtccaggtca gcagggacca    1560
gggcaacaag gcccaggtca gcaaggtcca ggccagcaag gtccttatgg tcctggtgcc    1620
agcgcagctg ctgcggcagc tggcggctac ggccctggtt cgggtcaaca aggaccagga    1680
caacagggcc cagggcaaca ggggccaggc ggacagggtc cgtacggtcc cggtgcagca    1740
agcgcagcag ttagcgttgg cggttatggt ccgcagagca gcagcgttcc ggttgcaagc    1800

gcagttgcaa gccgtctgag cagtccggca gcaagcagtc gtgtgagcag cgcagttagc 1860
agcctggtta gcagcggtcc gaccaaacat gcagcactga gcaataccat tagcagcgtt 1920
gttagccagg ttagcgcaag caatccgggt ctgagcggtt gtgatgttct ggtgcaggca 1980
ctgctggaag ttgttagcgc actggtgagc attctgggta gcagcagcat tggtcagatt 2040
aactatggtg cgagcgcaca gtatacccag atggttggtc agagcgttgc acaggcactg 2100
gcataa 2106

<210> 29
<211> 701
<212> PRT
<213> Araneus diadematus

<400> 29
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Val Thr Arg Gly Asp Val Phe Gln Gly Pro Gly
            100                 105                 110
Gly Gln Gly Pro Tyr Gly Pro Gly Ser Ser Ala Ala Ala Ala Ala Ala
        115                 120                 125
Gly Gly Asn Gly Pro Gly Ser Gly Gln Gln Gly Ala Gly Gln Gln Gly
    130                 135                 140
Pro Gly Gln Gln Gly Pro Gly Ala Ser Ala Ala Ala Ala Ala Ala Gly
145                 150                 155                 160
Gly Tyr Gly Pro Gly Ser Gly Gln Gln Val Thr Arg Gly Asp Val Phe
                165                 170                 175
Gly Pro Gly Gln Gln Gly Pro Gly Gly Gln Gly Pro Tyr Gly Pro Gly
            180                 185                 190
Ala Ser Ala Ala Ala Ala Ala Ala Gly Gly Tyr Gly Pro Gly Ser Gly
        195                 200                 205
Gln Val Thr Arg Gly Asp Val Phe Gly Pro Gly Gln Gln Gly Pro Gly
    210                 215                 220
Gly Gln Gly Pro Tyr Gly Pro Gly Ala Ser Ala Ala Ala Ala Ala Ala
225                 230                 235                 240
Gly Gly Tyr Gly Pro Gly Ser Gly Gln Gln Gly Pro Gly Gln Gln Gly
                245                 250                 255
Pro Gly Gln Gln Gly Pro Gly Gly Gln Gly Pro Tyr Gly Pro Gly Ala
            260                 265                 270
Ser Ala Ala Ala Ala Ala Ala Gly Gly Tyr Gly Pro Gly Tyr Gly Gln
        275                 280                 285
Gln Val Thr Arg Gly Asp Val Phe Gly Pro Gly Gln Gln Gly Pro Gly
    290                 295                 300
Gly Gln Gly Pro Tyr Gly Pro Gly Ala Ser Ala Ala Ser Ala Ala Ser
305                 310                 315                 320
Gly Gly Tyr Gly Pro Gly Ser Gly Gln Gln Val Thr Arg Gly Asp Val
                325                 330                 335
Phe Gly Pro Gly Gln Gln Gly Pro Gly Gly Gln Gly Pro Tyr Gly Pro
            340                 345                 350
Gly Ala Ser Ala Ala Ala Ala Ala Ala Gly Gly Tyr Gly Pro Gly Ser
        355                 360                 365
Gly Gln Gln Gly Pro Gly Gln Gln Gly Pro Gly Gln Gln Gly Pro Gly
    370                 375                 380
Gln Gln Gly Pro Gly Gly Gln Gly Pro Tyr Gly Pro Gly Ala Ser Ala

385                 390                 395                 400
Ala Ala Ala Ala Ala Gly Gly Tyr Gly Pro Gly Ser Gly Gln Gln Gly
                405                 410                 415
Pro Gly Gln Gln Gly Pro Gly Gln Gln Gly Pro Gly Gln Gln Val Thr
            420                 425                 430
Arg Gly Asp Val Phe Gly Pro Gly Gln Gln Gly Pro Gly Gln Gln Gly
        435                 440                 445
Pro Gly Gln Gln Gly Pro Gly Gln Gln Gly Pro Gly Gln Gln Gly Pro
    450                 455                 460
Gly Gly Gln Gly Ala Tyr Gly Pro Gly Ala Ser Ala Ala Ala Gly Ala
465                 470                 475                 480
Ala Gly Gly Tyr Gly Pro Gly Ser Gly Gln Gln Gly Pro Gly Gln Gln
                485                 490                 495
Gly Pro Gly Gln Gln Gly Pro Gly Gln Gln Val Thr Arg Gly Asp Val
            500                 505                 510
Phe Gly Pro Gly Gln Gln Gly Pro Gly Gln Gln Gly Pro Gly Gln Gln
        515                 520                 525
Gly Pro Gly Gln Gln Gly Pro Tyr Gly Pro Gly Ala Ser Ala Ala Ala
    530                 535                 540
Ala Ala Ala Gly Gly Tyr Gly Pro Gly Ser Gly Gln Gln Gly Pro Gly
545                 550                 555                 560
Gln Gln Gly Pro Gly Gln Gln Gly Pro Gly Gly Gln Gly Pro Tyr Gly
                565                 570                 575
Pro Gly Ala Ala Ser Ala Ala Val Ser Val Gly Gly Tyr Gly Pro Gln
            580                 585                 590
Ser Ser Ser Val Pro Val Ala Ser Ala Val Ala Ser Arg Leu Ser Ser
        595                 600                 605
Pro Ala Ala Ser Ser Arg Val Ser Ser Ala Val Ser Ser Leu Val Ser
    610                 615                 620
Ser Gly Pro Thr Lys His Ala Ala Leu Ser Asn Thr Ile Ser Ser Val
625                 630                 635                 640
Val Ser Gln Val Ser Ala Ser Asn Pro Gly Leu Ser Gly Cys Asp Val
                645                 650                 655
Leu Val Gln Ala Leu Leu Glu Val Val Ser Ala Leu Val Ser Ile Leu
            660                 665                 670
Gly Ser Ser Ser Ile Gly Gln Ile Asn Tyr Gly Ala Ser Ala Gln Tyr
        675                 680                 685
Thr Gln Met Val Gly Gln Ser Val Ala Gln Ala Leu Ala
    690                 695                 700

<210>  30
<211>  6
<212>  PRT
<213>  Homo sapiens

<400>  30
Gly Arg Gly Asp Asn Pro
1               5

<210>  31
<211>  13
<212>  PRT
<213>  Homo sapiens

<400>  31
Gly Ala Ala Gly Arg Gly Asp Ser Pro Ala Ala Gly Tyr
1               5                   10

<210>  32
<211>  25

<212>  PRT
<213>  Homo sapiens

<400>  32
Gly Pro Gly Gln Gln Gly Pro Gly Gln Gln Gly Pro Gly Gln Gln Val
1               5                   10                  15
Thr Gly Arg Gly Asp Ser Pro Ala Ser
            20                  25


<210>  33
<211>  20
<212>  PRT
<213>  Homo sapiens

<400>  33
Val Thr Gly Arg Gly Asp Ser Pro Ala Ser Val Thr Gly Arg Gly Asp
1               5                   10                  15
Ser Pro Ala Ser
            20


<210>  34
<211>  13
<212>  PRT
<213>  Homo sapiens

<400>  34
Gly Lys Lys Gln Arg Phe Arg His Arg Asn Arg Lys Gly
1               5                   10


<210>  35
<211>  2109
<212>  DNA
<213>  Araneus diadematus

<400>  35
atgcatcatc accaccatca ccatcatcat cacagcagcg gtagcagcct ggaagttctg      60
tttcagggtc cggcacgtgc aggtagcggt cagcagggtc cgggtcagca aggccctggt     120
caacagggac ctggccagca gggaccgtat ggtccgggtg caagtgcagc agcagccgca     180
gccggtggtt atggtcctgg ttcaggccag caaggtccga gccaacaggg tccaggacaa     240
caaggaccgg gtggtcaggg tccttatggc cctggtgcat cagctgcggc agcggcagca     300
ggcggatatg gaccgggtag tggccagggt cgtggtgata atccgcaggg tcctggcgga     360
cagggtccgt acggtcctgg tagtagcgct gctgccgcag cagcgggtgg taacggtccg     420
ggaagtggac agcagggtgc tgggcaacag ggcccaggtc agcaaggtcc aggtgcatca     480
gccgctgctg ctgcagccgg tggctacgga ccaggcagtg gtcaacaagg ccgtggcgac     540
aatccgggac cggggcaaca aggtcctggt ggccaaggcc cttacggtcc aggcgctagt     600
gcagccgcgg ctgcagctgg aggatatggt ccaggctcag gtcagggacg cggagataat     660
ccgggtcctg ggcagcaagg accaggcggt caaggaccat acggacctgg tgcctccgca     720
gcggctgctg cggctggcgg ttacggccct ggatctggcc aacaagggcc tggacagcaa     780
ggtcccggac agcagggacc cggtggccaa ggtccgtatg gaccaggtgc cagcgctgcc     840
gcggccgctg cgggagggta cggaccgggt tatggacaac aggggagagg cgataaccct     900
ggaccagggc agcaggggcc tggagggcag ggcccctatg gacccggagc ctcagctgca     960
agcgcagcaa gcggtgggta tggcccagga agtggccaac agggaagagg tgacaatcct    1020
ggtccgggac aacagggccc tggaggtcaa gggccttatg gtcccggtgc ttcagcagct    1080
gcagcagctg ccggaggata cgggcctggg agcggtcaac aagggccagg gcaacagggt    1140
cctggtcaac agggtccggg ccaacaaggt ccaggcggac agggacctta tggcccaggc    1200
gcttctgcag cagcggcagc tgcaggcggt tacggtcccg gtagcggaca gcaaggccca    1260
ggccaacaag gccccggtca gcagggccca ggacaacagg gtcgcggtga taaccctggt    1320
ccaggtcaac aaggccctgg ccagcaaggg ccgggtcaac aaggaccggg acaacaagga    1380
ccaggtcagc agggaccggg tggacagggt gcatacggtc cgggtgcctc tgctgcagct    1440
ggtgcagcgg gtggatatgg ccctgggtct ggacagcagg ggccaggcca gcagggccct    1500

ggccaacaag gacctgggca acagggacgt ggcgataacc caggcccggg ccaacaggga 1560
cccggtcaac aaggtcccgg gcagcaagga cctggtcagc aaggtccgta tggccctggc 1620
gcaagcgcag ccgctgcagc cgcaggaggc tatggacctg gatccggtca gcaaggaccg 1680
gggcagcagg gccccggaca gcaagggcct ggaggacaag gtccttacgg accgggagca 1740
gccagcgcag cagttagcgt tggaggctac ggtccgcaga gcagcagcgt tccggttgcg 1800
agcgcagttg caagccgtct gagcagtccg gcagcaagca gtcgtgtgag cagcgcagtt 1860
agcagcctgg ttagcagcgg tccgaccaaa catgcagcac tgagcaatac cattagcagc 1920
gttgttagcc aggttagcgc aagcaatcca ggtctgagcg gttgtgatgt tctggtgcag 1980
gcactgctgg aagttgttag cgcactggtg agcattctgg gtagcagcag cattggtcag 2040
attaactatg gtgccagtgc acagtatacc cagatggttg gtcagagcgt tgcacaggca 2100
ctggcataa 2109

<210> 36
<211> 702
<212> PRT
<213> Araneus diadematus

<400> 36
   Met His His His His His His His His His His Ser Ser Gly Ser Ser
   1 5 10 15
   Leu Glu Val Leu Phe Gln Gly Pro Ala Arg Ala Gly Ser Gly Gln Gln
   20 25 30
   Gly Pro Gly Gln Gln Gly Pro Gly Gln Gln Gly Pro Gly Gln Gln Gly
   35 40 45
   Pro Tyr Gly Pro Gly Ala Ser Ala Ala Ala Ala Ala Ala Gly Gly Tyr
   50 55 60
   Gly Pro Gly Ser Gly Gln Gln Gly Pro Ser Gln Gln Gly Pro Gly Gln
   65 70 75 80
   Gln Gly Pro Gly Gly Gln Gly Pro Tyr Gly Pro Gly Ala Ser Ala Ala
   85 90 95
   Ala Ala Ala Ala Gly Gly Tyr Gly Pro Gly Ser Gly Gln Gly Arg Gly
   100 105 110
   Asp Asn Pro Gln Gly Pro Gly Gly Gln Gly Pro Tyr Gly Pro Gly Ser
   115 120 125
   Ser Ala Ala Ala Ala Ala Ala Gly Gly Asn Gly Pro Gly Ser Gly Gln
   130 135 140
   Gln Gly Ala Gly Gln Gln Gly Pro Gly Gln Gln Gly Pro Gly Ala Ser
   145 150 155 160
   Ala Ala Ala Ala Ala Ala Gly Gly Tyr Gly Pro Gly Ser Gly Gln Gln
   165 170 175
   Gly Arg Gly Asp Asn Pro Gly Pro Gly Gln Gln Gly Pro Gly Gly Gln
   180 185 190
   Gly Pro Tyr Gly Pro Gly Ala Ser Ala Ala Ala Ala Ala Ala Gly Gly
   195 200 205
   Tyr Gly Pro Gly Ser Gly Gln Gly Arg Gly Asp Asn Pro Gly Pro Gly
   210 215 220
   Gln Gln Gly Pro Gly Gly Gln Gly Pro Tyr Gly Pro Gly Ala Ser Ala
   225 230 235 240
   Ala Ala Ala Ala Ala Gly Gly Tyr Gly Pro Gly Ser Gly Gln Gln Gly
   245 250 255
   Pro Gly Gln Gln Gly Pro Gly Gln Gln Gly Pro Gly Gly Gln Gly Pro
   260 265 270
   Tyr Gly Pro Gly Ala Ser Ala Ala Ala Ala Ala Ala Gly Gly Tyr Gly
   275 280 285
   Pro Gly Tyr Gly Gln Gln Gly Arg Gly Asp Asn Pro Gly Pro Gly Gln
   290 295 300
   Gln Gly Pro Gly Gly Gln Gly Pro Tyr Gly Pro Gly Ala Ser Ala Ala
   305 310 315 320
   Ser Ala Ala Ser Gly Gly Tyr Gly Pro Gly Ser Gly Gln Gln Gly Arg
   325 330 335
   Gly Asp Asn Pro Gly Pro Gly Gln Gln Gly Pro Gly Gly Gln Gly Pro
   340 345 350
   Tyr Gly Pro Gly Ala Ser Ala Ala Ala Ala Ala Ala Gly Gly Tyr Gly
           355                 360                 365
   Pro Gly Ser Gly Gln Gln Gly Pro Gly Gln Gln Gly Pro Gly Gln Gln
       370                 375                 380
   Gly Pro Gly Gln Gln Gly Pro Gly Gly Gln Gly Pro Tyr Gly Pro Gly
   385                 390                 395                 400
   Ala Ser Ala Ala Ala Ala Ala Ala Gly Gly Tyr Gly Pro Gly Ser Gly
                   405                 410                 415
   Gln Gln Gly Pro Gly Gln Gln Gly Pro Gly Gln Gln Gly Pro Gly Gln
               420                 425                 430
   Gln Gly Arg Gly Asp Asn Pro Gly Pro Gly Gln Gln Gly Pro Gly Gln
           435                 440                 445
   Gln Gly Pro Gly Gln Gln Gly Pro Gly Gln Gln Gly Pro Gly Gln Gln
       450                 455                 460
   Gly Pro Gly Gly Gln Gly Ala Tyr Gly Pro Gly Ala Ser Ala Ala Ala
   465                 470                 475                 480
   Gly Ala Ala Gly Gly Tyr Gly Pro Gly Ser Gly Gln Gln Gly Pro Gly
                   485                 490                 495
   Gln Gln Gly Pro Gly Gln Gln Gly Pro Gly Gln Gln Gly Arg Gly Asp
               500                 505                 510
   Asn Pro Gly Pro Gly Gln Gln Gly Pro Gly Gln Gln Gly Pro Gly Gln
           515                 520                 525
   Gln Gly Pro Gly Gln Gln Gly Pro Tyr Gly Pro Gly Ala Ser Ala Ala
       530                 535                 540
   Ala Ala Ala Ala Gly Gly Tyr Gly Pro Gly Ser Gly Gln Gln Gly Pro
   545                 550                 555                 560
   Gly Gln Gln Gly Pro Gly Gln Gln Gly Pro Gly Gly Gln Gly Pro Tyr
                   565                 570                 575
   Gly Pro Gly Ala Ala Ser Ala Ala Val Ser Val Gly Gly Tyr Gly Pro
               580                 585                 590
   Gln Ser Ser Ser Val Pro Val Ala Ser Ala Val Ala Ser Arg Leu Ser
           595                 600                 605
   Ser Pro Ala Ala Ser Ser Arg Val Ser Ser Ala Val Ser Ser Leu Val
       610                 615                 620
   Ser Ser Gly Pro Thr Lys His Ala Ala Leu Ser Asn Thr Ile Ser Ser
   625                 630                 635                 640
   Val Val Ser Gln Val Ser Ala Ser Asn Pro Gly Leu Ser Gly Cys Asp
                   645                 650                 655
   Val Leu Val Gln Ala Leu Leu Glu Val Val Ser Ala Leu Val Ser Ile
               660                 665                 670
   Leu Gly Ser Ser Ser Ile Gly Gln Ile Asn Tyr Gly Ala Ser Ala Gln
           675                 680                 685
   Tyr Thr Gln Met Val Gly Gln Ser Val Ala Gln Ala Leu Ala
       690                 695                 700

<210>  37
<211>  1749
<212>  DNA
<213>  Araneus diadematus

<400>  37
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggttcaggt     300
cagggtgcag caggtcgtgg tgatagtccg gcagccggtt atggtagctc tgccgctgct     360
gcagcagcgg gtggtaacgg tccgggtagt ggacagcaag gtgcaggtca acaaggcccc     420
gggcaacaag gtcctggcgc atctgcagct gccgccgctg cgggaggata tggtccaggc     480
tctgggcagc agggcgctgc cggacgtggt gattcaccag ccgcaggtta tggtgccagt     540
gccgctgccg cggccgctgg cggttatggc ccaggtagtg gtcaaggtgc tgctggccgt     600
ggcgatagcc ctgcggctgg ttatggcgca agcgcagctg cagcggcggc tggaggctac     660

ggtcctggaa gcggacaaca ggggcctgga cagcaaggcc caggacagca gggtccaggt 720
ggccaaggtc cgtatggccc aggcgcttca gccgcagctg ctgctgccgg tggctatgga 780
ccaggttatg ggcaacaggg tgccgctggt cgcggagatt ctcctgcagc aggctatggc 840
gcatcagcgg catcagcagc aagcggaggt tacggaccgg gatctggcca acaaggcgca 900
gcgggacgcg gtgatagccc agctgcggga tatggtgcga gcgcagccgc tgcagccgcc 960
ggtggatacg gcccaggaag tgggcagcaa ggacccggtc agcaaggacc gggacaacaa 1020
gggccaggcc aacagggacc aggcggacag ggtccgtacg gaccaggtgc tagcgctgcg 1080
gccgcagcgg ctggggggta tggacctggc agtggccaac agggtgcggc aggacgaggc 1140
gattcaccgg ctgctggcta cggtgcgtct gctgccgctg gggctgcagg cggttacggt 1200
ccagggtcag gtcaacaggg ggcagccggt agaggtgatt cccctgcagc gggttacggt 1260
gcctcagccg ccgcggctgc cgcagggggg tacggccctg gatctggtca gcaaggtcct 1320
ggccagcaag gtccagggca acaaggccct ggtggccaag gaccttacgg tcctggtgca 1380
gcaagcgcag cagttagcgt tggtggctat ggtccgcaga gcagcagcgt tccggttgcc 1440
agcgcagttg caagccgtct gagttcaccg gcagcaagca gtcgtgttag cagtgcagtt 1500
agcagcctgg ttagcagcgg tccgaccaaa catgcagcac tgagcaatac cattagcagc 1560
gttgttagcc aggttagcgc aagcaatccg ggtctgagcg gttgtgatgt tctggtgcag 1620
gcactgctgg aagttgttag cgcactggtg agcattctgg gtagcagcag cattggtcag 1680
attaactatg gtgcgtcagc acagtatacc cagatggttg gtcagagcgt tgcacaggca 1740
ctggcataa 1749

<210> 38
<211> 582
<212> PRT
<213> Araneus diadematus

<400> 38
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Gly Ala Ala Gly Arg Gly Asp Ser Pro Ala Ala
            100                 105                 110
Gly Tyr Gly Ser Ser Ala Ala Ala Ala Ala Ala Gly Gly Asn Gly Pro
        115                 120                 125
Gly Ser Gly Gln Gln Gly Ala Gly Gln Gln Gly Pro Gly Gln Gln Gly
    130                 135                 140
Pro Gly Ala Ser Ala Ala Ala Ala Ala Ala Gly Gly Tyr Gly Pro Gly
145                 150                 155                 160
Ser Gly Gln Gln Gly Ala Ala Gly Arg Gly Asp Ser Pro Ala Ala Gly
                165                 170                 175
Tyr Gly Ala Ser Ala Ala Ala Ala Ala Ala Gly Gly Tyr Gly Pro Gly
            180                 185                 190
Ser Gly Gln Gly Ala Ala Gly Arg Gly Asp Ser Pro Ala Ala Gly Tyr
        195                 200                 205
Gly Ala Ser Ala Ala Ala Ala Ala Ala Gly Gly Tyr Gly Pro Gly Ser
    210                 215                 220
Gly Gln Gln Gly Pro Gly Gln Gln Gly Pro Gly Gln Gln Gly Pro Gly
225                 230                 235                 240
Gly Gln Gly Pro Tyr Gly Pro Gly Ala Ser Ala Ala Ala Ala Ala Ala
                245                 250                 255
Gly Gly Tyr Gly Pro Gly Tyr Gly Gln Gln Gly Ala Ala Gly Arg Gly
            260                 265                 270
Asp Ser Pro Ala Ala Gly Tyr Gly Ala Ser Ala Ala Ser Ala Ala Ser
        275                 280                 285

Gly Gly Tyr Gly Pro Gly Ser Gly Gln Gln Gly Ala Ala Gly Arg Gly
    290                 295                 300
Asp Ser Pro Ala Ala Gly Tyr Gly Ala Ser Ala Ala Ala Ala Ala Ala
305                 310                 315                 320
Gly Gly Tyr Gly Pro Gly Ser Gly Gln Gln Gly Pro Gly Gln Gln Gly
                325                 330                 335
Pro Gly Gln Gln Gly Pro Gly Gln Gln Gly Pro Gly Gly Gln Gly Pro
            340                 345                 350
Tyr Gly Pro Gly Ala Ser Ala Ala Ala Ala Ala Ala Gly Gly Tyr Gly
        355                 360                 365
Pro Gly Ser Gly Gln Gln Gly Ala Ala Gly Arg Gly Asp Ser Pro Ala
    370                 375                 380
Ala Gly Tyr Gly Ala Ser Ala Ala Ala Gly Ala Ala Gly Gly Tyr Gly
385                 390                 395                 400
Pro Gly Ser Gly Gln Gln Gly Ala Ala Gly Arg Gly Asp Ser Pro Ala
                405                 410                 415
Ala Gly Tyr Gly Ala Ser Ala Ala Ala Ala Ala Ala Gly Gly Tyr Gly
            420                 425                 430
Pro Gly Ser Gly Gln Gln Gly Pro Gly Gln Gln Gly Pro Gly Gln Gln
        435                 440                 445
Gly Pro Gly Gly Gln Gly Pro Tyr Gly Pro Gly Ala Ala Ser Ala Ala
    450                 455                 460
Val Ser Val Gly Gly Tyr Gly Pro Gln Ser Ser Ser Val Pro Val Ala
465                 470                 475                 480
Ser Ala Val Ala Ser Arg Leu Ser Ser Pro Ala Ala Ser Ser Arg Val
                485                 490                 495
Ser Ser Ala Val Ser Ser Leu Val Ser Ser Gly Pro Thr Lys His Ala
            500                 505                 510
Ala Leu Ser Asn Thr Ile Ser Ser Val Val Ser Gln Val Ser Ala Ser
        515                 520                 525
Asn Pro Gly Leu Ser Gly Cys Asp Val Leu Val Gln Ala Leu Leu Glu
    530                 535                 540
Val Val Ser Ala Leu Val Ser Ile Leu Gly Ser Ser Ser Ile Gly Gln
545                 550                 555                 560
Ile Asn Tyr Gly Ala Ser Ala Gln Tyr Thr Gln Met Val Gly Gln Ser
                565                 570                 575
Val Ala Gln Ala Leu Ala
            580

<210>  39
<211>  2394
<212>  DNA
<213>  Araneus diadematus

<400>  39
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggtagtggt     300
caaggaccag ggcagcaggg ccccgggcaa caagggcccg gacagcaggt taccggtcgt     360
ggtgatagtc cggcaagcca gggtcctggt ggccaaggtc cgtacggtcc tggtagtagc     420
gctgctgccg cagcagcggg tggtaacggt ccgggtagcg gacaacaggg agccggtcaa     480
caaggtcccg gccaacaagg cccaggtgca tctgcagccg ctgcagcggc aggcggttat     540
ggcccaggat caggtcagca ggggccagga cagcagggtc ccggccaaca gggaccgggc     600
caacaggtga caggtcgcgg tgattcaccg gcaagcggtc ctgggcagca gggtccaggt     660
ggtcagggac catacggacc gggtgcctca gccgcagctg cagctgcggg tggctatggt     720
ccaggcagcg gtcaaggtcc tggacagcaa gggccaggcc aacaagggcc tggtcagcaa     780
gtgacaggca gaggtgatag ccctgcatca ggaccgggac aacaaggtcc tggcggtcaa     840
ggaccgtatg gcccaggcgc ttcagccgct gcggctgctg ctggcggtta cggacctggc     900
agcgggcaac agggtccagg tcagcaggga ccaggtcaac aaggaccagg cggacaaggt     960
ccatacggac caggcgcaag cgcagccgct gccgcagctg gcggatacgg tccaggttat    1020

gggcagcaag gacccggtca gcaaggtcca gggcaacagg gtccggggca gcaagttaca 1080
ggtcgtggcg attctcctgc ctcaggccca ggacaacagg ggccaggggg acagggccct 1140
tatggtcccg gagccagcgc agcatcagca gcaagtggtg ggtatgggcc aggtagtggc 1200
caacaaggtc cgggacagca gggacctgga cagcaaggcc cgggacagca agtgactggc 1260
cgtggggact caccggcttc agggccgggt caacagggcc caggaggcca aggaccttac 1320
gggccaggtg ctagtgctgc ggcggcagct gccggagggt atggacctgg atctggacaa 1380
cagggccctg gacaacaggg tccgggccaa caaggtcctg gccagcaagg tccgggtggc 1440
caaggccctt atggaccggg tgcgtctgcg gctgcagctg cagcgggtgg atatggccca 1500
ggaagtgggc agcaaggccc aggccagcag gggcctggac aacaaggtcc agggcagcag 1560
gtaacaggac gtggtgacag ccctgcctct gggcctggcc aacagggtcc cggtcaacag 1620
ggtcccgggc agcaggggcc tggtcagcag ggtcctggac agcagggccc aggtggccaa 1680
ggtgcctatg gtccaggtgc atcagcagca gcgggtgcag ccggtggcta tggaccggga 1740
agcggacagc aaggtcctgg tcaacaaggt cccggtcagc aaggaccggg acagcaggtt 1800
acgggacgcg gagattcacc agcatctgga ccgggccagc aaggacccgg acaacagggc 1860
ccaggtcagc aaggtccagg ccagcaagga ccttatggtc ctggcgcatc tgccgcagcc 1920
gcagcagccg gaggttacgg acccggaagt ggtcaacaag ggccaggaca gcaaggcccc 1980
ggtcagcagg gccctggtgg acagggtccg tatgggccag gggcagcaag cgcagcagtt 2040
agcgttggcg gttatggtcc gcagagcagc agcgttccgg ttgcaagcgc agttgcaagc 2100
cgtctgagca gtccggcagc atcaagccgt gttagcagtg cagttagcag cctggttagc 2160
tcaggtccga ccaaacatgc agcactgagc aataccatta gcagcgttgt gagccaggtt 2220
agcgcaagca atccgggtct gagcggttgt gatgttctgg tgcaggcact gctggaagtt 2280
gttagcgcac tggtgagcat tctgggtagc agcagcattg gtcagattaa ctatggtgcc 2340
agcgcacagt atacccagat ggttggtcag agcgttgcac aggcactggc ataa 2394

<210> 40
<211> 797
<212> PRT
<213> Araneus diadematus

<400> 40
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Gly Pro Gly Gln Gln Gly Pro Gly Gln Gln Gly
            100                 105                 110
Pro Gly Gln Gln Val Thr Gly Arg Gly Asp Ser Pro Ala Ser Gln Gly
        115                 120                 125
Pro Gly Gly Gln Gly Pro Tyr Gly Pro Gly Ser Ser Ala Ala Ala Ala
    130                 135                 140
Ala Ala Gly Gly Asn Gly Pro Gly Ser Gly Gln Gln Gly Ala Gly Gln
145                 150                 155                 160
Gln Gly Pro Gly Gln Gln Gly Pro Gly Ala Ser Ala Ala Ala Ala Ala
                165                 170                 175
Ala Gly Gly Tyr Gly Pro Gly Ser Gly Gln Gln Gly Pro Gly Gln Gln
            180                 185                 190
Gly Pro Gly Gln Gln Gly Pro Gly Gln Gln Val Thr Gly Arg Gly Asp
        195                 200                 205
Ser Pro Ala Ser Gly Pro Gly Gln Gln Gly Pro Gly Gly Gln Gly Pro
    210                 215                 220
Tyr Gly Pro Gly Ala Ser Ala Ala Ala Ala Ala Ala Gly Gly Tyr Gly
225                 230                 235                 240
Pro Gly Ser Gly Gln Gly Pro Gly Gln Gln Gly Pro Gly Gln Gln Gly
                245                 250                 255

Pro Gly Gln Gln Val Thr Gly Arg Gly Asp Ser Pro Ala Ser Gly Pro
            260                 265                 270
Gly Gln Gln Gly Pro Gly Gly Gln Gly Pro Tyr Gly Pro Gly Ala Ser
        275                 280                 285
Ala Ala Ala Ala Ala Ala Gly Gly Tyr Gly Pro Gly Ser Gly Gln Gln
    290                 295                 300
Gly Pro Gly Gln Gln Gly Pro Gly Gln Gln Gly Pro Gly Gly Gln Gly
305                 310                 315                 320
Pro Tyr Gly Pro Gly Ala Ser Ala Ala Ala Ala Ala Ala Gly Gly Tyr
                325                 330                 335
Gly Pro Gly Tyr Gly Gln Gln Gly Pro Gly Gln Gln Gly Pro Gly Gln
            340                 345                 350
Gln Gly Pro Gly Gln Gln Val Thr Gly Arg Gly Asp Ser Pro Ala Ser
        355                 360                 365
Gly Pro Gly Gln Gln Gly Pro Gly Gly Gln Gly Pro Tyr Gly Pro Gly
    370                 375                 380
Ala Ser Ala Ala Ser Ala Ala Ser Gly Gly Tyr Gly Pro Gly Ser Gly
385                 390                 395                 400
Gln Gln Gly Pro Gly Gln Gln Gly Pro Gly Gln Gln Gly Pro Gly Gln
                405                 410                 415
Gln Val Thr Gly Arg Gly Asp Ser Pro Ala Ser Gly Pro Gly Gln Gln
            420                 425                 430
Gly Pro Gly Gly Gln Gly Pro Tyr Gly Pro Gly Ala Ser Ala Ala Ala
        435                 440                 445
Ala Ala Ala Gly Gly Tyr Gly Pro Gly Ser Gly Gln Gln Gly Pro Gly
    450                 455                 460
Gln Gln Gly Pro Gly Gln Gln Gly Pro Gly Gln Gln Gly Pro Gly Gly
465                 470                 475                 480
Gln Gly Pro Tyr Gly Pro Gly Ala Ser Ala Ala Ala Ala Ala Ala Gly
                485                 490                 495
Gly Tyr Gly Pro Gly Ser Gly Gln Gln Gly Pro Gly Gln Gln Gly Pro
            500                 505                 510
Gly Gln Gln Gly Pro Gly Gln Gln Val Thr Gly Arg Gly Asp Ser Pro
        515                 520                 525
Ala Ser Gly Pro Gly Gln Gln Gly Pro Gly Gln Gln Gly Pro Gly Gln
    530                 535                 540
Gln Gly Pro Gly Gln Gln Gly Pro Gly Gln Gln Gly Pro Gly Gly Gln
545                 550                 555                 560
Gly Ala Tyr Gly Pro Gly Ala Ser Ala Ala Ala Gly Ala Ala Gly Gly
                565                 570                 575
Tyr Gly Pro Gly Ser Gly Gln Gln Gly Pro Gly Gln Gln Gly Pro Gly
            580                 585                 590
Gln Gln Gly Pro Gly Gln Gln Val Thr Gly Arg Gly Asp Ser Pro Ala
        595                 600                 605
Ser Gly Pro Gly Gln Gln Gly Pro Gly Gln Gln Gly Pro Gly Gln Gln
    610                 615                 620
Gly Pro Gly Gln Gln Gly Pro Tyr Gly Pro Gly Ala Ser Ala Ala Ala
625                 630                 635                 640
Ala Ala Ala Gly Gly Tyr Gly Pro Gly Ser Gly Gln Gln Gly Pro Gly
                645                 650                 655
Gln Gln Gly Pro Gly Gln Gln Gly Pro Gly Gly Gln Gly Pro Tyr Gly
            660                 665                 670
Pro Gly Ala Ala Ser Ala Ala Val Ser Val Gly Gly Tyr Gly Pro Gln
        675                 680                 685
Ser Ser Ser Val Pro Val Ala Ser Ala Val Ala Ser Arg Leu Ser Ser
    690                 695                 700
Pro Ala Ala Ser Ser Arg Val Ser Ser Ala Val Ser Ser Leu Val Ser
705                 710                 715                 720
Ser Gly Pro Thr Lys His Ala Ala Leu Ser Asn Thr Ile Ser Ser Val
                725                 730                 735
Val Ser Gln Val Ser Ala Ser Asn Pro Gly Leu Ser Gly Cys Asp Val
            740                 745                 750
Leu Val Gln Ala Leu Leu Glu Val Val Ser Ala Leu Val Ser Ile Leu

        755                 760                 765
Gly Ser Ser Ser Ile Gly Gln Ile Asn Tyr Gly Ala Ser Ala Gln Tyr
    770                 775                 780
Thr Gln Met Val Gly Gln Ser Val Ala Gln Ala Leu Ala
785                 790                 795

<210> 41
<211> 2196
<212> DNA
<213> Araneus diadematus

<400> 41
atgcatcatc accaccatca ccatcatcat cacagcagcg gtagcagcct ggaagttctg 60
tttcagggtc cggcacgtgc aggtagcggt cagcagggtc cgggtcagca aggccctggt 120
caacagggac ctggccagca gggaccgtat ggtccgggtg caagtgcagc agcagccgca 180
gccggtggtt atggtcctgg ttcaggccag caaggtcgtg gtgatagtcc gggtccgagc 240
caacagggtc caggacaaca aggaccgggt ggtcagccgt atggccctgg tgcttctgcc 300
gctgctgcag cagcgggtgg ctatggaccg ggtagtggtc agggtcgcgg tgattcaccg 360
cagggtcctg gtggccaggg tccttatggt ccaggtagta gcgcagctgc agcggcagca 420
ggcggtaacg gtccgggaag tggccaacaa ggccgtggcg attcaccggg tgcaggacag 480
cagggcccag ggcagcaggg gcccggtgca tctgctgccg ccgctgcggc tggcggttat 540
ggcccaggat ctggacagca aggacgcgga gacagcccag gaccggggca acaaggtcct 600
ggcggtcaag gtccgtacgg acctggtgca agcgctgcgg ccgcagctgc cggtggatac 660
ggaccaggat caggtcaggg acgtggcgat agcccaggcc ccggacagca aggtccaggt 720
ggccaaggac catacggccc aggtgccagt gccgcggctg ctgctgcggg aggttacggt 780
ccaggcagcg gacaacaggg gagaggcgat tctccaggcc caggccaaca agggcctggt 840
cagcaaggac caggcggaca gggtccctac gggccaggcg catctgccgc agcggctgca 900
gccggagggt acggaccggg ttatgggcaa caggggcgtg gtgactcacc agggcccggt 960
caacaaggtc cgggaggcca aggcccttat gggcctggtg catcagcagc aagcgcagcc 1020
agcggaggtt atggacccgg atcgggccaa cagggaagag gtgatagccc tggtccggga 1080
cagcagggac ccggaggtca gggaccttat ggccctggag ccagcgctgc cgcagcagca 1140
gctggaggat atggtccggg atccggacaa caaggtcggg gtgactcacc gggtcctggg 1200
cagcagggtc ctggccaaca aggtcccgga cagcaggggc ctggtggaca aggcccttac 1260
ggaccgggag cctctgctgc tgcagccgct gcaggcggat atggccctgg cagcggccag 1320
caaggaccgg gacaacaggg gccaggtcaa cagggtcccg ggcaacaggg taggggtgac 1380
agtccaggtc cagggcagca agggccagga cagcaaggtc ctggccagca gggcccagga 1440
caacagggcc ctggacaaca aggcccaggc ggtcagggag cctatggtcc aggggcttct 1500
gcagcagccg gtgccgcagg cggttacggg cctggttctg gacaacaggg accgggacag 1560
caagggccgg gccaacaagg acccggccaa caaggtagag gggattctcc tggtccaggg 1620
caacagggac ccggtcagca gggccctgga cagcagggtc cggggcaaca gggaccgtat 1680
ggcccaggcg catcagcagc tgcggcagcc gcaggaggat acggtcctgg ctcggggcag 1740
cagggacggg gagatagtcc tggcccaggt cagcagggac caggtcaaca agggccaggt 1800
gggcagggtc cgtatggccc tggggcagca tctgcagcag ttagcgttgg tgggtatggt 1860
ccgcagagca gcagcgttcc ggttgccagc gcagttgcaa gccgtctgag cagtccggca 1920
gcaagcagtc gtgttagcag tgcagttagc agcctggtta gcagcggtcc gaccaaacat 1980
gcagcactga gcaataccat tagcagcgtt gttagccagg ttagcgcaag caatccgggt 2040
ctgagcggtt gtgatgttct ggtgcaggca ctgctggaag ttgttagcgc actggtgagc 2100
attctgggta gcagcagcat tggtcagatt aactatggtg cgagcgcaca gtatacccag 2160
atggttggtc agagcgttgc acaggcactg gcataa 2196

<210> 42
<211> 731
<212> PRT
<213> Araneus diadematus

<400> 42
Met His His His His His His His His His His Ser Ser Gly Ser Ser
1               5                   10                  15
Leu Glu Val Leu Phe Gln Gly Pro Ala Arg Ala Gly Ser Gly Gln Gln
            20                  25                  30
Gly Pro Gly Gln Gln Gly Pro Gly Gln Gln Gly Pro Gly Gln Gln Gly

35                  40                  45
Pro Tyr Gly Pro Gly Ala Ser Ala Ala Ala Ala Ala Ala Gly Gly Tyr
    50                  55                  60
Gly Pro Gly Ser Gly Gln Gln Gly Arg Gly Asp Ser Pro Gly Pro Ser
65                  70                  75                  80
Gln Gln Gly Pro Gly Gln Gln Gly Pro Gly Gly Gln Pro Tyr Gly Pro
                85                  90                  95
Gly Ala Ser Ala Ala Ala Ala Ala Ala Gly Gly Tyr Gly Pro Gly Ser
            100                 105                 110
Gly Gln Gly Arg Gly Asp Ser Pro Gln Gly Pro Gly Gly Gln Gly Pro
        115                 120                 125
Tyr Gly Pro Gly Ser Ser Ala Ala Ala Ala Ala Ala Gly Gly Asn Gly
    130                 135                 140
Pro Gly Ser Gly Gln Gln Gly Arg Gly Asp Ser Pro Gly Ala Gly Gln
145                 150                 155                 160
Gln Gly Pro Gly Gln Gln Gly Pro Gly Ala Ser Ala Ala Ala Ala Ala
                165                 170                 175
Ala Gly Gly Tyr Gly Pro Gly Ser Gly Gln Gln Gly Arg Gly Asp Ser
            180                 185                 190
Pro Gly Pro Gly Gln Gln Gly Pro Gly Gly Gln Gly Pro Tyr Gly Pro
        195                 200                 205
Gly Ala Ser Ala Ala Ala Ala Ala Ala Gly Gly Tyr Gly Pro Gly Ser
    210                 215                 220
Gly Gln Gly Arg Gly Asp Ser Pro Gly Pro Gly Gln Gln Gly Pro Gly
225                 230                 235                 240
Gly Gln Gly Pro Tyr Gly Pro Gly Ala Ser Ala Ala Ala Ala Ala Ala
                245                 250                 255
Gly Gly Tyr Gly Pro Gly Ser Gly Gln Gln Gly Arg Gly Asp Ser Pro
            260                 265                 270
Gly Pro Gly Gln Gln Gly Pro Gly Gln Gln Gly Pro Gly Gly Gln Gly
        275                 280                 285
Pro Tyr Gly Pro Gly Ala Ser Ala Ala Ala Ala Ala Ala Gly Gly Tyr
    290                 295                 300
Gly Pro Gly Tyr Gly Gln Gln Gly Arg Gly Asp Ser Pro Gly Pro Gly
305                 310                 315                 320
Gln Gln Gly Pro Gly Gly Gln Gly Pro Tyr Gly Pro Gly Ala Ser Ala
                325                 330                 335
Ala Ser Ala Ala Ser Gly Gly Tyr Gly Pro Gly Ser Gly Gln Gln Gly
            340                 345                 350
Arg Gly Asp Ser Pro Gly Pro Gly Gln Gln Gly Pro Gly Gly Gln Gly
        355                 360                 365
Pro Tyr Gly Pro Gly Ala Ser Ala Ala Ala Ala Ala Ala Gly Gly Tyr
    370                 375                 380
Gly Pro Gly Ser Gly Gln Gln Gly Arg Gly Asp Ser Pro Gly Pro Gly
385                 390                 395                 400
Gln Gln Gly Pro Gly Gln Gln Gly Pro Gly Gln Gln Gly Pro Gly Gly
                405                 410                 415
Gln Gly Pro Tyr Gly Pro Gly Ala Ser Ala Ala Ala Ala Ala Ala Gly
            420                 425                 430
Gly Tyr Gly Pro Gly Ser Gly Gln Gln Gly Pro Gly Gln Gln Gly Pro
        435                 440                 445
Gly Gln Gln Gly Pro Gly Gln Gln Gly Arg Gly Asp Ser Pro Gly Pro
    450                 455                 460
Gly Gln Gln Gly Pro Gly Gln Gln Gly Pro Gly Gln Gln Gly Pro Gly
465                 470                 475                 480
Gln Gln Gly Pro Gly Gln Gln Gly Pro Gly Gly Gln Gly Ala Tyr Gly
                485                 490                 495
Pro Gly Ala Ser Ala Ala Ala Gly Ala Ala Gly Gly Tyr Gly Pro Gly
            500                 505                 510
Ser Gly Gln Gln Gly Pro Gly Gln Gln Gly Pro Gly Gln Gln Gly Pro
        515                 520                 525
Gly Gln Gln Gly Arg Gly Asp Ser Pro Gly Pro Gly Gln Gln Gly Pro
    530                 535                 540

Gly Gln Gln Gly Pro Gly Gln Gln Gly Pro Gly Gln Gln Gly Pro Tyr
545                 550                 555                 560
Gly Pro Gly Ala Ser Ala Ala Ala Ala Ala Ala Gly Gly Tyr Gly Pro
                565                 570                 575
Gly Ser Gly Gln Gln Gly Arg Gly Asp Ser Pro Gly Pro Gly Gln Gln
            580                 585                 590
Gly Pro Gly Gln Gln Gly Pro Gly Gly Gln Gly Pro Tyr Gly Pro Gly
        595                 600                 605
Ala Ala Ser Ala Ala Val Ser Val Gly Gly Tyr Gly Pro Gln Ser Ser
    610                 615                 620
Ser Val Pro Val Ala Ser Ala Val Ala Ser Arg Leu Ser Ser Pro Ala
625                 630                 635                 640
Ala Ser Ser Arg Val Ser Ser Ala Val Ser Ser Leu Val Ser Ser Gly
                645                 650                 655
Pro Thr Lys His Ala Ala Leu Ser Asn Thr Ile Ser Ser Val Val Ser
            660                 665                 670
Gln Val Ser Ala Ser Asn Pro Gly Leu Ser Gly Cys Asp Val Leu Val
        675                 680                 685
Gln Ala Leu Leu Glu Val Val Ser Ala Leu Val Ser Ile Leu Gly Ser
    690                 695                 700
Ser Ser Ile Gly Gln Ile Asn Tyr Gly Ala Ser Ala Gln Tyr Thr Gln
705                 710                 715                 720
Met Val Gly Gln Ser Val Ala Gln Ala Leu Ala
                725                 730

<210>  43
<211>  1995
<212>  DNA
<213>  Araneus diadematus

<400>  43
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggttctggg     300
caacaaggtc ctggcggtca aggcccttac ggtccgggta gtagcgctgc tgccgcagca     360
gcgggtggta atggcccagg cagtggacag cagggtgctg gacaacaggg cccaggtcag     420
caaggtccag gtgcatcagc cgctgctgct gcagccggtg gctacggacc aggttctggt     480
cagcaaggac cgggacagca ggggccaggt ggccaaggtc cgtatggacc aggagccagt     540
gctgctgcgg ctgccgctgg cggttacggt ccaggatcag gtcaaggacc cggccaacaa     600
ggccccggtg gacaaggacc atacggtcct ggtgcctccg cagccgcggc tgcagcggga     660
ggatatgggc ctggaagcgg gcagcaggga cccggacaac agggtcccgg gcaacagggg     720
cctggcggac agggtcccta cggacctggc gctagtgcag cggctgcggc cgctggaggc     780
tatggacctg gctacggtca acaaggccca gggcagcagg gtcctggagg ccagggacct     840
tacggcccag gcgcaagcgc tgcaagcgca gcatcaggtg gttacggccc tgggagcgga     900
cagcaaggcc cagggcaaca aggcccaggg gggcaagggc cttatggtcc cggagcgagc     960
gcagcggcgg cagctgccgg aggttacgga ccgggaagtg gtcaacaagg tcccggacag    1020
caaggtcccg gccagcaggg accgggtcaa caaggtccag gcggacaggg accgtatggg    1080
cctggcgcat cagcagctgc cgctgcggct ggcggatatg gccctgggag tggccaacaa    1140
gggcctggcc aacaaggtcc gggtcagcag ggaccaggcc agcaaggtcg tggtgatagt    1200
ccgggtccag gtcaacaggg gccaggtcaa cagggcccgg gccaacaagg accaggacaa    1260
caggggcctg gacagcaagg ccctggcggt cagggtgcct atggtccagg tgcttctgca    1320
gcagccggtg cagcgggcgg ttacggaccc ggtagcggcc aacagggtcc tggtcaacag    1380
ggtccaggac agcagggtcc gggtcaacag ggacgtggtg attcaccggg tcctgggcag    1440
caagggccgg ggcagcaagg tccgggacaa caaggacctg gtcagcaggg tccatacggt    1500
ccaggtgcca gcgcagctgc agcggccgcg ggtggatacg ggccaggctc aggccagcag    1560
ggccctgggc agcagggccc tggacagcaa ggaccaggtg ggcagggacc gtacggaccc    1620
ggtgctgcat cagcagcagt tagcgttggt ggctatggcc ctcagagcag cagcgttccg    1680
gttgccagtg cagttgcaag ccgtctgagc agtccggcag caagcagtcg tgtgagcagc    1740
gcagtgagca gcctggttag ctcaggtccg accaaacatg cagcactgag caataccatt    1800
agcagcgttg ttagccaggt tagcgcaagc aatccgggtc tgagcggttg tgatgttctg    1860

gtgcaggcac tgctggaagt tgttagcgca ctggttagca ttctgggtag cagcagcatt 1920
ggtcagatta actatggtgc gagcgcacag tatacccaga tggttggtca gagcgttgca 1980
caggcactgg cataa 1995

<210> 44
<211> 664
<212> PRT
<213> Araneus diadematus

<400> 44
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Gln Gly Pro Gly Gly Gln Gly Pro Tyr Gly Pro
            100                 105                 110
Gly Ser Ser Ala Ala Ala Ala Ala Ala Gly Gly Asn Gly Pro Gly Ser
        115                 120                 125
Gly Gln Gln Gly Ala Gly Gln Gln Gly Pro Gly Gln Gln Gly Pro Gly
    130                 135                 140
Ala Ser Ala Ala Ala Ala Ala Ala Gly Gly Tyr Gly Pro Gly Ser Gly
145                 150                 155                 160
Gln Gln Gly Pro Gly Gln Gln Gly Pro Gly Gly Gln Gly Pro Tyr Gly
                165                 170                 175
Pro Gly Ala Ser Ala Ala Ala Ala Ala Ala Gly Gly Tyr Gly Pro Gly
            180                 185                 190
Ser Gly Gln Gly Pro Gly Gln Gln Gly Pro Gly Gly Gln Gly Pro Tyr
        195                 200                 205
Gly Pro Gly Ala Ser Ala Ala Ala Ala Ala Ala Gly Gly Tyr Gly Pro
    210                 215                 220
Gly Ser Gly Gln Gln Gly Pro Gly Gln Gln Gly Pro Gly Gln Gln Gly
225                 230                 235                 240
Pro Gly Gly Gln Gly Pro Tyr Gly Pro Gly Ala Ser Ala Ala Ala Ala
                245                 250                 255
Ala Ala Gly Gly Tyr Gly Pro Gly Tyr Gly Gln Gln Gly Pro Gly Gln
            260                 265                 270
Gln Gly Pro Gly Gly Gln Gly Pro Tyr Gly Pro Gly Ala Ser Ala Ala
        275                 280                 285
Ser Ala Ala Ser Gly Gly Tyr Gly Pro Gly Ser Gly Gln Gln Gly Pro
    290                 295                 300
Gly Gln Gln Gly Pro Gly Gly Gln Gly Pro Tyr Gly Pro Gly Ala Ser
305                 310                 315                 320
Ala Ala Ala Ala Ala Ala Gly Gly Tyr Gly Pro Gly Ser Gly Gln Gln
                325                 330                 335
Gly Pro Gly Gln Gln Gly Pro Gly Gln Gln Gly Pro Gly Gln Gln Gly
            340                 345                 350
Pro Gly Gly Gln Gly Pro Tyr Gly Pro Gly Ala Ser Ala Ala Ala Ala
        355                 360                 365
Ala Ala Gly Gly Tyr Gly Pro Gly Ser Gly Gln Gln Gly Pro Gly Gln
    370                 375                 380
Gln Gly Pro Gly Gln Gln Gly Pro Gly Gln Gln Gly Arg Gly Asp Ser
385                 390                 395                 400
Pro Gly Pro Gly Gln Gln Gly Pro Gly Gln Gln Gly Pro Gly Gln Gln
                405                 410                 415

Gly Pro Gly Gln Gln Gly Pro Gly Gln Gln Gly Pro Gly Gly Gln Gly
            420                 425                 430
Ala Tyr Gly Pro Gly Ala Ser Ala Ala Ala Gly Ala Ala Gly Gly Tyr
        435                 440                 445
Gly Pro Gly Ser Gly Gln Gln Gly Pro Gly Gln Gln Gly Pro Gly Gln
    450                 455                 460
Gln Gly Pro Gly Gln Gln Gly Arg Gly Asp Ser Pro Gly Pro Gly Gln
465                 470                 475                 480
Gln Gly Pro Gly Gln Gln Gly Pro Gly Gln Gln Gly Pro Gly Gln Gln
                485                 490                 495
Gly Pro Tyr Gly Pro Gly Ala Ser Ala Ala Ala Ala Ala Ala Gly Gly
            500                 505                 510
Tyr Gly Pro Gly Ser Gly Gln Gln Gly Pro Gly Gln Gln Gly Pro Gly
        515                 520                 525
Gln Gln Gly Pro Gly Gly Gln Gly Pro Tyr Gly Pro Gly Ala Ala Ser
    530                 535                 540
Ala Ala Val Ser Val Gly Gly Tyr Gly Pro Gln Ser Ser Ser Val Pro
545                 550                 555                 560
Val Ala Ser Ala Val Ala Ser Arg Leu Ser Ser Pro Ala Ala Ser Ser
                565                 570                 575
Arg Val Ser Ser Ala Val Ser Ser Leu Val Ser Ser Gly Pro Thr Lys
            580                 585                 590
His Ala Ala Leu Ser Asn Thr Ile Ser Ser Val Val Ser Gln Val Ser
        595                 600                 605
Ala Ser Asn Pro Gly Leu Ser Gly Cys Asp Val Leu Val Gln Ala Leu
    610                 615                 620
Leu Glu Val Val Ser Ala Leu Val Ser Ile Leu Gly Ser Ser Ser Ile
625                 630                 635                 640
Gly Gln Ile Asn Tyr Gly Ala Ser Ala Gln Tyr Thr Gln Met Val Gly
                645                 650                 655
Gln Ser Val Ala Gln Ala Leu Ala
            660

<210>  45
<211>  2379
<212>  DNA
<213>  Araneus diadematus

<400>  45
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggtagtggt     300
caggttaccg gtcgtggtga tagtccggca agcgtgacag gtcgcggtga ttcaccggca     360
agccagggtc ctggtggcca aggtccgtac ggtcctggta gtagcgctgc tgccgcagca     420
gcgggtggta acggtccggg tagcggacaa cagggagccg gtcaacaagg gcccgggcaa      480
caaggtcctg gcgcaagcgc agccgctgct gctgcgggtg gctatggccc aggttctgga     540
cagcaggtta caggccgtgg cgattctcct gccagtgtga cgggacgtgg cgatagccct     600
gcatcaggcc ccgggcagca gggcccaggt ggacaaggac cttatggacc aggtgcctcc     660
gcagccgcgg cagccgccgg tggatacggt ccaggctctg gtcaggtaac gggaagaggt     720
gatagcccag cctcagttac gggtagaggc gattcaccag catcaggacc gggacagcaa     780
ggacccggag gccagggacc atacggacct ggtgcttcgg ctgccgctgc cgccgctggc     840
ggttacggac caggttccgg ccaacaaggc ccaggacagc aggggcctgg acaacagggt     900
cccggaggac agggtcccta tgggccaggc gctagtgcgg ctgcagctgc tgcaggaggc     960
tacggaccgg gatatggcca acaggtgact gggcgtggtg actctccggc atctgtaact    1020
ggtcgtggcg acagtcctgc cagcggacca ggccaacagg gccctggagg tcaaggccca    1080
tacggcccag gcgcatcagc agccagcgca gcaagtggcg ggtatggtcc aggcagcggt    1140
caacaggtca ccggtcgggg agacagccct gcgtcagtaa ccggacgcgg agattctcca    1200
gcaagcggac cggggcagca gggtcctggc gggcagggcc cttacggacc cggcgccagt    1260
gctgcggcgg ctgcggctgg agggtatgga cctggcagtg gacagcaggg tccaggccag    1320
caggggcccg gccagcaagg accagggcaa caagggccag gcggacaggg gccatacggt    1380

ccgggagctt cagcagcggc tgctgcggca gggggttacg gccctggaag cggtcaacaa 1440
ggtccgggac aacagggacc aggtcagcag ggcccaggtc agcaggttac aggtcgcgga 1500
gatagccctg ccagcgtgac cggtagaggc gacagtccgg caagtggtcc tggacagcaa 1560
gggccaggac agcaaggtcc tgggcagcag ggaccgggtc aacagggtcc aggccaacag 1620
ggtcctggcg gacagggtgc gtatggtcct ggagcttctg ccgcagcggg tgccgcaggc 1680
ggttatggcc ccggatctgg tcagcaagga cccggtcagc aaggaccggg acagcagggc 1740
ccagggcaac aggtaactgg cagaggcgat agtcctgcgt ctgttactgg acggggtgac 1800
tcaccagcga gcgggcctgg tcagcaggga cctggacaac aagggcctgg tcaacaaggc 1860
ccaggccaac aagggcctta tggtccaggc gctagcgcag cagcggcagc tgctggcggt 1920
tatggtccgg gaagtggcca acaaggaccc ggccaacagg gtccgggcca gcaaggtcca 1980
ggtggccaag gcccctatgg ccctggggca gcaagcgcag cagttagcgt tggtggctat 2040
ggtccgcaga gcagcagcgt tccggttgcg agcgcagttg caagccgtct gtcaagtccg 2100
gcagcaagta gccgtgtgag cagcgcagtt agcagcctgg ttagcagcgg tccgaccaaa 2160
catgcagcac tgagcaatac cattagcagc gttgttagcc aggttagcgc aagcaatccg 2220
ggtctgagcg gttgtgatgt tctggtgcag gcactgctgg aagttgttag cgcactggtg 2280
agcattctgg gtagcagcag cattggtcag attaactatg gtgccagcgc acagtatacc 2340
cagatggttg gtcagagcgt tgcacaggca ctggcataa 2379

<210> 46
<211> 792
<212> PRT
<213> Araneus diadematus

<400> 46
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Val Thr Gly Arg Gly Asp Ser Pro Ala Ser Val
            100                 105                 110
Thr Gly Arg Gly Asp Ser Pro Ala Ser Gln Gly Pro Gly Gly Gln Gly
        115                 120                 125
Pro Tyr Gly Pro Gly Ser Ser Ala Ala Ala Ala Ala Ala Gly Gly Asn
    130                 135                 140
Gly Pro Gly Ser Gly Gln Gln Gly Ala Gly Gln Gln Gly Pro Gly Gln
145                 150                 155                 160
Gln Gly Pro Gly Ala Ser Ala Ala Ala Ala Ala Ala Gly Gly Tyr Gly
                165                 170                 175
Pro Gly Ser Gly Gln Gln Val Thr Gly Arg Gly Asp Ser Pro Ala Ser
            180                 185                 190
Val Thr Gly Arg Gly Asp Ser Pro Ala Ser Gly Pro Gly Gln Gln Gly
        195                 200                 205
Pro Gly Gly Gln Gly Pro Tyr Gly Pro Gly Ala Ser Ala Ala Ala Ala
    210                 215                 220
Ala Ala Gly Gly Tyr Gly Pro Gly Ser Gly Gln Val Thr Gly Arg Gly
225                 230                 235                 240
Asp Ser Pro Ala Ser Val Thr Gly Arg Gly Asp Ser Pro Ala Ser Gly
                245                 250                 255
Pro Gly Gln Gln Gly Pro Gly Gly Gln Gly Pro Tyr Gly Pro Gly Ala
            260                 265                 270
Ser Ala Ala Ala Ala Ala Ala Gly Gly Tyr Gly Pro Gly Ser Gly Gln
        275                 280                 285
Gln Gly Pro Gly Gln Gln Gly Pro Gly Gln Gln Gly Pro Gly Gly Gln
    290                 295                 300

Gly Pro Tyr Gly Pro Gly Ala Ser Ala Ala Ala Ala Ala Ala Gly Gly
305                 310                 315                 320
Tyr Gly Pro Gly Tyr Gly Gln Gln Val Thr Gly Arg Gly Asp Ser Pro
                325                 330                 335
Ala Ser Val Thr Gly Arg Gly Asp Ser Pro Ala Ser Gly Pro Gly Gln
            340                 345                 350
Gln Gly Pro Gly Gly Gln Gly Pro Tyr Gly Pro Gly Ala Ser Ala Ala
        355                 360                 365
Ser Ala Ala Ser Gly Gly Tyr Gly Pro Gly Ser Gly Gln Gln Val Thr
    370                 375                 380
Gly Arg Gly Asp Ser Pro Ala Ser Val Thr Gly Arg Gly Asp Ser Pro
385                 390                 395                 400
Ala Ser Gly Pro Gly Gln Gln Gly Pro Gly Gly Gln Gly Pro Tyr Gly
                405                 410                 415
Pro Gly Ala Ser Ala Ala Ala Ala Ala Ala Gly Gly Tyr Gly Pro Gly
            420                 425                 430
Ser Gly Gln Gln Gly Pro Gly Gln Gln Gly Pro Gly Gln Gln Gly Pro
        435                 440                 445
Gly Gln Gln Gly Pro Gly Gly Gln Gly Pro Tyr Gly Pro Gly Ala Ser
    450                 455                 460
Ala Ala Ala Ala Ala Ala Gly Gly Tyr Gly Pro Gly Ser Gly Gln Gln
465                 470                 475                 480
Gly Pro Gly Gln Gln Gly Pro Gly Gln Gln Gly Pro Gly Gln Gln Val
                485                 490                 495
Thr Gly Arg Gly Asp Ser Pro Ala Ser Val Thr Gly Arg Gly Asp Ser
            500                 505                 510
Pro Ala Ser Gly Pro Gly Gln Gln Gly Pro Gly Gln Gln Gly Pro Gly
        515                 520                 525
Gln Gln Gly Pro Gly Gln Gln Gly Pro Gly Gln Gln Gly Pro Gly Gly
    530                 535                 540
Gln Gly Ala Tyr Gly Pro Gly Ala Ser Ala Ala Ala Gly Ala Ala Gly
545                 550                 555                 560
Gly Tyr Gly Pro Gly Ser Gly Gln Gln Gly Pro Gly Gln Gln Gly Pro
                565                 570                 575
Gly Gln Gln Gly Pro Gly Gln Gln Val Thr Gly Arg Gly Asp Ser Pro
            580                 585                 590
Ala Ser Val Thr Gly Arg Gly Asp Ser Pro Ala Ser Gly Pro Gly Gln
        595                 600                 605
Gln Gly Pro Gly Gln Gln Gly Pro Gly Gln Gln Gly Pro Gly Gln Gln
    610                 615                 620
Gly Pro Tyr Gly Pro Gly Ala Ser Ala Ala Ala Ala Ala Ala Gly Gly
625                 630                 635                 640
Tyr Gly Pro Gly Ser Gly Gln Gln Gly Pro Gly Gln Gln Gly Pro Gly
                645                 650                 655
Gln Gln Gly Pro Gly Gly Gln Gly Pro Tyr Gly Pro Gly Ala Ala Ser
            660                 665                 670
Ala Ala Val Ser Val Gly Gly Tyr Gly Pro Gln Ser Ser Ser Val Pro
        675                 680                 685
Val Ala Ser Ala Val Ala Ser Arg Leu Ser Ser Pro Ala Ala Ser Ser
    690                 695                 700
Arg Val Ser Ser Ala Val Ser Ser Leu Val Ser Ser Gly Pro Thr Lys
705                 710                 715                 720
His Ala Ala Leu Ser Asn Thr Ile Ser Ser Val Val Ser Gln Val Ser
                725                 730                 735
Ala Ser Asn Pro Gly Leu Ser Gly Cys Asp Val Leu Val Gln Ala Leu
            740                 745                 750
Leu Glu Val Val Ser Ala Leu Val Ser Ile Leu Gly Ser Ser Ser Ile
        755                 760                 765
Gly Gln Ile Asn Tyr Gly Ala Ser Ala Gln Tyr Thr Gln Met Val Gly
    770                 775                 780
Gln Ser Val Ala Gln Ala Leu Ala
785                 790

<210>  47
<211>  2316
<212>  DNA
<213>  Araneus diadematus

<400>  47
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggtagt     180
ggacagcagg ttaccggtcg tggtgatagt ccggcaagcg gtccgagcca gcaaggtcca     240
ggccaacaag ggccaggtgg tcagccgtat ggccctggtg cttctgccgc tgctgcagca     300
gcgggtggct atggaccggg ttcaggtcag gtgacaggtc gcggtgattc accggcaagc     360
cagggtcctg gtggtcaggg tccttatggt ccaggttctt cagcagctgc agcggcagca     420
ggcggtaacg gaccgggaag tggccagcaa gtgacaggcc gtggcgatag ccctgccagc     480
ggtgctggac aacagggccc agggcaacag gggccgggag catctgcagc tgcggctgct     540
gctggcggtt atggcccagg ctctggccaa caggttacgg gacgtggcga ttctcctgca     600
tcaggccctg gacagcaagg gcccggagga cagggtccgt acggtcctgg cgcatctgcg     660
gcagctgccg ccgcaggcgg atatggacct ggcagcggtc aagtgactgg acgcggagat     720
agtccagcat caggacccgg tcaacaaggt cctggaggcc aaggccctta cggacctggt     780
gcaagcgctg cggccgcagc ggctggaggt tacggtccag gatcgggaca acaagtgacc     840
ggtagaggtg atagcccagc ctcaggccca ggccagcagg gtccaggaca gcagggaccg     900
ggtggtcaag gtccatacgg accaggcgca agtgccgccg cggctgccgc aggaggctac     960
ggccctggat atgggcagca ggtaacagga cggggtgaca gccctgcgtc aggaccgggg    1020
cagcaaggac caggtggcca gggaccctat gggccaggcg catcagcagc cagcgcagca    1080
agtggtggat acggtcccgg atcaggtcag caagttacgg gtagaggcga ttcaccagcc    1140
agcggaccag gacaacaggg tcccggcgga caaggaccgt atggacccgg agcatcagcc    1200
gcagctgctg ctgccggtgg ctacggtcca ggttccggtc aacaggtaac tggcagaggc    1260
gatagtcccg ctagcgggcc tgggcaacaa ggtccggggc agcaggggcc agggcagcaa    1320
gggccaggcg gacaaggccc ttatggccca ggtgcctctg cagcagcggc agctgcggga    1380
ggatatggtc cgggaagcgg tcagcaagga cccggacagc aaggtccggg acaacagggt    1440
cctggtcagc aggttactgg tcggggagac tcaccggcat cagggccagg acaacaagga    1500
ccaggccagc aaggccctgg ccagcaagga ccgggtcaac aaggcccagg acaacaaggc    1560
ccgggtggcc agggtgcata cggtccgggt gcttcagctg cagccggtgc agcgggaggt    1620
tatgggcctg gctcaggtca gcaggggccc ggccaacaag gtcccgggca acaaggacct    1680
ggacaacagg tgactgggcg tggtgactca ccagcgagtg ggcctggaca gcagggtcct    1740
ggccaacagg gcccaggtca gcaagggcct gggcagcagg gcccttacgg tccaggggct    1800
tctgctgccg cagctgcggc aggtggttat ggaccaggaa gtggtcaaca agtaaccgga    1860
cggggagatt ctccagcgtc tgggccaggt cagcagggac caggtcaaca gggtccaggt    1920
ggccaggggc cgtacggacc aggtgcagca agcgcagcag ttagcgttgg tggctatggt    1980
ccgcagagca gcagcgttcc ggttgcgagc gcagttgcaa gccgtctgag cagtccggca    2040
gcaagcagtc gtgtgagcag cgcagttagc agcctggtta gcagtggtcc gaccaaacat    2100
gcagcactga gcaataccat tagcagcgtt gttagccagg ttagcgcaag caatccgggt    2160
ctgagcggtt gtgatgttct ggttcaggca ctgctggaag ttgttagcgc actggtgagc    2220
attctgggta gcagcagcat tggtcagatt aactatggtg ccagcgcaca gtatacccag    2280
atggttggtc agagcgttgc acaggcactg gcataa                              2316

<210>  48
<211>  771
<212>  PRT
<213>  Araneus diadematus

<400>  48
   Met His His His His His His His His His His Ser Ser Gly Ser Ser
   1               5                   10                  15
   Ala Arg Ala Gly Ser Gly Gln Gln Gly Pro Gly Gln Gln Gly Pro Gly
               20                  25                  30
   Gln Gln Gly Pro Gly Gln Gln Gly Pro Tyr Gly Pro Gly Ala Ser Ala
           35                  40                  45
   Ala Ala Ala Ala Ala Gly Gly Tyr Gly Pro Gly Ser Gly Gln Gln Val
       50                  55                  60
   Thr Gly Arg Gly Asp Ser Pro Ala Ser Gly Pro Ser Gln Gln Gly Pro
   65                  70                  75                  80
   Gly Gln Gln Gly Pro Gly Gly Gln Pro Tyr Gly Pro Gly Ala Ser Ala
                   85                  90                  95
   Ala Ala Ala Ala Ala Gly Gly Tyr Gly Pro Gly Ser Gly Gln Val Thr
               100                 105                 110
   Gly Arg Gly Asp Ser Pro Ala Ser Gln Gly Pro Gly Gly Gln Gly Pro
           115                 120                 125
   Tyr Gly Pro Gly Ser Ser Ala Ala Ala Ala Ala Ala Gly Gly Asn Gly
       130                 135                 140
   Pro Gly Ser Gly Gln Gln Val Thr Gly Arg Gly Asp Ser Pro Ala Ser
   145                 150                 155                 160
   Gly Ala Gly Gln Gln Gly Pro Gly Gln Gln Gly Pro Gly Ala Ser Ala
                   165                 170                 175
   Ala Ala Ala Ala Ala Gly Gly Tyr Gly Pro Gly Ser Gly Gln Gln Val
               180                 185                 190
   Thr Gly Arg Gly Asp Ser Pro Ala Ser Gly Pro Gly Gln Gln Gly Pro
           195                 200                 205
   Gly Gly Gln Gly Pro Tyr Gly Pro Gly Ala Ser Ala Ala Ala Ala Ala
       210                 215                 220
   Ala Gly Gly Tyr Gly Pro Gly Ser Gly Gln Val Thr Gly Arg Gly Asp
   225                 230                 235                 240
   Ser Pro Ala Ser Gly Pro Gly Gln Gln Gly Pro Gly Gly Gln Gly Pro
                   245                 250                 255
   Tyr Gly Pro Gly Ala Ser Ala Ala Ala Ala Ala Ala Gly Gly Tyr Gly
               260                 265                 270
   Pro Gly Ser Gly Gln Gln Val Thr Gly Arg Gly Asp Ser Pro Ala Ser
           275                 280                 285
   Gly Pro Gly Gln Gln Gly Pro Gly Gln Gln Gly Pro Gly Gly Gln Gly
       290                 295                 300
   Pro Tyr Gly Pro Gly Ala Ser Ala Ala Ala Ala Ala Ala Gly Gly Tyr
   305                 310                 315                 320
   Gly Pro Gly Tyr Gly Gln Gln Val Thr Gly Arg Gly Asp Ser Pro Ala
                   325                 330                 335
   Ser Gly Pro Gly Gln Gln Gly Pro Gly Gly Gln Gly Pro Tyr Gly Pro
               340                 345                 350
   Gly Ala Ser Ala Ala Ser Ala Ala Ser Gly Gly Tyr Gly Pro Gly Ser
           355                 360                 365
   Gly Gln Gln Val Thr Gly Arg Gly Asp Ser Pro Ala Ser Gly Pro Gly
       370                 375                 380
   Gln Gln Gly Pro Gly Gly Gln Gly Pro Tyr Gly Pro Gly Ala Ser Ala
   385                 390                 395                 400
   Ala Ala Ala Ala Ala Gly Gly Tyr Gly Pro Gly Ser Gly Gln Gln Val
                   405                 410                 415
   Thr Gly Arg Gly Asp Ser Pro Ala Ser Gly Pro Gly Gln Gln Gly Pro
               420                 425                 430
   Gly Gln Gln Gly Pro Gly Gln Gln Gly Pro Gly Gly Gln Gly Pro Tyr
           435                 440                 445
   Gly Pro Gly Ala Ser Ala Ala Ala Ala Ala Ala Gly Gly Tyr Gly Pro
       450                 455                 460
   Gly Ser Gly Gln Gln Gly Pro Gly Gln Gln Gly Pro Gly Gln Gln Gly
   465                 470                 475                 480
   Pro Gly Gln Gln Val Thr Gly Arg Gly Asp Ser Pro Ala Ser Gly Pro
                   485                 490                 495
   Gly Gln Gln Gly Pro Gly Gln Gln Gly Pro Gly Gln Gln Gly Pro Gly
               500                 505                 510
   Gln Gln Gly Pro Gly Gln Gln Gly Pro Gly Gly Gln Gly Ala Tyr Gly
           515                 520                 525
   Pro Gly Ala Ser Ala Ala Ala Gly Ala Ala Gly Gly Tyr Gly Pro Gly
       530                 535                 540
   Ser Gly Gln Gln Gly Pro Gly Gln Gln Gly Pro Gly Gln Gln Gly Pro
   545                 550                 555                 560
   Gly Gln Gln Val Thr Gly Arg Gly Asp Ser Pro Ala Ser Gly Pro Gly
                   565                 570                 575
   Gln Gln Gly Pro Gly Gln Gln Gly Pro Gly Gln Gln Gly Pro Gly Gln
               580                 585                 590
   Gln Gly Pro Tyr Gly Pro Gly Ala Ser Ala Ala Ala Ala Ala Ala Gly
           595                 600                 605
   Gly Tyr Gly Pro Gly Ser Gly Gln Gln Val Thr Gly Arg Gly Asp Ser
       610                 615                 620
   Pro Ala Ser Gly Pro Gly Gln Gln Gly Pro Gly Gln Gln Gly Pro Gly
   625                 630                 635                 640
   Gly Gln Gly Pro Tyr Gly Pro Gly Ala Ala Ser Ala Ala Val Ser Val
                   645                 650                 655
   Gly Gly Tyr Gly Pro Gln Ser Ser Ser Val Pro Val Ala Ser Ala Val
               660                 665                 670
   Ala Ser Arg Leu Ser Ser Pro Ala Ala Ser Ser Arg Val Ser Ser Ala
           675                 680                 685
   Val Ser Ser Leu Val Ser Ser Gly Pro Thr Lys His Ala Ala Leu Ser
       690                 695                 700
   Asn Thr Ile Ser Ser Val Val Ser Gln Val Ser Ala Ser Asn Pro Gly
   705                 710                 715                 720
   Leu Ser Gly Cys Asp Val Leu Val Gln Ala Leu Leu Glu Val Val Ser
                   725                 730                 735
   Ala Leu Val Ser Ile Leu Gly Ser Ser Ser Ile Gly Gln Ile Asn Tyr
               740                 745                 750
   Gly Ala Ser Ala Gln Tyr Thr Gln Met Val Gly Gln Ser Val Ala Gln
           755                 760                 765
   Ala Leu Ala
       770

<210>  49
<211>  2256
<212>  DNA
<213>  Araneus diadematus

<400>  49
atgcatcatc accaccatca ccatcatcat cacagcagcg gtagcagcct ggaagttctg      60
tttcagggtc cggcacgtgc aggtagcggt cagcagggtc ctggccagca aggtccgggt     120
caacagggac cgggacagca gggaccttat ggtccgggtg caagcgcagc agcagccgca     180
gccggtggtt atggtcctgg ttcaggccaa cagggtccaa gtcagcaagg cccaggacaa     240
caaggtcctg gtggccaggg tccgtatggc cctggtgcat ctgctgcggc agcggcagca     300
ggcggatatg gaccgggtag tggtcagggt aaaaaacagc gttttcgtca tcgtaatcgt     360
aaaggtcagg gtccaggtgg tcaaggtcca tacggtccgg gtagcagtgc agctgctgca     420
gccgctggcg gtaatggtcc aggtagtgga cagcaaggtg caggtcagca aggacctggg     480
caacagggtc caggcgcaag tgcagccgca gctgctgcag gcggttatgg tccaggctct     540
ggtcagcagg gcaaaaaaca acgctttcgc caccgcaacc gcaaaggtgg cccagggcag     600
cagggtccgg gtggacaagg cccttatggc ccaggcgcat ccgcagcagc ggcagccgca     660
gggggttacg gacctggtag cggacaaggc aaaaaacagc ggttccggca tcggaaccgc     720
aaaggcggtc cgggacagca aggccctggc ggtcaaggtc cttatggtcc tggggcatca     780
gctgcagccg cagcggcagg gggatatggc cctggaagtg gccagcaggg accaggccaa     840
caagggccag ggcagcaagg gccaggtggt cagggacctt acggaccagg tgcctcagcg     900
gcagcagccg cagctggggg atacggtccg ggttatggtc agcaaggtaa aaaacagcgc     960
ttccgtcacc gcaatcggaa aggcggacct ggtcaacaag gaccaggtgg acaaggccct    1020
tacggtcctg gcgctagtgc agcaagcgca gccagcggtg gctatggtcc gggaagcgga    1080
caacagggaa aaaaacaacg tttccggcac cggaatcgta aaggtgggcc tggacaacag    1140
ggacctggcg gtcagggtcc ttacggtccg ggtgcttctg cagcggcagc cgcagcgggt    1200
ggctatggcc caggtagtgg tcaacaaggc cctggtcagc agggacctgg acaacaaggc    1260
cctggacaac aagggccagg gggtcaaggc ccttatggac cgggtgccag tgccgcagct    1320
gcagcggcag gcggttacgg tccgggttcc ggacaacaag gaccgggtca gcaaggtcca    1380
ggccagcagg gtccagggca gcagggaaaa aaacagcggt tccgccaccg taatcgcaaa    1440
gggggtccag gccaacaagg acctggccaa caaggtcctg gtcagcaagg ccctgggcag     1500
caaggcccag gacagcaggg accaggtggg cagggtgcgt atggccaggg ggctagtgct    1560
gcagcgggtg ctgcgggtgg atacggaccg ggatctggac aacagggtcc tggtcaacag     1620
ggtccaggac agcaaggtcc tggacagcaa gggaaaaaac aacggtttcg tcatcgtaac    1680
cggaaagggg gtcctggcca gcagggaccg ggacagcaag gaccaggtca acaaggccca    1740

ggtcaacaag gaccttatgg tccaggtgcg tcagcagccg cagctgcagc gggtggatac 1800
ggtcctggta gcggtcagca agggccaggt caacagggac ctggtcagca gggtccgggt 1860
ggtcagggtc cttatggacc tggcgcagca agtgcagcag ttagcgttgg tggttatgga 1920
ccgcagagca gcagcgttcc ggttgccagc gcagttgcaa gccgtctgag cagtccggca 1980
gcaagcagtc gtgttagcag cgcagtgagc agtctggtta gctcaggtcc gaccaaacat 2040
gcagcactga gcaataccat tagcagcgtt gttagccagg ttagcgcaag caatccgggt 2100
ctgagcggtt gtgatgttct ggtgcaggca ctgctggaag ttgttagcgc actggttagc 2160
attctgggta gcagcagcat tggtcagatt aactatggtg cgagcgcaca gtatacccag 2220
atggttggtc agagcgttgc acaggcactg gcctaa 2256

<210> 50
<211> 751
<212> PRT
<213> Araneus diadematus

<400> 50
Met His His His His His His His His His His Ser Ser Gly Ser Ser
1               5                   10                  15
Leu Glu Val Leu Phe Gln Gly Pro Ala Arg Ala Gly Ser Gly Gln Gln
            20                  25                  30
Gly Pro Gly Gln Gln Gly Pro Gly Gln Gln Gly Pro Gly Gln Gln Gly
        35                  40                  45
Pro Tyr Gly Pro Gly Ala Ser Ala Ala Ala Ala Ala Ala Gly Gly Tyr
    50                  55                  60
Gly Pro Gly Ser Gly Gln Gln Gly Pro Ser Gln Gln Gly Pro Gly Gln
65                  70                  75                  80
Gln Gly Pro Gly Gly Gln Gly Pro Tyr Gly Pro Gly Ala Ser Ala Ala
                85                  90                  95
Ala Ala Ala Ala Gly Gly Tyr Gly Pro Gly Ser Gly Gln Gly Lys Lys
            100                 105                 110
Gln Arg Phe Arg His Arg Asn Arg Lys Gly Gln Gly Pro Gly Gly Gln
        115                 120                 125
Gly Pro Tyr Gly Pro Gly Ser Ser Ala Ala Ala Ala Ala Ala Gly Gly
    130                 135                 140
Asn Gly Pro Gly Ser Gly Gln Gln Gly Ala Gly Gln Gln Gly Pro Gly
145                 150                 155                 160
Gln Gln Gly Pro Gly Ala Ser Ala Ala Ala Ala Ala Ala Gly Gly Tyr
                165                 170                 175
Gly Pro Gly Ser Gly Gln Gln Gly Lys Lys Gln Arg Phe Arg His Arg
            180                 185                 190
Asn Arg Lys Gly Gly Pro Gly Gln Gln Gly Pro Gly Gly Gln Gly Pro
        195                 200                 205
Tyr Gly Pro Gly Ala Ser Ala Ala Ala Ala Ala Ala Gly Gly Tyr Gly
    210                 215                 220
Pro Gly Ser Gly Gln Gly Lys Lys Gln Arg Phe Arg His Arg Asn Arg
225                 230                 235                 240
Lys Gly Gly Pro Gly Gln Gln Gly Pro Gly Gly Gln Gly Pro Tyr Gly
                245                 250                 255
Pro Gly Ala Ser Ala Ala Ala Ala Ala Ala Gly Gly Tyr Gly Pro Gly
            260                 265                 270
Ser Gly Gln Gln Gly Pro Gly Gln Gln Gly Pro Gly Gln Gln Gly Pro
        275                 280                 285
Gly Gly Gln Gly Pro Tyr Gly Pro Gly Ala Ser Ala Ala Ala Ala Ala
    290                 295                 300
Ala Gly Gly Tyr Gly Pro Gly Tyr Gly Gln Gln Gly Lys Lys Gln Arg
305                 310                 315                 320
Phe Arg His Arg Asn Arg Lys Gly Gly Pro Gly Gln Gln Gly Pro Gly
                325                 330                 335
Gly Gln Gly Pro Tyr Gly Pro Gly Ala Ser Ala Ala Ser Ala Ala Ser
            340                 345                 350
Gly Gly Tyr Gly Pro Gly Ser Gly Gln Gln Gly Lys Lys Gln Arg Phe
        355                 360                 365

Arg His Arg Asn Arg Lys Gly Gly Pro Gly Gln Gln Gly Pro Gly Gly
    370                 375                 380
Gln Gly Pro Tyr Gly Pro Gly Ala Ser Ala Ala Ala Ala Ala Ala Gly
385                 390                 395                 400
Gly Tyr Gly Pro Gly Ser Gly Gln Gln Gly Pro Gly Gln Gln Gly Pro
                405                 410                 415
Gly Gln Gln Gly Pro Gly Gln Gln Gly Pro Gly Gly Gln Gly Pro Tyr
            420                 425                 430
Gly Pro Gly Ala Ser Ala Ala Ala Ala Ala Ala Gly Gly Tyr Gly Pro
        435                 440                 445
Gly Ser Gly Gln Gln Gly Pro Gly Gln Gln Gly Pro Gly Gln Gln Gly
    450                 455                 460
Pro Gly Gln Gln Gly Lys Lys Gln Arg Phe Arg His Arg Asn Arg Lys
465                 470                 475                 480
Gly Gly Pro Gly Gln Gln Gly Pro Gly Gln Gln Gly Pro Gly Gln Gln
                485                 490                 495
Gly Pro Gly Gln Gln Gly Pro Gly Gln Gln Gly Pro Gly Gly Gln Gly
            500                 505                 510
Ala Tyr Gly Pro Gly Ala Ser Ala Ala Ala Gly Ala Ala Gly Gly Tyr
        515                 520                 525
Gly Pro Gly Ser Gly Gln Gln Gly Pro Gly Gln Gln Gly Pro Gly Gln
    530                 535                 540
Gln Gly Pro Gly Gln Gln Gly Lys Lys Gln Arg Phe Arg His Arg Asn
545                 550                 555                 560
Arg Lys Gly Gly Pro Gly Gln Gln Gly Pro Gly Gln Gln Gly Pro Gly
                565                 570                 575
Gln Gln Gly Pro Gly Gln Gln Gly Pro Tyr Gly Pro Gly Ala Ser Ala
            580                 585                 590
Ala Ala Ala Ala Ala Gly Gly Tyr Gly Pro Gly Ser Gly Gln Gln Gly
        595                 600                 605
Pro Gly Gln Gln Gly Pro Gly Gln Gln Gly Pro Gly Gly Gln Gly Pro
    610                 615                 620
Tyr Gly Pro Gly Ala Ala Ser Ala Ala Val Ser Val Gly Gly Tyr Gly
625                 630                 635                 640
Pro Gln Ser Ser Ser Val Pro Val Ala Ser Ala Val Ala Ser Arg Leu
                645                 650                 655
Ser Ser Pro Ala Ala Ser Ser Arg Val Ser Ser Ala Val Ser Ser Leu
            660                 665                 670
Val Ser Ser Gly Pro Thr Lys His Ala Ala Leu Ser Asn Thr Ile Ser
        675                 680                 685
Ser Val Val Ser Gln Val Ser Ala Ser Asn Pro Gly Leu Ser Gly Cys
    690                 695                 700
Asp Val Leu Val Gln Ala Leu Leu Glu Val Val Ser Ala Leu Val Ser
705                 710                 715                 720
Ile Leu Gly Ser Ser Ser Ile Gly Gln Ile Asn Tyr Gly Ala Ser Ala
                725                 730                 735
Gln Tyr Thr Gln Met Val Gly Gln Ser Val Ala Gln Ala Leu Ala
            740                 745                 750

<210>  51
<211>  2025
<212>  DNA
<213>  Araneus diadematus

<400>  51
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagca gcggcagtta ccggtcgtgg tgatagtccg     300
gcaagccagg gtcctggtgg ccaaggtccg tacggtcctg gtagttctgc tgccgctgcc     360
gcagcaggcg gtaacggtcc aggcagtgga cagcagggtg ctgggcaaca gggcccaggt     420

cagcaaggtc caggtgcatc agcagcagcg gctgcagccg tgaccggtcg cggagattca 480
ccggcaagcg gaccggggca acaaggtcct ggcggacaag gcccttatgg tccaggagcg 540
agcgctgctg ctgcagctgc ggtgacaggt cgtggcgact ctccggcatc aggacccggc 600
caacaagggc ccggaggtca aggaccttac ggaccaggtg ccagtgccgc tgcagcggcg 660
gcaggcggtt atggaccggg aagcgggcag caagggccag ggcagcaggg gcctggacaa 720
cagggtcccg gtggacaggg cccatacgga cctggtgcaa gcgcagccgc cgcagcagca 780
gtaacaggcc gtggcgatag ccctgcaagc ggtcctggac agcaagggcc tggagggcag 840
ggtccatacg gtccgggagc atcagccgca tctgcagcaa gcgttacagg acgcggtgat 900
tctccagcca gcgggccagg tcaacaaggc ccaggcggac agggtccgta cggcccaggc 960
gctagtgcag ccgctgctgc cgcaggagga tacggaccgg gtagtggtca gcaaggaccc 1020
ggacagcaag gtccgggaca gcagggaccg ggccaacaag gaccaggggg ccagggaccc 1080
tacgggccag gagcttccgc ggctgccgcg gcagccggag gctatggccc aggtagtggc 1140
caacaaggtc caggccagca aggccccggg caacaagggc cgggtcaaca ggttacaggc 1200
cgtggtgaca gtcctgcctc aggtcctggg cagcagggtc ccggccagca ggggccagga 1260
cagcaaggtc ctggccaaca aggcccagga cagcagggtc cgggtggcca gggtgcttat 1320
ggtccaggcg cttctgcagc agccggtgct gcgggtggtt acggtcccgg tagcggacaa 1380
caggggccag gccaacaggg acccgggcag caaggacctg gccagcaagt gaccggtcgc 1440
ggtgatagcc ctgcatcagg tccagggcaa cagggacctg gtcaacaagg gcctgggcaa 1500
caaggaccag gccaacaagg tccgtatggt ccaggtgcct ccgcagctgc agcagctgct 1560
ggcggatacg gtccaggatc aggtcagcag ggaccagggc aacagggtcc ggggcagcag 1620
ggtcctggtg gtcagggacc gtatggacct ggcgcagcaa gcgcagcagt tagcgttggt 1680
ggctatggac cgcagagcag cagtgttccg gttgccagcg cagttgcaag ccgtctgagc 1740
agtccggcag caagcagtcg tgttagcagt gcagttagca gcctggttag cagcggtccg 1800
accaaacatg cagcactgag caataccatt agcagcgttg ttagccaggt tagcgcaagc 1860
aatccgggtc tgagcggttg tgatgttctg gtgcaggcac tgctggaagt tgttagcgca 1920
ctggtgagca ttctgggtag cagcagcatt ggtcagatta actatggtgc gagcgcacag 1980
tatacccaga tggttggtca gagcgttgca caggcactgg cataa 2025

<210> 52
<211> 674
<212> PRT
<213> Araneus diadematus

<400> 52
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Val Thr Gly Arg
                85                  90                  95
Gly Asp Ser Pro Ala Ser Gln Gly Pro Gly Gly Gln Gly Pro Tyr Gly
            100                 105                 110
Pro Gly Ser Ser Ala Ala Ala Ala Ala Ala Gly Gly Asn Gly Pro Gly
        115                 120                 125
Ser Gly Gln Gln Gly Ala Gly Gln Gln Gly Pro Gly Gln Gln Gly Pro
    130                 135                 140
Gly Ala Ser Ala Ala Ala Ala Ala Ala Val Thr Gly Arg Gly Asp Ser
145                 150                 155                 160
Pro Ala Ser Gly Pro Gly Gln Gln Gly Pro Gly Gly Gln Gly Pro Tyr
                165                 170                 175
Gly Pro Gly Ala Ser Ala Ala Ala Ala Ala Ala Val Thr Gly Arg Gly
            180                 185                 190
Asp Ser Pro Ala Ser Gly Pro Gly Gln Gln Gly Pro Gly Gly Gln Gly
        195                 200                 205
Pro Tyr Gly Pro Gly Ala Ser Ala Ala Ala Ala Ala Ala Gly Gly Tyr
    210                 215                 220

Gly Pro Gly Ser Gly Gln Gln Gly Pro Gly Gln Gln Gly Pro Gly Gln
225                 230                 235                 240
Gln Gly Pro Gly Gly Gln Gly Pro Tyr Gly Pro Gly Ala Ser Ala Ala
                245                 250                 255
Ala Ala Ala Ala Val Thr Gly Arg Gly Asp Ser Pro Ala Ser Gly Pro
            260                 265                 270
Gly Gln Gln Gly Pro Gly Gly Gln Gly Pro Tyr Gly Pro Gly Ala Ser
        275                 280                 285
Ala Ala Ser Ala Ala Ser Val Thr Gly Arg Gly Asp Ser Pro Ala Ser
    290                 295                 300
Gly Pro Gly Gln Gln Gly Pro Gly Gly Gln Gly Pro Tyr Gly Pro Gly
305                 310                 315                 320
Ala Ser Ala Ala Ala Ala Ala Ala Gly Gly Tyr Gly Pro Gly Ser Gly
                325                 330                 335
Gln Gln Gly Pro Gly Gln Gln Gly Pro Gly Gln Gln Gly Pro Gly Gln
            340                 345                 350
Gln Gly Pro Gly Gly Gln Gly Pro Tyr Gly Pro Gly Ala Ser Ala Ala
        355                 360                 365
Ala Ala Ala Ala Gly Gly Tyr Gly Pro Gly Ser Gly Gln Gln Gly Pro
    370                 375                 380
Gly Gln Gln Gly Pro Gly Gln Gln Gly Pro Gly Gln Gln Val Thr Gly
385                 390                 395                 400
Arg Gly Asp Ser Pro Ala Ser Gly Pro Gly Gln Gln Gly Pro Gly Gln
                405                 410                 415
Gln Gly Pro Gly Gln Gln Gly Pro Gly Gln Gln Gly Pro Gly Gln Gln
            420                 425                 430
Gly Pro Gly Gly Gln Gly Ala Tyr Gly Pro Gly Ala Ser Ala Ala Ala
        435                 440                 445
Gly Ala Ala Gly Gly Tyr Gly Pro Gly Ser Gly Gln Gln Gly Pro Gly
    450                 455                 460
Gln Gln Gly Pro Gly Gln Gln Gly Pro Gly Gln Gln Val Thr Gly Arg
465                 470                 475                 480
Gly Asp Ser Pro Ala Ser Gly Pro Gly Gln Gln Gly Pro Gly Gln Gln
                485                 490                 495
Gly Pro Gly Gln Gln Gly Pro Gly Gln Gln Gly Pro Tyr Gly Pro Gly
            500                 505                 510
Ala Ser Ala Ala Ala Ala Ala Ala Gly Gly Tyr Gly Pro Gly Ser Gly
        515                 520                 525
Gln Gln Gly Pro Gly Gln Gln Gly Pro Gly Gln Gln Gly Pro Gly Gly
    530                 535                 540
Gln Gly Pro Tyr Gly Pro Gly Ala Ala Ser Ala Ala Val Ser Val Gly
545                 550                 555                 560
Gly Tyr Gly Pro Gln Ser Ser Ser Val Pro Val Ala Ser Ala Val Ala
                565                 570                 575
Ser Arg Leu Ser Ser Pro Ala Ala Ser Ser Arg Val Ser Ser Ala Val
            580                 585                 590
Ser Ser Leu Val Ser Ser Gly Pro Thr Lys His Ala Ala Leu Ser Asn
        595                 600                 605
Thr Ile Ser Ser Val Val Ser Gln Val Ser Ala Ser Asn Pro Gly Leu
    610                 615                 620
Ser Gly Cys Asp Val Leu Val Gln Ala Leu Leu Glu Val Val Ser Ala
625                 630                 635                 640
Leu Val Ser Ile Leu Gly Ser Ser Ser Ile Gly Gln Ile Asn Tyr Gly
                645                 650                 655
Ala Ser Ala Gln Tyr Thr Gln Met Val Gly Gln Ser Val Ala Gln Ala
            660                 665                 670
Leu Ala

<210>  53
<211>  2019
<212>  DNA

<213>  Araneus diadematus

<400>  53
atgcatcatc accaccatca ccatcatcat cacagcagcg gtagcagcgc acgtgcaggt      60
agcggtcagc agggtccggg tcagcaaggc cctggtcaac agggacctgg ccagcaggga     120
ccgtatggtc cgggtgcaag tgcagcagca gccgcagccg gtggttatgg tcctggttca     180
ggccagcaag gtccgagcca acagggtcca ggacaacaag gaccgggtgg tcagggtcct     240
tatggccctg gtgcatcagc tgcggcagcg gcagcaggcg gatatggacc gggttctggg     300
caacaaggtc ctggcggtca aggcccttac ggtccgggta gtagcgctgc tgccgcagca     360
gcgggtggta atggcccagg cagtggacag cagggtgctg gacaacaggg cccaggtcag     420
caaggtccag gtgcatcagc cgctgctgct gcagccggtg gctacggacc aggttctggt     480
cagcaaggac cgggacagca ggggccaggt ggccaaggtc cgtatggacc aggagccagt     540
gctgctgcgg ctgccgctgg cggttacggt ccaggatcag gtcaaggacc cggccaacaa     600
ggccccggtg gacaaggacc atacggtcct ggtgcctccg cagccgcggc tgcagcggga     660
ggatatgggc ctggaagcgg gcagcaggga cccggacaac agggtcccgg gcaacagggg     720
cctggcggac agggtcccta cggacctggc gctagtgcag cggctgcggc cgctggaggc     780
tatggacctg gctacggtca acaagggcca gggcagcagg gtcctggagg ccagggacct     840
tacggcccag gcgcaagcgc tgcaagcgca gcatcaggtg gttacggccc tgggagcgga     900
cagcaaggcc cagggcaaca aggcccaggg gggcaagggc cttatggtcc cggagcgagc     960
gcagcggcgg cagctgccgg aggttacgga ccgggaagtg gtcaacaagg tcccggacag    1020
caaggtcccg gccagcaggg accgggtcaa caaggtccag gcggacaggg accgtatggg    1080
cctggcgcat cagcagctgc cgctgcggct ggcggatatg gccctgggag tggccaacaa    1140
gggcctggcc aacaaggtcc gggtcagcag ggaccagggc agcaggttac cggtcgtggt    1200
gatagtccgg caagcgggcc aggccaacag ggccctggac agcaggggcc tggacagcaa    1260
ggtccgggac aacaaggccc tggtcagcaa gggcctggtg gtcagggtgc ctacggtccg    1320
ggtgcttcag cagctgcagg ggctgccggt ggatatggcc caggctcagg tcaacagggt    1380
cccggtcagc agggtccggg acaacaggga ccaggccaac aagtgacagg tcgcggtgat    1440
tcaccggcat ctggcccagg acagcaagga cctggtcagc agggtccagg ccaacaaggt    1500
cctggccagc agggtcctta tggacccgga gcctcagcag ctgcagcagc cgcaggcggc    1560
tatggtcctg gtagtggcca gcaaggacca ggccagcaag gaccaggaca acagggaccg     1620
ggaggccaag gaccgtacgg acccggtgca gcaagcgcgg cagttagcgt tggtggatac    1680
ggtccgcaga gcagcagcgt tccggttgcc agcgcagttg caagccgtct gagcagtccg    1740
gcagcaagca gtcgtgttag cagtgcagtt agcagcctgg ttagctcagg tccgaccaaa    1800
catgcagcac tgagcaatac cattagcagc gttgtgagcc aggttagcgc aagcaatccg    1860
ggtctgagcg gttgtgatgt tctggtgcag gcactgctgg aagttgttag cgcactggtg    1920
agcattctgg gtagcagcag cattggtcag attaactatg gtgcgagcgc acagtatacc    1980
cagatggttg gtcagagcgt tgcacaggca ctggcataa                           2019


<210>  54
<211>  672
<212>  PRT
<213>  Araneus diadematus

<400>  54
Met His His His His His His His His His His Ser Ser Gly Ser Ser
1               5                   10                  15
Ala Arg Ala Gly Ser Gly Gln Gln Gly Pro Gly Gln Gln Gly Pro Gly
            20                  25                  30
Gln Gln Gly Pro Gly Gln Gln Gly Pro Tyr Gly Pro Gly Ala Ser Ala
        35                  40                  45
Ala Ala Ala Ala Ala Gly Gly Tyr Gly Pro Gly Ser Gly Gln Gln Gly
    50                  55                  60
Pro Ser Gln Gln Gly Pro Gly Gln Gln Gly Pro Gly Gly Gln Gly Pro
65                  70                  75                  80
Tyr Gly Pro Gly Ala Ser Ala Ala Ala Ala Ala Ala Gly Gly Tyr Gly
                85                  90                  95
Pro Gly Ser Gly Gln Gln Gly Pro Gly Gly Gln Gly Pro Tyr Gly Pro
            100                 105                 110
Gly Ser Ser Ala Ala Ala Ala Ala Ala Gly Gly Asn Gly Pro Gly Ser
        115                 120                 125
Gly Gln Gln Gly Ala Gly Gln Gln Gly Pro Gly Gln Gln Gly Pro Gly
    130                 135                 140

Ala Ser Ala Ala Ala Ala Ala Ala Gly Gly Tyr Gly Pro Gly Ser Gly
145                 150                 155                 160
Gln Gln Gly Pro Gly Gln Gln Gly Pro Gly Gly Gln Gly Pro Tyr Gly
                165                 170                 175
Pro Gly Ala Ser Ala Ala Ala Ala Ala Ala Gly Gly Tyr Gly Pro Gly
            180                 185                 190
Ser Gly Gln Gly Pro Gly Gln Gln Gly Pro Gly Gly Gln Gly Pro Tyr
        195                 200                 205
Gly Pro Gly Ala Ser Ala Ala Ala Ala Ala Ala Gly Gly Tyr Gly Pro
    210                 215                 220
Gly Ser Gly Gln Gln Gly Pro Gly Gln Gln Gly Pro Gly Gln Gln Gly
225                 230                 235                 240
Pro Gly Gly Gln Gly Pro Tyr Gly Pro Gly Ala Ser Ala Ala Ala Ala
                245                 250                 255
Ala Ala Gly Gly Tyr Gly Pro Gly Tyr Gly Gln Gln Gly Pro Gly Gln
            260                 265                 270
Gln Gly Pro Gly Gly Gln Gly Pro Tyr Gly Pro Gly Ala Ser Ala Ala
        275                 280                 285
Ser Ala Ala Ser Gly Gly Tyr Gly Pro Gly Ser Gly Gln Gln Gly Pro
    290                 295                 300
Gly Gln Gln Gly Pro Gly Gly Gln Gly Pro Tyr Gly Pro Gly Ala Ser
305                 310                 315                 320
Ala Ala Ala Ala Ala Ala Gly Gly Tyr Gly Pro Gly Ser Gly Gln Gln
                325                 330                 335
Gly Pro Gly Gln Gln Gly Pro Gly Gln Gln Gly Pro Gly Gln Gln Gly
            340                 345                 350
Pro Gly Gly Gln Gly Pro Tyr Gly Pro Gly Ala Ser Ala Ala Ala Ala
        355                 360                 365
Ala Ala Gly Gly Tyr Gly Pro Gly Ser Gly Gln Gln Gly Pro Gly Gln
    370                 375                 380
Gln Gly Pro Gly Gln Gln Gly Pro Gly Gln Gln Val Thr Gly Arg Gly
385                 390                 395                 400
Asp Ser Pro Ala Ser Gly Pro Gly Gln Gln Gly Pro Gly Gln Gln Gly
                405                 410                 415
Pro Gly Gln Gln Gly Pro Gly Gln Gln Gly Pro Gly Gln Gln Gly Pro
            420                 425                 430
Gly Gly Gln Gly Ala Tyr Gly Pro Gly Ala Ser Ala Ala Ala Gly Ala
        435                 440                 445
Ala Gly Gly Tyr Gly Pro Gly Ser Gly Gln Gln Gly Pro Gly Gln Gln
    450                 455                 460
Gly Pro Gly Gln Gln Gly Pro Gly Gln Gln Val Thr Gly Arg Gly Asp
465                 470                 475                 480
Ser Pro Ala Ser Gly Pro Gly Gln Gln Gly Pro Gly Gln Gln Gly Pro
                485                 490                 495
Gly Gln Gln Gly Pro Gly Gln Gln Gly Pro Tyr Gly Pro Gly Ala Ser
            500                 505                 510
Ala Ala Ala Ala Ala Ala Gly Gly Tyr Gly Pro Gly Ser Gly Gln Gln
        515                 520                 525
Gly Pro Gly Gln Gln Gly Pro Gly Gln Gln Gly Pro Gly Gly Gln Gly
    530                 535                 540
Pro Tyr Gly Pro Gly Ala Ala Ser Ala Ala Val Ser Val Gly Gly Tyr
545                 550                 555                 560
Gly Pro Gln Ser Ser Ser Val Pro Val Ala Ser Ala Val Ala Ser Arg
                565                 570                 575
Leu Ser Ser Pro Ala Ala Ser Ser Arg Val Ser Ser Ala Val Ser Ser
            580                 585                 590
Leu Val Ser Ser Gly Pro Thr Lys His Ala Ala Leu Ser Asn Thr Ile
        595                 600                 605
Ser Ser Val Val Ser Gln Val Ser Ala Ser Asn Pro Gly Leu Ser Gly
    610                 615                 620
Cys Asp Val Leu Val Gln Ala Leu Leu Glu Val Val Ser Ala Leu Val
625                 630                 635                 640
Ser Ile Leu Gly Ser Ser Ser Ile Gly Gln Ile Asn Tyr Gly Ala Ser

                645                 650                 655
Ala Gln Tyr Thr Gln Met Val Gly Gln Ser Val Ala Gln Ala Leu Ala
            660                 665                 670